# EUROPEAN PATENT APPLICATION

(11) **EP 4 596 694 A1**
(43) Date of publication of application: **06.08.2025**
(21) Application number: 23872322.5
(22) Date of filing: 26.09.2023
(51) Int. Cl.: C12N 15/113, A61K 31/712, A61K 48/00, A61P 25/00

(54) **NUCLEIC ACID MOLECULE CONTAINING 5'-CYCLOPROPYLENE MODIFICATION**

(30) Priority: 29.09.2022 JP 2022155777
(71) Applicant: INSTITUTE OF SCIENCE TOKYO, Tokyo 152-8550 (JP); OSAKA UNIVERSITY, Suita-shi Osaka 565-0871 (JP)
(72) Inventor: YOKOTA Takanori, Tokyo 152-8550 (JP); YOSHIOKA Kotaro, Tokyo 152-8550 (JP); KURODA Takayuki, Tokyo 152-8550 (JP); OBIKA Satoshi, Suita-shi, Osaka 565-0871 (JP); YAMAGUCHI Takao, Suita-shi, Osaka 565-0871 (JP)
(74) Representative: Mewburn Ellis LLP
(86) International application number: PCT/JP2023/034892
(87) International publication number: WO 2024/071099

(57) **Abstract**

A problem to be addressed is to provide a novel nucleic acid molecule capable of simultaneously achieving both a high gene regulatory effect and reduced toxicity. Provided is a nucleic acid molecule having an antisense effect on a target gene or a transcriptional product thereof, comprising a base sequence complementary to at least part of the target gene or the transcriptional product thereof, wherein the nucleic acid molecule comprises: [1] a central region which comprises at least three consecutive deoxyribonucleosides, [2] a 5'-wing region which comprises a non-natural nucleoside, positioned on the 5' end side of said central region, and [3] a 3'-wing region which comprises a non-natural nucleoside, positioned on the 3' end side of said central region, and wherein the nucleic acid molecule comprises the 5'-modified nucleoside of formula (I) at the 1st, 3rd, and/or 9th base position from the 5' side of said central region.

## Description

### Technical Field

The present invention relates to a nucleic acid molecule comprising a 5'-cyclopropylene modification, a double-stranded nucleic acid complex, and a composition.

### Background Art

In recent years, an oligonucleotide has been drawing attention in the ongoing development of a pharmaceutical called nucleic acid medicine, and in particular, development of nucleic acid medicine utilizing the antisense method is actively carried out from the viewpoint of high selectivity on the target gene and low toxicity. The antisense method is a method comprising selectively modifying or inhibiting the expression of a protein encoded by a target gene or the activity of miRNA by introducing into a cell an oligonucleotide complementary to a target sense strand that is a partial sequence of mRNA or miRNA transcribed from the target gene (antisense oligonucleotide, herein often referred to as "ASO").

As a nucleic acid utilizing the antisense method, the present inventors have developed a double-stranded nucleic acid complex (heteroduplex oligonucleotide, HDO) in which an antisense oligonucleotide and a complementary strand thereto are annealed (Patent Literature 1 and 2, Non-Patent Literature 1 and 2). A double-stranded nucleic acid complex provides an epoch-making technology that has a high antisense effect.

As clinical development of nucleic acid medicine such as an antisense method has advanced and the results of preclinical tests have been accumulated, it has been revealed that avoidance of the toxicity of the nucleic acid medicine is important. In the nucleic acid medicine, here is a known trade-off between gene suppression effect and toxicity. That is, the stronger the gene suppression effect of the nucleic acid medicine, the higher the toxicity tends to be in general. In particular, a gapmer antisense nucleic acid that induces cleavage of a target transcription product and an antisense nucleic acid comprising a bridged nucleic acid have a strong gene suppression effect, but are highly toxic and, in some cases, exhibit lethal toxicity.

Therefore, a novel technology for producing a high gene regulatory effect while reducing toxicity is demanded.

### Citation List

### Patent Literature

Patent Literature 1: WO2013/089283
Patent Literature 2: WO2014/192310
Patent Literature 3: WO2020/158910

### Non-Patent Literature

Non-Patent Literature 1: Nishina K, et. al., "DNA/RNA heteroduplex oligonucleotide for highly efficient gene silencing", Nature Communications, 2015, 6:7969.
Non-Patent Literature 2: Asami Y, et al., "Drug delivery system of therapeutic oligonucleotides", Drug Discoveries & Therapeutics. 2016; 10(5):256-262.

### Summary of Invention

### Technical Problem

A problem to be addressed is to provide a novel nucleic acid molecule capable of simultaneously achieving both a high gene regulatory effect and reduced toxicity.

### Solution to Problem

The present inventors have studied vigorously to solve the above-described problem and introduced a 5'-cyclopropylene modification (often abbreviated herein as a "5'-CP modification") into a DNA nucleoside in a gap region of a gapmer nucleic acid molecule. As a result, the present inventors have found that the introduction of a 5'-CP modification into a nucleic acid molecule at a particular position thereof can significantly reduce or eliminate the toxicity of the nucleic acid molecule. This toxicity reduction effect depends on the base position at which the 5'-CP modification is introduced, and it has been found that a noticeable toxicity-reducing effect can be produced by placing the 5'-CP-modified nucleoside at the 1st, 3rd, or 9th base position from 5' side of the gap region. Meanwhile, it has been revealed that nucleic acid molecule can achieve both reduced toxicity and high efficacy without impairing the antisense effect. The present invention is based on the above-described findings, and provides the following.
(1) A nucleic acid molecule having an antisense effect on a target gene or a transcriptional product thereof, comprising a base sequence complementary to at least part of the target gene or the transcriptional product thereof,
   wherein the nucleic acid molecule comprises:
   [1] a central region which comprises at least three consecutive deoxyribonucleosides,
   [2] a 5'-wing region which comprises a non-natural nucleoside, positioned on the 5' end side of said central region, and
   [3] a 3'-wing region which comprises a non-natural nucleoside, positioned on the 3' end side of said central region, and
   wherein the nucleic acid molecule comprises a 5'-modified nucleoside of the following formula (I) at the 1st, 3rd, and/or 9th base position from the 5' side of said central region. 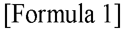 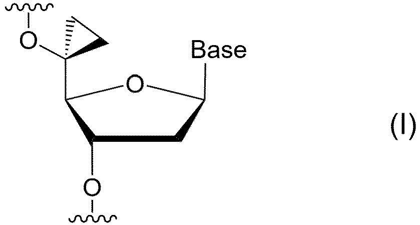
(2) The nucleic acid molecule of (1), wherein the internucleoside linkage at 5' side of said 5'-modified nucleoside is a modified internucleoside linkage or phosphodiester linkage.
(3) The nucleic acid molecule of (2), wherein said modified internucleoside linkage is phosphorothioate linkage.
(4) The nucleic acid molecule of (1), comprising a 2'-modified nucleoside at: the terminal base position adjacent to said central region in said 5'-wing region, the 2nd base position from 5' side of said central region, and/or the 8th base position from 5' side of said central region.
(5) The nucleic acid molecule of (1) or (4), comprising a 2'-modified nucleoside at the base position adjacent to 5' side of said 5'-modified nucleoside.
(6) The nucleic acid molecule of (4) or (5), wherein said 2'-modified nucleoside is 2'-O-methyl-modified nucleoside, 2'-O-methoxyethyl-modified nucleoside, 2'-O-[2-(N-methylcarbamoyl)ethyl]-modified nucleoside, or 2'-fluoro-modified nucleoside.
(7) The nucleic acid molecule of any one of (1) to (6), which is 12 to 30 bases in length.
(8) The nucleic acid molecule of (1), wherein said 5'-wing region and said 3'-wing region comprise a bridged nucleoside and/or a 2'-modified nucleoside.
(9) The nucleic acid molecule of (8), wherein said bridged nucleoside is selected from the group consisting of LNA nucleoside, 2', 4'- BNA^{NC} nucleoside, cET BNA nucleoside, ENA nucleoside, AmNA nucleoside, GuNA nucleoside, scpBNA nucleoside, scpBNA2 nucleoside, and BANA3 nucleoside.
(10) The nucleic acid molecule of (8) or (9), wherein said 2'-modified nucleoside is 2'-O-methyl-modified nucleoside or 2'-O-methoxyethyl-modified nucleoside.
(11) The nucleic acid molecule of any one of (1) to (10), wherein all or a part of the internucleoside linkages in said nucleic acid molecule are modified internucleoside linkages.
(12) The nucleic acid molecule of (11), wherein said modified internucleoside linkage is phosphorothioate linkage.
(13) The nucleic acid molecule of any one of (1) to (12), comprising a modified nucleobase.
(14) The nucleic acid molecule of any one of (1) to (13), which is capable of inducing steric blocking, exon skipping, and/or exon inclusion for said target gene or a transcriptional product thereof.
(15) A double-stranded nucleic acid complex comprising a first nucleic acid strand consisting of the nucleic acid molecule of any one of (1) to (14) and a second nucleic acid strand comprising a base sequence complementary to said first nucleic acid strand.
(16) The double-stranded nucleic acid complex of (15), wherein said second nucleic acid strand is at least 8 bases in length.
(17) The double-stranded nucleic acid complex of (15) or (16), wherein said second nucleic acid strand comprises any one or more selected from the group consisting of a deoxyribonucleoside, 2'-modified nucleoside, 5'-modified nucleoside, and bridged nucleoside.
(18) The double-stranded nucleic acid complex of any one of (15) to (17), wherein said second nucleic acid strand comprises a noncomplementary base, and/or an insertion sequence and/or deletion of one or more bases, relative to said first nucleic acid strand.
(19) The double-stranded nucleic acid complex of (18), wherein said second nucleic acid strand comprises one to three of said noncomplementary base.
(20) The double-stranded nucleic acid complex of (18) or (19), wherein said insertion sequence consists of 1 to 8 bases.
(21) The double-stranded nucleic acid complex of any one of (18) to (20), wherein said deletion consists of 1 to 4 consecutive bases.
(22) The double-stranded nucleic acid complex of any one of (15) to (21), wherein said second nucleic acid strand comprises at least one overhang region positioned on the 5' end side and/or 3' end side of a region consisting of the base sequence complementary to said first nucleic acid strand.
(23) The double-stranded nucleic acid complex of (22), wherein said overhang region is 1 to 20 bases in length.
(24) The double-stranded nucleic acid complex of any one of (15) to (23), wherein said first nucleic acid strand and said second nucleic acid strand are bound via a linker.
(25) The double-stranded nucleic acid complex of (24), wherein said linker is a cleavable or uncleavable linker.
(26) The double-stranded nucleic acid complex of (24) or (25), wherein said linker comprises the group represented by the following formula (IV). 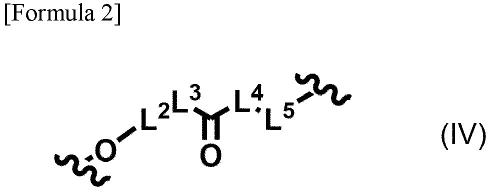 [wherein
   L₂ represents a substituted or unsubstituted C₁-C₁₂ alkylene group, a substituted or unsubstituted C₃-C₈ cycloalkylene group, -(CH₂)₂-O-(CH₂)₂-O-(CH₂)₂-O-(CH₂)₃-, or CH(CH₂-OH)-CH₂-O-(CH₂)₂-O-(CH₂)₂-O-(CH₂)₂-O-(CH₂)₃-,
   L₃ represents -NH- or a bond,
   L₄ represents a substituted or unsubstituted C₁-C₁₂ alkylene group, a substituted or unsubstituted C₃₋₈ cycloalkylene group, -(CH₂)₂-[O-(CH₂)₂]ₘ-, or a bond, wherein m is an integer of 1 to 25, L₅ represents -NH-(C=O)-, -(C=O)-, or a bond.]
(27) The double-stranded nucleic acid complex of (24) or (25), wherein said linker comprises a nucleic acid, polyether group, and/or alkylamino group.
(28) The double-stranded nucleic acid complex of (27), wherein said nucleic acid consists of one or 2 to 10 nucleosides and/or non-natural nucleosides linked via internucleoside linkages.
(29) The double-stranded nucleic acid complex of (27), wherein said polyether group is a polyethylene glycol group or triethylene glycol group.
(30) The double-stranded nucleic acid complex of (27), wherein said alkylamino group is a hexylamino group.
(31) The double-stranded nucleic acid complex of any one of (15) to (30), wherein all or a part of the internucleoside linages in said second nucleic acid strand are modified internucleoside linkages.
(32) The double-stranded nucleic acid complex of (31), wherein said modified internucleoside linkages are phosphorothioate linages.
(33) A composition comprising the nucleic acid molecule of any one of (1) to (14) or the double-stranded nucleic acid complex of any one of (15) to (32).
(34) The composition of (33) for treating a central nervous system disease of a subject.
(35) The composition of (34), wherein said central nervous system is selected from the group consisting of cerebral cortex, basal ganglia, cerebral white matter, diencephalon, brainstem, cerebellum, and spinal cord.
(36) The composition of (34), wherein said central nervous system is selected from the group consisting of frontal lobe, temporal lobe, hippocampus, parahippocampal gyrus, parietal lobe, occipital lobe, striatum, globus pallidus, claustrum, thalamus, subthalamic nucleus, midbrain, substantia nigra, pons, medulla oblongata, cerebellar cortex, cerebellar nuclei, cervical cord, thoracic cord, and lumbar cord.
(37) The composition of (33), which is for intrathecal administration, intranasal administration, intravenous administration, subcutaneous administration, intraperitoneal administration, or intramuscular administration.
(38) The composition of (37), wherein said intrathecal administration is intraventricular administration, suboccipital puncture or lumbar puncture.
(39) The composition of (37) or (38), which is administered with a shunt, indwelling catheter, or subcutaneous port.
(40) The composition of any one of (33) to (39), wherein 0.1 mg to 200 mg of said double-stranded nucleic acid complex is administered.

The present specification encompasses the contents disclosed in Japanese Patent Application No. 2022-155777, which is the basis of the priority of the present application.

### Advantageous Effects of Invention

The present invention provides novel nucleic acid medicine capable of simultaneously achieving both a high gene regulatory effect and reduced toxicity.

### Brief Description of Drawings

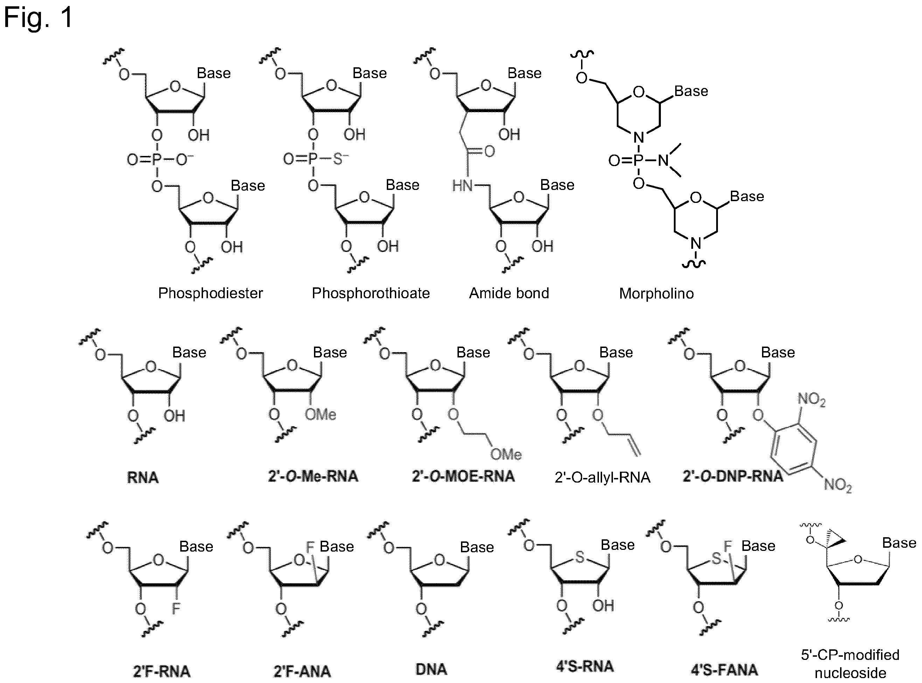

[Figure 1] Figure 1 shows the structures of various natural nucleotides or non-natural nucleotides.
[Figure 2] Figure 2 shows the structures of various bridged nucleic acids.
[Figure 3] Figure 3 shows the structures of nucleic acids used in Example 1.
[Figure 4] Figure 4 shows the results of measurement of the number of viable cells after transfection with various nucleic acid agents. The error bars indicate standard errors.
[Figure 5] Figure 5 shows the LDH activity in the supernatant after transfection with various nucleic acid agents. The error bars indicate standard errors.
[Figure 6] Figure 6 shows the Cdkn1a mRNA expression level after transfection with various nucleic acid agents. The error bars indicate standard errors.
[Figure 7] Figure 7 shows the Il-6 mRNA expression level after transfection with various nucleic acid agents. The error bars indicate standard errors.
[Figure 8] Figure 8 shows the structures of nucleic acids used in Example 2.
[Figure 9] Figure 9 shows the results of measurement of the number of viable cells after transfection with various nucleic acid agents. The error bars indicate standard errors.
[Figure 10] Figure 10 shows the LDH activity in the supernatant after transfection with various nucleic acid agents. The error bars indicate standard errors.
[Figure 11] Figure 11 shows the structures of nucleic acids used in Example 3.
[Figure 12] Figure 12 shows the body weight of mice and their food intake at 20 days after various nucleic acid agents were intraventricularly administered to the mice. Figure 12A shows the body weight. Figure 12B shows the food intake. The error bars indicate standard errors.
[Figure 13] Figure 13 shows the results of evaluation of the motor function of mice at 10, 14, 17, and 20 days after various nucleic acid agents were intraventricularly administered to the mice. The vertical axis indicates the maximum moving speed in the open-field test. The error bars indicate standard errors.
[Figure 14] Figure 14 shows the Mapt mRNA expression level in the lumbar spinal cord at 21 days after the intraventricular administration of various nucleic acid agents.
[Figure 15] Figure 15 shows the structures of nucleic acids used in Example 4.
[Figure 16] Figure 16 shows the results of measurement of the number of viable cells after transfection with various nucleic acid agents. The error bars indicate standard errors.
[Figure 17] Figure 17 shows the LDH activity in the supernatant after transfection with various nucleic acid agents. The error bars indicate standard errors.
[Figure 18] Figure 18 shows the Cdkn1a mRNA expression level after transfection with various nucleic acid agents. The error bars indicate standard errors.
[Figure 19] Figure 19 shows the structures of nucleic acids used in Example 5.
[Figure 20] Figure 20 shows the results of measurement of the number of viable cells after transfection with various nucleic acid agents. The error bars indicate standard errors.
[Figure 21] Figure 21 shows the LDH activity in the supernatant after transfection with various nucleic acid agents. The error bars indicate standard errors.
[Figure 22] Figure 22 shows the structures of nucleic acids used in Example 6.
[Figure 23] Figure 23 shows the results of measurement of the number of viable cells after transfection with various nucleic acid agents. The error bars indicate standard errors.
[Figure 24] Figure 24 shows the LDH activity in the supernatant after transfection with various nucleic acid agents. The error bars indicate standard errors.
[Figure 25] Figure 25 shows the structures of nucleic acids used in Example 7.
[Figure 26] Figure 26 shows the Hdac2 mRNA expression level after transfection with various nucleic acid agents. The error bars indicate standard errors.
[Figure 27] Figure 27 shows the structures of nucleic acids used in Example 8.
[Figure 28] Figure 28 shows a scoring system used to evaluate the acute tolerability scores.
[Figure 29] Figure 29 shows the body weight of mice and their food intake after various nucleic acid agents were intraventricularly administered to the mice. Figure 29A shows the body weight. Figure 29B shows the food intake. The error bars indicate standard errors.
[Figure 30] Figure 30 shows the results of evaluation of delayed central nervous system toxicity in mice at 6 days after various nucleic acid agents were intraventricularly administered to the mice. Figure 30A shows the motor function. Figure 30B shows the behavior evaluation. The error bars indicate standard errors.
[Figure 31] Figure 31 shows the structures of nucleic acids used in Example 9.
[Figure 32] Figure 32 shows the body weight of mice after various nucleic acid agents were intraventricularly administered to the mice. The error bars indicate standard errors.
[Figure 33] Figure 33 shows the food intake in mice after various nucleic acid agents were intraventricularly administered to the mice. The error bars indicate standard errors.
[Figure 34] Figure 34 shows the results of evaluation of the motor function of mice after various nucleic acid agents were intraventricularly administered to the mice. The vertical axis indicates the maximum moving speed in the open-field test. The error bars indicate standard errors.
[Figure 35] Figure 35 shows evaluation of behavior of mice to which various nucleic acid agents were intraventricularly administered on the basis of a scoring system. The error bars indicate standard errors.
[Figure 36] Figure 36 shows the expression amounts of Tnf-α mRNA and Gfap mRNA in the hippocampus of mice to which various nucleic acid agents were intraventricularly administered. Figure 36A shows the Tnf-α mRNA expression level. Figure 36B shows the Gfap mRNA expression level. The error bars indicate standard errors.
[Figure 37] Figure 37 shows the body weight of mice and their food intake after various nucleic acid agents were intraventricularly administered to the mice. Figure 37A shows the body weight. Figure 37B shows the food intake. The error bars indicate standard errors.
[Figure 38] Figure 38 shows the results of evaluation of the motor function of mice after various nucleic acid agents were intraventricularly administered to the mice. The vertical axis indicates the maximum moving speed in the open-field test. The error bars indicate standard errors.
[Figure 39] Figure 39 shows the results of evaluation of the motor function of mice after various nucleic acid agents were intraventricularly administered to the mice. The vertical axis indicates the maximum moving speed in the open-field test. The error bars indicate standard errors.
[Figure 40] Figure 40 shows the Hdac2 mRNA expression level in the left hippocampus at 7 days after the intraventricular administration of various nucleic acid agents.

### Description of Embodiments

### 1. Nucleic acid molecule

### 1-1. Overview

A first aspect of the present invention is a nucleic acid molecule. The nucleic acid molecule of the present invention is a nucleic acid molecule having an antisense effect on a target gene or a transcriptional product thereof, comprising a base sequence complementary to at least part of the target gene or the transcriptional product thereof, and comprises a 5'-cyclopropylene modified nucleoside at the particular base position. The nucleic acid molecule of the present invention can simultaneously achieve a high gene regulatory effect and low toxicity or non-toxicity.

### 1-2. Definition of terms

A "transcription product" of a target gene means herein any RNA that is synthesized by an RNA polymerase. Specifically, mRNA transcribed from a target gene (comprising, e.g., mature mRNA, mRNA precursor, and mRNA without base modification), non-coding RNA (ncRNA) such as miRNA, long non-coding RNA (lncRNA), and natural antisense RNA can be included.

A "target gene" means herein a gene wherein the expression amount of a transcription product or a translation product thereof can be reduced or increased by the antisense effect of the nucleic acid molecule or the double-stranded nucleic acid complex; a gene wherein the function of a transcription product or a translation product thereof can be inhibited by the effect; or a gene for which steric blocking, splicing control (e.g., splicing switching, exon skipping, and exon inclusion), base editing, or RNA editing can be induced by the effect. There is no particular restriction on the kind of target gene. Examples thereof include a gene which is derived from an organism into which a nucleic acid strand, a nucleic acid molecule, or a double-stranded nucleic acid complex is to be introduced, a gene whose expression is increased in various diseases (e.g., central nervous system diseases), and a gene that is expressed in vivo, such as the central nervous system. Specific examples thereof include a scavenger receptor B1 (often referred to herein as "SR-B1") gene, and a metastasis associated lung adenocarcinoma transcript 1 (herein often referred to as "Malat1") gene, a microtubule-associated protein tau (herein often referred to as "Mapt") gene, β-secretase 1 (herein often referred to as "BACE1") gene, a histone deacetylase 2 (herein often referred to as "Hdac2") gene, a DMPK (dystrophia myotonica-protein kinase) gene, and a dystrophin gene.

A "target transcription product" means herein a transcription product that is a direct target of the nucleic acid molecule or the double-stranded nucleic acid complex, and a "transcription product of a target gene" is also a target transcription product. The base sequence information for target transcription products and target genes can be obtained from publicly known databases, such as the database of NCBI (The U.S. National Center for Biotechnology Information).

An "antisense nucleic acid" herein refers to a single-stranded nucleic acid molecule that comprises a base sequence capable of hybridizing (i.e., complementary) to at least a part of a target transcription product (mainly, a transcription product of a target gene), and can produce an antisense effect on the target transcription product. In addition, an "antisense oligonucleotide" herein means an antisense nucleic acid composed of oligonucleotides. The "antisense nucleic acid" or "antisense oligonucleotide" is herein often referred to as "ASO". The nucleic acid molecule of the present invention or the first nucleic acid strand of the double-stranded nucleic acid complex functions as ASO, and its target region may comprise 3'UTR, 5'UTR, exon, intron, coding region, translation initiation region, translation termination region, or any other nucleic acid region. The target region of a target transcription product may be at least 8 bases in length, e.g., 10 to 35 bases in length, 12 to 25 bases in length, 13 to 20 bases in length, 14 to 19 bases in length, 15 to 18 bases in length, 13 to 22 bases in length, 16 to 22 bases in length, or 16 to 20 bases in length.

An "antisense effect" herein means any effect produced by hybridization of ASO to a target transcription product (e.g., RNA sense strand), such as an effect of regulating expression or editing of the target transcription product. The phrase "regulating expression or editing of a target transcription product" includes suppression or reduction of the expression of a target gene or the expression amount of a target transcription product ("expression amount of a target transcription product" is herein often referred to as "expression level of a target transcription product"), inhibition of translation, RNA editing, base editing, splicing control or a splicing function modifying effect (e.g., splicing switching, exon inclusion, and exon skipping), steric blocking, or degradation of a transcription product. For example, in the case of post-transcriptional inhibition of a target gene, when an RNA oligonucleotide is introduced into a cell as ASO, the ASO forms a partial double strand by annealing to mRNA which is a transcription product of a target gene. This partial double strand serves as a cover to prevent translation by ribosomes, so as to inhibit the expression of the target protein encoded by the target gene at the translation level (steric blocking). Meanwhile, when an oligonucleotide comprising DNA is introduced into a cell as ASO, a partial DNA-RNA heteroduplex is formed. This heteroduplex structure is recognized by RNase H, and as a result mRNA of the target gene is degraded and the expression of the protein encoded by the target gene is inhibited at the expression level. In addition, an antisense effect can also be produced for an intron in an mRNA precursor as a target. Furthermore, an antisense effect can also be produced for miRNA as a target. In this case, as a result of functional inhibition of the miRNA, the expression of the gene whose expression is normally regulated by the miRNA may be increased. In one embodiment, expression regulation of a target transcription product may be a decrease in the amount of a target transcription product.

The antisense effect can be measured, e.g., as follows: a subject nucleic acid compound is administered to a subject (e.g., a mouse); and, e.g., after several days (e.g., after 2 to 7 days), a measurement is made of the expression amount of a target gene or the level (amount) of the target transcription product (e.g., the amount of mRNA, the amount of RNA such as microRNA, the amount of cDNA, the amount of protein, or the like), wherein the expression of the target gene is regulated by the antisense effect provided by the subject nucleic acid compound.

For example, when the expression amount measured of the target gene or the level measured of the target transcription product is decreased by at least 10%, at least 20%, at least 25%, at least 30%, or at least 40%, compared with a negative control (e.g., vehicle administration), the measurement shows that the subject nucleic acid compound can produce an antisense effect (e.g., a decrease in the amount of the target transcription product).

The number, kind, and position of a non-natural nucleotide in a nucleic acid strand may influence the antisense effect and the like provided by the nucleic acid strand, the nucleic acid molecule, or the nucleic acid complex. The choice of a modification may vary depending on the sequence of a target gene or the like, but those skilled in the art can determine a suitable embodiment by referring to the descriptions in the literature related to the antisense method (e.g., WO 2007/143315, WO 2008/043753, and WO 2008/049085). Furthermore, when the antisense effect of a nucleic acid complex after the modification is measured and the obtained measured value is not significantly lower than the measured value of the nucleic acid complex before the modification (e.g., when the measured value obtained after the modification is 70% or more, 80% or more, or 90% or more of the measured value of the nucleic acid complex before the modification), a relevant modification may be evaluated.

A "translation product of the target gene" means herein any polypeptide or protein that is synthesized by translation of a target transcription product or a transcription product of a target gene which is a direct target of a nucleic acid molecule or double-stranded nucleic acid complex.

Herein, "decoy" refers to a nucleic acid having the sequence of a binding site of a transcription factor (e.g., NF-kB) or a similar sequence, which is introduced into a cell as a "decoy" to inhibit the function of a transcription factor (inhibit transcription in the case of a transcription activator or promote transcription in the case of a transcription repressor). A decoy nucleic acid can be easily designed based on information about a binding sequence of a target transcription factor.

Herein, "bait" refers to a nucleic acid molecule that specifically binds to a particular target molecule in a cell and modifies a function of the target molecule. A target that interacts with a bait is also referred to as a "prey".

The term "nucleic acid" or "nucleic acid molecule" used herein may refer to a nucleotide or nucleoside of a monomer, and may mean an oligonucleotide composed of a plurality of monomers or a plurality of nucleosides linked via an internucleoside linkage, or means a polynucleotide in the case of a polymer. A "natural nucleic acid" refers to a naturally-occurring nucleic acid. Examples of the natural nucleic acid include a natural nucleoside, natural nucleotide, and the like, as described below. A "non-natural nucleic acid" or "artificial nucleic acid" refers to any nucleic acid other than a natural nucleic acid. Examples of the non-natural nucleic acid or the artificial nucleic acid include a non-natural nucleoside, non-natural nucleotide, and the like, as described below.

A "nucleic acid strand" or simply "strand" herein means two or more nucleosides linked via an internucleoside linkage, and may be, e.g., an oligonucleotide or a polynucleotide. A full-length strand or a partial length strand of a nucleic acid strand can be produced, e.g., by a chemical synthesis using an automated synthesizer, or by an enzymatic step using a polymerase, a ligase, or a restricted reaction. A nucleic acid strand may comprise a natural nucleotide and/or a non-natural nucleotide.

A "nucleoside" generally means a molecule consisting of a combination of a base and a sugar. The sugar moiety of a nucleoside is usually, but not limited to, composed of pentofuranosyl sugar, and specific examples thereof include ribose and deoxyribose. The base moiety of nucleoside (nucleobase) is usually a heterocyclic base moiety. Without limitation, examples thereof include adenine, cytosine, guanine, thymine, or uracil as well as other modified nucleobases (modified bases).

A "nucleotide" refers to a molecule in which a phosphate group is covalently bound to the sugar moiety of the nucleoside. In the case of a nucleotide comprising pentofuranosyl sugar, a phosphate group is usually linked to a hydroxyl group at the 2', 3', or 5' position of the sugar.

An "oligonucleotide" refers to a linear oligomer formed by linking several to dozens of neighboring nucleotides through a covalent bond between a hydroxyl group and a phosphate group in the sugar moiety. Furthermore, a "polynucleotide" refers to a linear polymer formed by linking with covalent bonds dozens or more, preferably hundreds or more of nucleotides, namely more nucleotides than in an oligonucleotide. It is considered that the phosphate group generally forms an internucleoside linkage inside the structure of an oligonucleotide or a polynucleotide.

A "natural nucleoside" refers herein to a nucleoside that exists in nature. Examples thereof include a ribonucleoside consisting of a ribose and the aforementioned base such as adenine, cytosine, guanine, or uracil, or a deoxyribonucleoside consisting of a deoxyribose and the aforementioned base such as adenine, cytosine, guanine, or thymine. In this regard, a ribonucleoside found in RNA, and a deoxyribonucleoside found in DNA are herein often referred to as "RNA nucleoside" and "DNA nucleoside", respectively.

A "natural nucleotide" means herein a nucleotide that exists in nature, namely a molecule in which a phosphate group is covalently bound to the sugar moiety of the aforementioned natural nucleoside. Examples thereof include a ribonucleotide which is known as a constituent of RNA, and in which a phosphate group is bound to a ribonucleoside, and a deoxyribonucleotide, which is known as a constituent of DNA, and in which a phosphate group is bound to a deoxyribonucleoside.

A "non-natural nucleotide" means herein any nucleotide other than a natural nucleotide. For example, it comprises a modified nucleotide and a nucleotide mimic. A "modified nucleotide" means herein a nucleotide having any one or more of a modified sugar moiety, a modified internucleoside linkage, and a modified nucleobase. The term "nucleotide mimic" herein comprises a structure used to substitute a nucleoside and a linkage at one or more positions in an oligomer compound. Examples of the nucleotide mimic comprise a peptide nucleic acid, and a morpholino nucleic acid (morpholino linked with -N(H)-C(=O)-O- or other non-phosphodiester linkages). The peptide nucleic acid (PNA) is a nucleotide mimic having a main chain in which N-(2-aminoethyl)glycine in place of a sugar is linked with an amide linkage. A nucleic acid strand comprising a non-natural oligonucleotide herein has in many cases preferable properties, such as enhanced cellular uptake, enhanced affinity for a target nucleic acid, increased stability in the presence of a nuclease, and increase in inhibitory activity. Therefore, it is more preferable than a natural nucleotide.

A "non-natural nucleoside" means herein any nucleoside other than a natural nucleoside. For example, it comprises a modified nucleoside and a nucleoside mimic. A "modified nucleoside" means herein a nucleoside having a modified sugar moiety and/or a modified nucleobase.

A "mimic" refers herein to a functional group that substitutes a sugar, a nucleobase, and/or an internucleoside linkage. In general, a mimic is used in place of a sugar or a combination of a sugar-internucleoside linkage, and a nucleobase is maintained for hybridization to a target to be selected. The term "nucleoside mimic" used herein comprises a structure to be used for substituting a sugar at one or more positions of an oligomer compound, or substituting a sugar and a base, or substituting a bond between monomer subunits constituting an oligomer compound. An "oligomer compound" means a polymer composed of linked monomer subunits that can at least hybridize to a region of a nucleic acid molecule. Examples of a nucleoside mimic comprise a morpholino, cyclohexenyl, cyclohexyl, tetrahydropyranyl, bicyclic or tricyclic sugar mimic, such as a nucleoside mimic having a non-furanose sugar unit.

A "modified sugar" refers to a sugar in which a natural sugar moiety (i.e., sugar moiety found in DNA(2'-H) or RNA(2'-OH)) has undergone a substitution and/or any change. "Sugar modification" refers to substitution and/or any change from a natural sugar moiety. A nucleic acid strand may comprise in some cases one or more modified nucleosides comprising a modified sugar. A "sugar-modified nucleoside" means a nucleoside having a modified sugar moiety. Such a sugar-modified nucleoside can confer a beneficial biological property such as enhanced nuclease stability, increased binding affinity, or the like to a nucleic acid strand. In a specific embodiment, a nucleoside comprises a chemically modified ribofuranose ring moiety. Examples of a chemically modified ribofuranose ring comprise, but are not limited to, addition of a substituent (comprising 5' and 2' substituents), formation of a bicyclic nucleic acid (bridged nucleic acid, BNA) through bridge formation of a non-geminal ring atom, substitution of a ribosyl ring oxygen atom with S, N(R), or C(R1)(R2) (wherein R, R1, and R2 independently represent H, a C₁-C₁₂ alkyl, or a protecting group), and a combination thereof.

Examples of the sugar-modified nucleoside comprise, but are not limited to, a nucleoside comprising a substituent such as 5'-cyclopropylene, 5'-vinyl, and 5'-alkyl (e.g., 5'-methyl (R or S), 5'-ethyl(R or S)), 5'-allyl (R or S), 4'-S, 2'-F(2'-fluoro group), 2'-OCH₃ (2'-O-Me group or 2'-O-methyl group), 2'-O-[2-(N-methylcarbamoyl)ethyl] (2'-O-MCE group), and 2'-O-methoxyethyl (2'-O-MOE or 2-O(CH₂)₂OCH₃). A substituent at the 2' position may be selected from allyl, amino, azide, thio, -O-allyl, -O-C₁-C₁₀ alkyl, -OCF₃, -O(CH₂)₂SCH₃, - O(CH₂)₂-O-N(Rm)(Rn), and O-CH₂-C(=O)-N(Rm)(Rn), wherein Rm and Rn are independently H or a substituted or unsubstituted C₁-C₁₀ alkyl. A "2'-modified sugar" means a furanosyl sugar modified at the 2' position. A nucleoside comprising a 2'-modified sugar may be referred to as a "2'-modified nucleoside" or a "2'-sugar-modified nucleoside". In addition, a "5'-modified sugar" means a furanosyl sugar modified at the 5' position. A nucleoside comprising a 5'-modified sugar is referred to as a "5'-modified nucleoside" or a "5'-sugar-modified nucleoside", and in particular, a deoxyribonucleoside comprising a 5'-modified sugar and a ribonucleoside comprising a 5'-modified sugar are respectively referred to as a "5'-modified deoxyribonucleoside" and a "5'-modified ribonucleoside", for example, to distinguish between them.

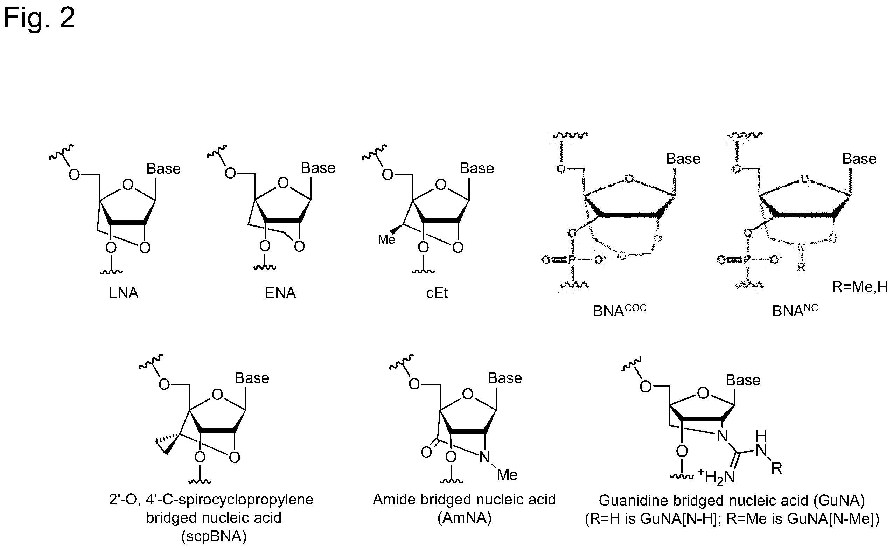

A "bicyclic nucleoside" refers to a modified nucleoside comprising a bicyclic sugar moiety. A nucleic acid comprising a bicyclic sugar moiety is commonly referred to as bridged nucleic acid (BNA). A nucleoside comprising a bicyclic sugar moiety is sometimes referred to as a "bridged nucleoside", "bridged-type non-natural nucleoside", or "BNA nucleoside". Some examples of a bridged nucleic acid are shown in Figure 2.

A bicyclic sugar may be a sugar in which the carbon atom at the 2' position and the carbon atom at the 4' position are bridged with two or more atoms. Examples of a bicyclic sugar are known to those skilled in the art. A subgroup of nucleic acids comprising a bicyclic sugar (BNA) or of BNA nucleosides may be described as having a carbon atom at the 2' position and a carbon atom at the 4' position which are bridged with 4'-(CH₂)ₚ-O-2', 4'-(CH₂)ₚ-CH₂-2', 4'-(CH₂)ₚ-S-2', 4'-(CH₂)ₚ-OCO-2', 4'-(CH₂)ₙ-N(R₃)-O-(CH₂)ₘ-2' [wherein p, m, and n represent integers from 1 to 4, from 0 to 2, and from 1 to 3, respectively; and R₃ represents a hydrogen atom, an alkyl group, an alkenyl group, a cycloalkyl group, an aryl group, an aralkyl group, an acyl group, a sulfonyl group, and a unit substituent (e.g., a fluorescently or chemiluminescently labeled molecule, a functional group with nucleic acid cleavage activity, and an intracellular or nuclear localization signal peptide)]. Furthermore, with respect to a BNA or a BNA nucleoside in a certain embodiment, in the OR₂ substituent of the carbon atom at the 3' position and the OR₁ substituent of the carbon atom at the 5' position, R₁ and R₂ are typically hydrogen atoms, but may be the same or different from each other, or may also be a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, a phosphate group protected by a protecting group for nucleic acid synthesis, or P(R₄)R₅ [wherein R₄ and R₅, may be the same or different from each other, and respectively represent a hydroxyl group, a hydroxyl group protected by a protecting group for nucleic acid synthesis, a mercapto group, a mercapto group protected by a protecting group for nucleic acid synthesis, an amino group, a C₁-C₅ alkoxy group, a C₁-C₅ alkylthio group, a C₁-C₆ cyanoalkoxy group, or an amino group substituted with a C₁-C₅ alkyl group]. Non-limiting examples of such BNA comprise methyleneoxy(4'-CH₂-O-2') BNA (LNA, Locked Nucleic Acid^{®}, also known as 2',4'-BNA) (e.g., α-L-methyleneoxy(4'-CH₂-O-2') BNA or β-D-methyleneoxy(4'-CH₂-O-2') BNA), ethyleneoxy(4'-(CH₂)₂-O-2') BNA (also known as ENA), β-D-thio(4'-CH₂-S-2') BNA, aminooxy(4'-CH₂-O-N(R₃)-₂') BNA, oxyamino(4'-CH₂-N(R₃)-O-2') BNA (also known as 2',4'-BNA^{NC}; R=H is 2',4'-BNA^{NC}[N-H], R=Me is 2',4'-BNA^{NC}[N-Me]), 2',4'-BNAcoc, 3'-amino-2',4'-BNA, 5'-methyl BNA, (4'-CH(CH₃)-O-2') BNA (also known as cEt BNA), (4'-CH(CH₂OCH₃)-O-2') BNA (also known as cMOE BNA), amide BNA (amide-bridged nucleic acid) or (4'-C(O)-N(R)-2') BNA (R=H, or Me) (also known as AmNA; R=H in Figure 2 is AmNA[N-H], R=Me is AmNA[N-Me]), guanidine BNA (also known as GuNA (e.g., R=H is GuNA[N-H], R=Me is GuNA[N-Me]) in Figure 2), amine BNA (also known as 2'-Amino-LNA) (e.g., 3-(Bis(3-aminopropyl)amino)propanoyl substitution product), 2'-O,4'-C-spirocyclopropylene-bridged nucleic acid (also known as scpBNA), and other BNA known to those skilled in the art. Non-limiting examples of such BNA nucleoside comprise methyleneoxy(4'-CH₂-O-2') BNA nucleoside (also known as LNA nucleoside or 2',4'-BNA nucleoside) (e.g., α-L-methyleneoxy(4'-CH₂-O-2') BNA nucleoside, β-D-methyleneoxy(4'-CH₂-O-2') BNA nucleoside), ethyleneoxy(4'-(CH₂)₂-O-2') BNA nucleoside (also known as ENA nucleoside), β-D-thio(4'-CH₂-S-2') BNA nucleoside, aminooxy(4'-CH₂-O-N(R₃)-2') BNA nucleoside, oxyamino(4'-CH₂-N(R₃)-O-2') BNA nucleoside (also known as 2',4'-BNA^{NC} nucleoside; R=H is 2',4'-BNA^{NC}[N-H] nucleoside, and R=Me is 2',4'-BNA^{NC}[N-Me] nucleoside), 2',4'-BNA^{coc} nucleoside, 3'-amino-2',4'-BNA nucleoside, 5'-methyl BNA nucleoside, (4'-CH(CH₃)-O-2') BNA nucleoside (also known as cEt nucleoside), (4'-CH(CH₂OCH₃)-O-2') BNA nucleoside (also known as cMOE nucleoside), amide BNA nucleoside or (4'-C(O)-N(R)-2') BNA nucleoside (R=H, or Me) (also known as AmNA nucleoside; R=H in Figure 2 is AmNA[N-H] nucleoside, and R=Me is AmNA[N-Me] nucleoside), guanidine BNA nucleoside (GuNA nucleoside (e.g., R=H in Figure 2 is also known as GuNA[N-H] nucleoside, and R=Me is also known as GuNA[N-Me] nucleoside)), amine BNA nucleoside (also known as 2'-Amino-LNA nucleoside) (e.g., 3-(Bis(3-aminopropyl)amino)propanoyl-substituted nucleoside), 2'-O,4'-C-spirocyclopropylene-bridged nucleoside (also known as scpBNA nucleoside), and other BNA nucleosides known to those skilled in the art.

A "cationic nucleoside" herein is a modified nucleoside existing in cationic form, compared with a neutral form (such as the neutral form of ribonucleoside), at a pH (e.g., the physiological pH (approximately 7.4) of a human, a pH of a delivery site (e.g., an organelle, cell, tissue, organ, or organism), or the like). The cationic nucleoside may comprise one or more cationic modified groups at any position of a nucleoside. In one embodiment, the cationic nucleoside is 2'-Amino-LNA nucleoside (e.g., 3-(Bis(3-aminopropyl)amino)propanoyl-substituted nucleoside), aminoalkyl-modified nucleoside (e.g., 2'-O-methyl- and 4'-CH₂CH₂CH₂NH₂-substituted nucleoside), GuNA nucleoside (e.g., R=H in Figure 2 is GuNA[N-H] nucleoside, and R=Me is GuNA[N-Me] nucleoside), or the like. A bicyclic nucleoside having a methyleneoxy(4'-CH₂-O-2') bridge is also referred to as LNA nucleoside.

A "modified internucleoside linkage" means herein an internucleoside linkage that has a substitution or any change from a naturally occurring internucleoside linkage (i.e., phosphodiester linkage). A modified internucleoside linkage comprises an internucleoside linkage that comprises a phosphorus atom, and an internucleoside linkage that does not comprise a phosphorus atom. Typical examples of the phosphorus-containing internucleoside linkage comprise, but are not limited to, a phosphodiester linkage, a phosphorothioate linkage, a phosphorodithioate linkage, a phosphotriester linkage (a methylphosphotriester linkage and an ethylphosphotriester linkage described in U.S. Pat. No. 5,955,599), an alkylphosphonate linkage (e.g., a methylphosphonate linkage described in U.S. Pat. Nos. 5,264,423 and 5,286,717, and a methoxypropylphosphonate linkage described in WO2015/168172), an alkylthiophosphonate linkage, a methylthiophosphonate linkage, a boranophosphate linkage, an internucleoside linkage comprising a cyclic guanidine moiety (e.g., a partial structure represented by the following Formula (V): 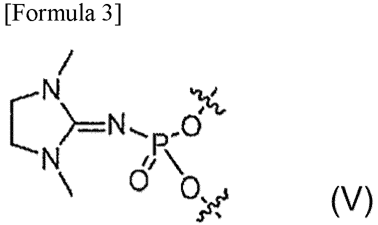 ), an internucleoside linkage comprising a guanidine moiety substituted with one to four C₁₋₆ alkyl groups (e.g., a tetramethyl guanidine (TMG) moiety) (e.g., a partial structure represented by the following Formula (VI): 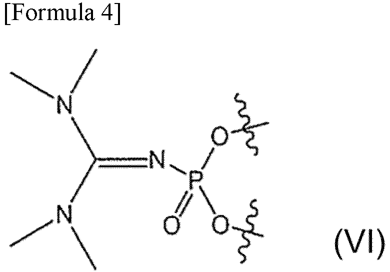 ), and an internucleoside linkage and a phosphoramidate linkage that are to be used for a self-neutralizing nucleic acid (ZON) as described in WO2016/081600. A phosphorothioate linkage refers to an internucleoside linkage in which an unbridged oxygen atom in a phosphodiester linkage is substituted with a sulfur atom. A method for preparing a phosphorus-containing and a phosphorus-free linkage is well known. It is preferable that a modified internucleoside linkage is a linkage having a higher resistance to a nuclease than a naturally occurring internucleoside linkage.

When an internucleoside linkage has a chiral center, the internucleoside linkage may be chirally controlled. The term "chirally controlled" means that a single diastereomer is present with respect to the chiral center, e.g., chirally bound phosphorus.

For example, the internucleoside linkage may be a phosphorothioate linkage chirally controlled to an Rp configuration or Sp configuration, an internucleoside linkage comprising a guanidine moiety substituted with one to four C₁₋₆ alkyl groups (e.g., a tetramethyl guanidine (TMG) moiety; see, e.g., Alexander A. Lomzov et al., Biochem Biophys Res Commun., 2019, 513 (4), 807-811), and/or an internucleoside linkage comprising a cyclic guanidine moiety. A phosphorothioate linkage chirally controlled to an Rp configuration or Sp configuration is also publicly known. A chirally controlled phosphorothioate linkage in the Sp configuration is known to be more stable than those in the Rp configuration, and chirally controlled ASOs in the Sp configuration are also known to promote target RNA cleavage by RNase H1, resulting in a more sustained response in vivo.

The term "nucleobase" or "base" used herein is a base component (heterocyclic moiety) constituting a nucleic acid. As the component, mainly adenine, guanine, cytosine, thymine, and uracil are known. The "nucleobase" or "base" herein encompasses both of a modified and an unmodified nucleobase (base), unless otherwise specified. Accordingly, a purine base may be any of a modified and an unmodified purine base, unless otherwise specified. In addition, a pyrimidine base may be any of a modified and an unmodified pyrimidine base, unless otherwise specified.

A "modified nucleobase" or a "modified base" means any nucleobase other than adenine, cytosine, guanine, thymine, or uracil. The term "unmodified nucleobase" or "unmodified base" (a natural nucleobase) means adenine (A) and guanine (G), which are purine bases, and thymine (T), cytosine (C), and uracil (U), which are pyrimidine bases. Examples of a modified nucleobase comprise, but are not limited to, hypoxanthine, 5-methylcytosine, 5-fluorocytosine, 5-bromocytosine, 5-iodocytosine, and N4-methylcytosine; N6-methyladenine or 8-bromoadenine; 2-thio-thymine; and N2-methylguanine or 8-bromoguanine.

Herein, the "gapmer" refers to a single-stranded nucleic acid that, in principle, comprises or consists of a "central region" (DNA gap region) and wing regions positioned directly at the 5' end and 3' end of the central region (the wing regions are referred to as a "5'-wing region" and a "3'-wing region" respectively). The central region in the gapmer comprises at least three or at least four consecutive deoxyribonucleosides. The 5'-wing region and the 3'-wing region each comprise at least one non-natural nucleoside.

The position of the boundary between the central region (DNA gap region) and the 5'-wing region and the 3'-wing region in the gapmer can be easily determined from the sequence of the nucleoside by those skilled in the art. For example, the central region can be functionally defined as a region recognizable by RNase H (e.g., RNase H1). Here, the phrase "recognizable by RNase H" means that, when a gapmer is bound to a target RNA, the sequence that is in the target RNA, and paired with the gapmer can be cleaved by RNase H. Accordingly, in the gapmer, a region recognizable by RNase H can be regarded as the central region, and a region not recognizable by RNase H (more specifically, a region in which cleavage activity by RNase H is not substantially detected under physiological conditions) can be regarded as a wing region (5'-wing region and 3'-wing region), so that the position of the boundary can be determined. Herein, in the 5'-wing region and the 3'-wing region, the terminal nucleosides adjacent to the central region are non-natural nucleosides (e.g., 2'-modified nucleosides or bridged nucleosides), and in the central region, the nucleoside adjacent to the 5'-wing region or the 3'-wing region is a deoxyribonucleoside or a sugar-modified form thereof. In one embodiment, in the central region, both of the nucleosides adjacent to the 5'- wing region and the 3'-wing region are deoxyribonucleosides.

In the gapmer, the central region may comprise a modified nucleobase recognized by RNase H, for example, 5-methylcytosine. The central region can also comprise a non-natural nucleoside such as a 2'-modified nucleoside and a 5'-modified nucleoside, except for two terminal nucleosides adjacent to the 5'-wing region and the 3'-wing region. In one embodiment, the central region may be constituted such that all the internucleoside linkages in the region paired in the target RNA can be cleaved by RNase H.

Without limitation, a non-natural nucleoside comprised in the wing region usually has a higher bonding force to RNA than a natural nucleoside, and has high resistance to a nucleic acid-degrading enzyme (nuclease or the like). A non-natural nucleoside constituting the 5'-wing region and the 3'-wing region may be, e.g., a bridged nucleoside and/or a 2'-modified nucleoside. When a non-natural nucleoside constituting the wing region comprises or consists of a bridged nucleoside, the gapmer is specifically referred to as a "BNA/DNA gapmer". The number of bridged nucleosides comprised in the 5'-wing region and the 3'-wing region may be at least one, e.g., two or three. The bridged nucleosides comprised in the 5'-wing region and the 3'-wing region may be consecutively or inconsecutively present in the 5'-wing region and the 3'-wing region. The bridged nucleoside may further comprise a modified nucleobase (e.g., 5-methylcytosine). The bridged nucleoside may be an LNA nucleoside or an ENA nucleoside. When the bridged nucleoside is an LNA nucleoside, the gapmer is referred to as an "LNA/DNA gapmer". When the bridged nucleoside is an ENA nucleoside, said gapmer is referred to as an "ENA/DNA gapmer". When a non-natural nucleoside constituting the 5'-wing region and the 3'-wing region comprises or consists of a peptide nucleic acid, the gapmer is specifically referred to as a "peptide nucleic acid gapmer". When a non-natural nucleoside constituting the 5'-wing region and the 3'-wing region comprises or consists of a morpholino nucleic acid, the gapmer is specifically referred to as a "morpholino nucleic acid gapmer". When a non-natural nucleoside constituting the 5'-wing region and the 3'-wing region comprises or consists of a 2'-modified nucleoside, the 2'-modified group of the 2'-modified nucleoside may be a 2'-O-methyl group or a 2'-O-methoxyethyl group. The number of the 2'-modified nucleosides comprised in the 5'-wing region and the 3'-wing region may be at least one, e.g., two or three. the 2'-modified nucleosides comprised in the 5'-wing region and the 3'-wing region may be consecutively or inconsecutively present in the 5'-wing region and the 3'-wing region. The 2'-modified nucleoside may further comprise a modified nucleobase (e.g., 5-methylcytosine). When a non-natural nucleoside constituting the 5'-wing region and the 3'-wing region comprises or consists of a bridged nucleoside and a 2'-modified nucleoside, the non-natural nucleoside may be composed of a combination of two or more bridged nucleosides and/or 2'-modified nucleosides. In this regard, a nucleic acid strand having a wing region on only one of the 5' end side or 3' end side is referred to as a "hemi-gapmer" in the art, but herein, the hemi-gapmer is also encompassed in the gapmer.

The term "complementary" as used herein includes the relationship that nucleobases can form via hydrogen bonds so-called Watson-Crick base pairs (natural base pairs) or Wobble base pairs (guanine-thymine or guanine-uracil) and a relationship that a natural nucleobase and a modified nucleobase or modified nucleobases can form similar base pairs.

In the present invention, the antisense region of the nucleic acid molecule is not necessarily required to be completely complementary to at least a part of a target transcription product (e.g., the transcription product of a target gene), and it is acceptable if the base sequence has at least 70%, preferably at least 80%, and further preferably at least 90% (e.g., 95%, 96%, 97%, 98%, or 99% or more) of complementarity. The antisense region of the nucleic acid molecule can hybridize to a target transcription product when the base sequence is complementary (typically when the base sequence is complementary to the base sequence of at least a part of the target transcription product). In the double-stranded nucleic acid complex, similarly, the complementary region in the second nucleic acid strand is not necessarily required to be completely complementary to at least a part of the nucleic acid molecule that is the first nucleic acid strand, and it is acceptable if the base sequence has a complementarity of at least 70%, preferably at least 80%, and further preferably at least 90% (e.g., 95%, 96%, 97%, 98%, or 99% or more). The complementary region in the second nucleic acid strand can be annealed when the base sequence of the region is complementary to the base sequence of at least a part of the first nucleic acid strand. Base sequence complementarity can be determined by using a BLAST program or the like. Those skilled in the art can easily determine the conditions (temperature, salt concentration, and the like) under which two strands can anneal or hybridize, taking into account the degree of complementarity between the strands. Furthermore, those skilled in the art can easily design an antisense nucleic acid complementary to a target transcription product, e.g., based on information about the base sequence of a target gene.

Hybridization conditions may be variously stringent, e.g., low-stringent and high-stringent conditions. Low-stringent conditions may be relatively low temperature and high salt concentration conditions, e.g., 30°C, 2 × SSC, 0.1% SDS. High-stringent conditions may be relatively high temperature and low salt concentration conditions, e.g., 65°C, 0.1 × SSC, 0.1% SDS. The stringency of hybridization can be adjusted by changing conditions such as temperature and salt concentration. In this connection, 1 × SSC comprises 150 mM sodium chloride and 15 mM sodium citrate.

Herein, "toxicity" refers to an effect that induces an objective or subjective symptom or dysfunction that is not preferable to a subject. The toxicity may be toxicity in any organ, e.g., neurotoxicity, hepatotoxicity, or nephrotoxicity. Neurotoxicity refers to an effect that induces damage in a nervous tissue including a central nervous tissue and a peripheral nervous tissue, and prevents the normal activity of the nervous system. In particular, toxicity to the central nervous system is referred to as central nervous system toxicity. Neurotoxicity can induce a symptom selected from death, respiratory abnormality, cardiovascular abnormality, headache, nausea or vomiting, unresponsiveness or low responsiveness, consciousness disorder, psychiatric disorder, character change, hallucination, delusion, cognitive dysfunction, abnormal posture, involuntary movement, shivering, convulsion, hyperactivity motor dysfunction, paralysis, sensory disorder, or autonomic nervous system dysfunction. The neurotoxicity may be either acute neurotoxicity or delayed neurotoxicity. Acute neurotoxicity can be neurotoxicity caused within hours to tens of hours after administration, e.g., within 1, 3, 6, 9, 12, 24, or 48 hours, and can be distinguished from delayed neurotoxicity caused later. Nephrotoxicity refers to the property causing dysfunction and/or hypofunction in the kidney. Evaluation of hepatotoxicity and renal function can be performed by any technique known to those skilled in the art, such as serum biochemistry. For example, as described below in Examples, toxicity can be evaluated with an acute tolerability score, side-effect event rate, or death rate. Hepatotoxicity refers to the property causing dysfunction and/or hypofunction in the liver.

A "subject" herein refers to the object to which the nucleic acid molecule, the double-stranded nucleic acid complex, or pharmaceutical composition of the present invention is applied. A subject comprises an individual as well as an organ, a tissue, and a cell. When the subject is an individual, any animal including a human may be applicable. For example, in addition to a human, a variety of domestic animals, domestic fowls, pets, and laboratory animals are included. Without limitation, the subject may be an individual in need of a decrease in the expression amount of a target transcription product, or an individual in need of treatment or prevention of a disease such as a central nervous system disease.

The term "plurality" herein refers to, e.g., 2, 2 or 3, 2 to 4, 2 to 5, 2 to 6, 2 to 7, 2 to 8, 2 to 10, 2 to 12, 2 to 14, 2 to 16, 2 to 18, 2 to 20, 2 to 25, 2 to 30, 2 to 35, 2 to 40, or more.

### 1-3. Configuration

The nucleic acid molecule of the present invention comprises a base sequence complementary to at least part of a target gene or a transcriptional product thereof and has an antisense effect on said target gene or the transcriptional product thereof.

The nucleic acid molecule of the present aspect has a gapmer structure and comprises a central region which comprises at least three consecutive deoxyribonucleosides; a 5'-wing region which comprises a non-natural nucleoside, positioned on the 5' end side of said central region; and a 3'-wing region which comprises a non-natural nucleoside, positioned on the 3' end side of said central region.

The nucleic acid molecule of the present aspect comprises a 5'-modified nucleoside of the following formula (I) at a particular base position in the central region. 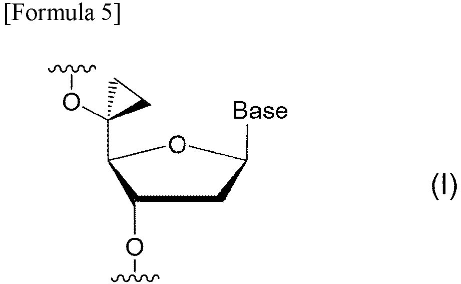 Since the modified group at the 5' position in the above-described formula (I) is a cyclopropane group, the 5'-modified nucleoside of the above-described formula (I) is herein referred to as a "5'-cyclopropane-modified nucleoside" or a "5'-cyclopropylene-modified nucleoside" and often abbreviated as a "5'-CP-modified nucleoside". The 5'-modified nucleoside of the above-described formula (I) can also be referred to as a 5'-cyclopropane-modified deoxyribonucleoside or a 5'-cyclopropylene-modified deoxyribonucleoside.

The nucleic acid molecule of the present aspect comprises the 5'-modified nucleoside of the above-described formula (I) at the 1st, 3rd, and/or 9th base position from the 5' side of the central region. Herein, the "Nth base position from the 5' side of the central region" means a base located at the Nth position in the 5' to 3' direction starting from a terminal base adjacent to the 5'-wing region in the central region. Therefore, the 1st base position from the 5' side of the central region means the terminal base adjacent to the 5'-wing region in the central region, and the 3rd base position from the 5' side of the central region means the base located at the 3rd position counting in the 3' direction starting from the terminal base adjacent to the 5'- wing region in the central region. The nucleic acid molecule of the present aspect may comprise the 5'-modified nucleoside of the above-described formula (I) at any one of the 1st, 3rd, and 9th base positions from the 5' side of the central region, preferably the 5'-modified nucleoside of the above-described formula (I) at the 3rd or 9th base position.

The number of the 5'-modified nucleosides of the above-described formula (I) comprised in the nucleic acid molecule of the present aspect is at least one, may be two or more or three, and is preferably two or less, e.g., one.

In the central region of the nucleic acid molecule of the present aspect, the kind of the nucleosides other than the 5'-CP nucleoside is not limited as long as the central region can be recognized by RNase H. For example, all the nucleosides other than the 5'-CP nucleoside in the central region may be deoxyribonucleosides or may comprise one or more non-natural nucleosides in addition to deoxyribonucleosides. Examples of a non-natural nucleoside that can be introduced into the central region without impairing the antisense effect include a non-natural deoxyribonucleoside such as 5'-modified deoxyribonucleoside. The 5'-modified deoxyribonucleoside is not particularly limited, and examples thereof include a 5'-alkyl-modified deoxyribonucleoside (e.g., a 5'-methyl-modified deoxyribonucleoside and a 5'-ethyl-modified deoxyribonucleoside), and a 5'-allyl-modified deoxyribonucleoside. The chirality of the 5'-modification in the deoxyribonucleoside is not limited, and may be either R- or S-type. It is preferable that non-natural nucleosides are not consecutive in the central region.

In the nucleic acid molecule of present aspect, the internucleoside linkage at 5' side of the 5'-modified nucleoside of the above-described formula (I) is a modified internucleoside linkage or phosphodiester linkage. The modified internucleoside linkage is not limited, and may be, e.g., a phosphorothioate linkage. Herein, the "internucleoside linkage at the 5' side of the 5'-modified nucleoside" means an internucleoside linkage bound to the 5' position of the nucleoside. The structure of a 5'-CP-modified nucleoside bound to a phosphorothioate linkage at the 5' side is represented by the following formula (II). 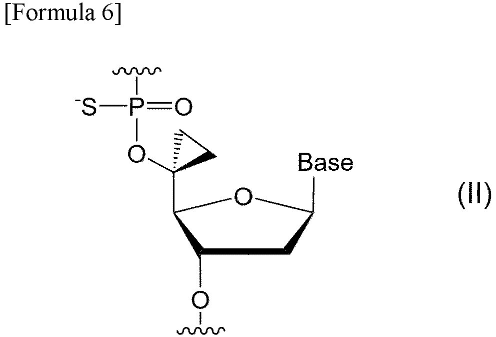 The structure of a 5'-CP-modified nucleoside bound to a phosphodiester linkage at the 5' side is represented by the following formula (III). 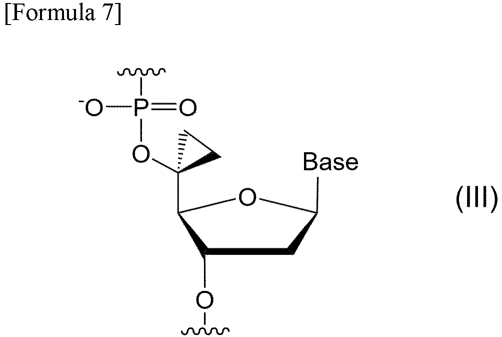 In the nucleic acid molecule of the present aspect, the internucleoside linkage at the 5' side of the 5'-modified nucleoside of the above-described formula (I) is preferably a phosphorothioate linkage.

In one embodiment, a nucleic acid molecule of the present aspect comprises a 5'-modified nucleoside of the above-described formula (I) at the 1st base position from 5' side of the central region, wherein the internucleoside linkage at 5' side of the 5'-modified nucleoside is, e.g., a modified internucleoside linkage or a phosphodiester linkage, preferably a phosphorothioate linkage.

In one embodiment, a nucleic acid molecule of the present aspect comprises a 5'-modified nucleoside of the above-described formula (I) at the 3rd base position from 5' side of the central region, wherein the internucleoside linkage at 5' side of the 5'-modified nucleoside is, e.g., a modified internucleoside linkage or a phosphodiester linkage, preferably a phosphorothioate linkage.

In one embodiment, a nucleic acid molecule of the present aspect comprises a 5'-modified nucleoside of the above-described formula (I) at the 9th base position from 5' side of the central region, wherein the internucleoside linkage at 5' side of the 5'-modified nucleoside is, e.g., a modified internucleoside linkage or a phosphodiester linkage, preferably a phosphorothioate linkage.

In the above embodiments, the kind of internucleoside linkages other than the internucleoside linkage at the 5' side of the 5'-CP nucleoside in the nucleic acid molecule of the present aspect is not limited. For example, all the internucleoside linkages other than the internucleoside linkage at the 5'-side of the 5'-CP nucleoside may be modified internucleoside linkages such as phosphorothioate linkages.

In a further embodiment, the nucleic acid molecule of the present aspect comprises a 2'-modified nucleoside at the terminal base position adjacent to said central region in the 5'-wing region, the 2nd base position from 5' side of said central region, and/or the 8th base position from 5' side of said central region. The "the terminal base position adjacent to said central region in the 5'-wing region" means a base position located at the most 3' end in the 5'-wing region. Said 2'-modified nucleoside may be placed on either the 5' side or the 3' side of the 5'-modified nucleoside of the above-described formula (I), either adjacent to or not adjacent to the 5'-modified nucleoside, and preferably adjacent to the 5' side of the 5'-modified nucleoside. The 2'-modification in the 2'-modified nucleoside is not limited, and may be, e.g., a 2'-O-methyl group, a 2'-O-[2-(N-methylcarbamoyl) ethyl] group, a 2'-O-methoxyethyl group, or a 2'-fluoro group, preferably a 2'-O-methyl group.

In a further embodiment, the nucleic acid molecule of the present aspect comprises a 5'-modified nucleoside at the 4th base position from the 5' side of the central region. Examples of the 5'-modified nucleoside include a 5'-modified deoxyribonucleoside, such as a 5'-alkyl-modified deoxyribonucleoside (e.g., a 5'-methyl-modified deoxyribonucleoside or a 5'-ethyl-modified deoxyribonucleoside), or a 5'-allyl-modified deoxyribonucleoside. The chirality of the 5'-modification in the deoxyribonucleoside is not limited, and may be either R- or S-type. However, the R-type such as (R-)5'-methyl-modified deoxyribonucleoside is particularly preferred.

In the nucleic acid molecule of the present aspect, the central region (gap region) may be, e.g., 3 to 12 bases in length, 4 to 11 bases in length, 5 to 10 bases in length, 6 to 9 bases in length, or 7 or 8 bases in length.

In addition, in the nucleic acid molecule of the present aspect, the base length of each of the 5'-wing region and the 3'-wing region may be independently at least 2 bases in length, e.g., 2 to 10 bases in length, 2 to 7 bases in length, 3 to 5 bases in length, 3 or 4 bases in length, or 3 bases in length.

The nucleic acid molecule of the present invention can comprise a 2'-modified nucleoside and/or bridged nucleoside in the 5'-wing region and the 3'-wing region. The 2'-modified nucleoside may be, e.g., 2'-O-methyl-modified nucleoside or 2'-O-methoxyethyl-modified nucleoside. The bridged nucleoside may be, e.g., LNA nucleoside, 2', 4'-BNA^{NC} nucleoside, cET BNA nucleoside, ENA nucleoside, AmNA nucleoside, GuNA nucleoside, scpBNA nucleoside, scpBNA2 nucleoside, or BANA3 nucleoside, preferably a combination of 2'-O-methyl-modified nucleosides, 2'-O-methoxyethyl-modified nucleosides, 2'-LNA, or ENA. The kind of modification may include one to four, two or three, e.g., two, which may be the same or different from each other in the 5'-wing region and the 3'-wing region.

In one embodiment, one or consecutive two or three nucleosides at the end adjacent to the central region in the 5'-wing region and/or the 3'-wing region consist of 2'-modified nucleosides and/or bridged nucleosides.

In a further embodiment, the 5'-wing region and/or the 3'-wing region consist of two or more 2'-modified nucleosides and/or bridged nucleosides linked via an internucleoside linkage.

In the nucleic acid molecule of the present aspect, examples of the base length of each region of the 5'-wing region, the central region, and the 3'-wing region include 2-12-3, 3-12-2, 3-12-3, 4-12-3, 2-11-3, 3-11-2, 3-11-3, 4-11-3, 2-10-3, 3-10-2, 3-10-3, 4-10-3, 2-9-3, 3-9-2, 3-9-3, 4-9-3, 2-8-3, 3-8-2, 3-7-3, 4-6-3, 3-6-4, 4-5-4, 4-7-3, 3-7-4, 4-6-4, 5-6-3, 3-6-5, 3-7-5, 5-7-3, 4-7-4, 4-6-5, 5-6-4, 5-5-5, and 5-6-5. Here, in the notation "A-B-C," "A" denotes the base length of the 5'-wing region, "B" denotes the base length of the central region, and "C" denotes the base length of the 3'-wing region.

The base length of the nucleic acid molecule of the present aspect is not particularly limited, and may be at least 8 bases in length, at least 9 bases in length, at least 10 bases in length, at least 11 bases in length, at least 12 bases in length, at least 13 bases in length, at least 14 bases in length, or at least 15 bases in length. Furthermore, the base length of the nucleic acid molecule may be 40 bases in length or less, 35 bases in length or less, 30 bases in length or less, 25 bases in length or less, 24 bases in length or less, 23 bases in length or less, 22 bases in length or less, 21 bases in length or less, 20 bases in length or less, 19 bases in length or less, 18 bases in length or less, 17 bases in length or less, or 16 bases in length or less. The base length of the nucleic acid molecule may be, e.g., 10 to 40 bases in length, 12 to 30 bases in length, or 15 to 25 bases in length. The length can be determined according to the balance between the strength of the antisense effect and the specificity of the nucleic acid strand to the target, among other factors such as cost, and synthesis yield. When a nucleic acid such as an aptamer is bound to a nucleic acid molecule, the base length of the nucleic acid molecule as a whole may be the sum of the above-described base length and the base length of the bound nucleic acid.

The internucleoside linkage in the nucleic acid molecule of the present aspect may be a naturally occurring internucleoside linkage and/or a modified internucleoside linkage. Without limitation, at least one, at least two, or at least three internucleoside linkages from an end (5' end, 3' end, or both the ends) of the nucleic acid molecule of the present aspect are preferably modified internucleoside linkages. In this regard, e.g., two internucleoside linkages from the end of a nucleic acid strand refers to an internucleoside linkage closest to the end of the nucleic acid strand, and an internucleoside linkage positioned next thereto on the opposite side to the end of the nucleic acid strand. Modified internucleoside linkages in the terminal region of a nucleic acid strand are preferred because they can reduce or inhibit undesired degradation of the nucleic acid strand.

In one embodiment, all or a part of the internucleoside linkages in the nucleic acid molecule may be modified internucleoside linkages. The modified internucleoside linkage may be a phosphorothioate linkage.

The nucleic acid molecule of the present invention may comprise, as a whole or in part, a nucleoside mimic or a nucleotide mimic. The nucleotide mimic may be a peptide nucleic acid and/or a morpholino nucleic acid.

The antisense effect on the target transcription product of the nucleic acid molecule of the present aspect can be measured by a method publicly known in the art. For example, after introducing a nucleic acid molecule into a cell and the like, it can be measured using a publicly known technique such as Northern blotting, quantitative PCR, or Western blotting. By measuring the expression amount of a target gene or the level of a target transcription product in specific tissues (e.g., the amount of RNA such as the amount of mRNA, and the amount of cDNA), it can be judged whether or not the target gene expression is suppressed by the nucleic acid molecule in these sites. When the expression amount measured of the target gene or the level measured of the target transcription product is decreased by at least 20%, at least 25%, at least 30%, at least 40%, or at least 50%, compared with a negative control (e.g., vehicle administration or no treatment), the measurement shows that the subject nucleic acid compound can produce an antisense effect.

### 1-4. Effects

A nucleic acid molecule of the present invention can reduce or eliminate toxicity (e.g., neurotoxicity such as central nervous system toxicity) of nucleic acid medicine without impairing high efficacy (e.g., steric blocking, exon skipping, exon inclusion, splicing control, decreased expression, increased expression, and/or base editing). In addition, the nucleic acid molecule can reduce cell death, induction of inflammation or gliosis, abnormal elevation of cytokines or chemokines, motor function, or the like, and behavioral abnormalities associated with the administration of antisense nucleic acids.

A gapmer antisense nucleic acid that can cleave a target, particularly a gapmer comprising a bridged nucleoside, has high efficacy but enhanced toxicity, which has been a major obstacle to practical use. The nucleic acid molecule of the present invention is based on a surprising effect of significantly reducing toxicity while maintaining efficacy.

The nucleic acid molecule of the present invention is less toxic than a gapmer nucleic acid molecule that does not comprise 5'-CP-modified nucleoside (e.g., a nucleic acid molecule that does not comprise a 5'-CP-modified nucleoside at the 1st, 3rd, and/or 9th base position from the 5' side of the central region).

Although 5'-CP modification is a nucleic acid modification that can strengthen nuclease resistance when a more biocompatible phosphodiester linkage is used for the purpose of avoiding a phosphorothioate linkage, which is concerned about its accumulation in a particular organ or the like (Patent Literature 3, WO 2020/158910, ABSTRACT), the effect of the 5'-CP modification itself in reducing toxicity in this Example was unexpected. In particular, it is an unexpected that the modification produces the toxicity-reducing effect regardless of whether the internucleoside linkage bound to the 5' side of the 5'-CP-modified nucleoside is a PS linkage or a PO linkage.

The present invention provides an agent for reducing toxicity of a gapmer nucleic acid molecule which consists of a 5'-CP-modified nucleoside. The agent for reducing toxicity can reduce the toxicity of a nucleic acid molecule placing it at a particular position in the gap region (central region) of the gapmer nucleic acid molecule. The present invention also provides the use of a 5'-CP-modified nucleoside to reduce the toxicity of the gapmer nucleic acid molecule.

### 2. Double-stranded nucleic acid complex

### 2-1. Overview

A second aspect of the present invention is a double-stranded nucleic acid complex. A double-stranded nucleic acid complex of the present aspect comprises a first nucleic acid strand which may function as an antisense nucleic acid and a second nucleic acid strand comprising a base sequence complementary to said first nucleic acid strand. The double-stranded nucleic acid complex of the present aspect can simultaneously achieve a high gene regulatory effect and low toxicity or non-toxicity.

### 2-2. Configuration

The double-stranded nucleic acid complex of the present aspect comprises the first nucleic acid strand and the second nucleic acid strand. Since the first nucleic acid strand is a nucleic acid molecule described in the first aspect and has a structure as described above, the description thereof is omitted here.

In the double-stranded nucleic acid complex of the present aspect, the second nucleic acid strand is a nucleic acid molecule comprising a base sequence complementary to the first nucleic acid strand. In the double-stranded nucleic acid complex of the present aspect, the second nucleic acid strand is annealed to the first nucleic acid strand via a hydrogen bond of a complementary base pair.

In the double-stranded nucleic acid complex of the present aspect, the second nucleic acid strand can comprise a deoxyribonucleoside, a 2'-modified nucleoside, a 5'-modified nucleoside, and/or a bridged nucleoside. For example, in the second nucleic acid strand, all of the nucleosides in a region consisting of a base sequence complementary to the central region of the first nucleic acid strand may be (a) deoxyribonucleosides; (b) deoxyribonucleosides and ribonucleosides; (c) deoxyribonucleosides and 2'-modified nucleosides; (d) ribonucleosides and 2'-modified nucleosides; or (e) deoxyribonucleosides, ribonucleosides, and 2'-modified nucleosides.

In one embodiment, the second nucleic acid strand comprises a region comprising at least three or at least four consecutive ribonucleosides and/or deoxyribonucleosides which are complementary to at least three or at least four consecutive deoxyribonucleosides in the central region of the first nucleic acid strand.

In a further embodiment, the second nucleic acid strand may comprise a region consisting of a base sequence complementary to the 5'-wing region and/or the 3'-wing region in the first nucleic acid strand. In the second nucleic acid strand, the region consisting of a base sequence complementary to the 5'-wing region and/or the 3'-wing region in the first nucleic acid strand may comprise at least one non-natural nucleoside, wherein the non-natural nucleoside may a bridged nucleoside and/or a 2'-modified nucleoside. In addition, the 2'-modified group of the 2'-modified nucleoside in the second nucleic acid strand may be a 2'-O-methyl group or a 2'-O-methoxyethyl group. When the first nucleic acid strand and the second nucleic acid strand both comprise a bridged nucleoside and/or a 2'-modified nucleoside, the bridged nucleoside and/or the 2'-modified nucleoside in the first nucleic acid strand and the second nucleic acid strand may be the same or different from each other.

The internucleoside linkage in the second nucleic acid strand may be a naturally occurring internucleoside linkage and/or a modified internucleoside linkage. Without limitation, at least one, at least two, or at least three internucleoside linkages from an end (5' end, 3' end, or both the ends) of the second nucleic acid strand are preferably modified internucleoside linkages. In one embodiment, all or a part of the internucleoside linkages in the second nucleic acid strand may be modified internucleoside linkages. In one embodiment, the second nucleic acid strand may comprise a modified internucleoside linkage in a region consisting of a base sequence complementary to the 5'-wing region and/or the 3'-wing region in the first nucleic acid strand. The modified internucleoside linkage may be a phosphorothioate linkage.

In a further embodiment, the second nucleic acid strand can comprise a 2'-modified nucleoside (e.g., 2'-O-methoxyethyl-modified nucleoside). The number of 2'-modified nucleosides (e.g., 2'-O-methoxyethyl-modified nucleosides) in the second nucleic acid strand is not limited. For example, at least 20%, at least 30%, at least 40%, at least 50%, at least 60%, at least 70%, at least 75%, at least 80%, at least 85%, at least 90%, at least 95%, or 100% of the total number of nucleosides in said second nucleic acid strand may be 2'-modified nucleosides (e.g., 2'-O-methoxyethyl-modified nucleosides). In one embodiment, all of the nucleosides in the second nucleic acid strand are 2'-modified nucleosides (e.g., 2'-O-methoxyethyl-modified nucleosides).

In a further embodiment, the second nucleic acid strand can comprise one or consecutive two or more 2'-modified nucleosides (e.g., 2'-O-methoxyethyl-modified nucleosides) at the 5' end, and/or one or consecutive two or more 2'-modified nucleosides (e.g., 2'-O-methoxyethyl-modified nucleosides) at the 3' end. The number of the 2'-modified nucleosides (e.g., 2'-O-methoxyethyl-modified nucleosides) at the 5' side and/or 3' side is not limited. For example, the second nucleic acid strand may comprise one or consecutive two, three, four, five, six, or seven 2'-modified nucleosides (e.g., 2'-O-methoxyethyl-modified nucleosides) at 5' side, and/or one or consecutive two, three, four, five, six, or seven 2'-modified nucleosides (e.g., 2'-O-methoxyethyl-modified nucleosides) at the 3' end.

In one embodiment, the second nucleic acid strand may comprise a modified nucleobase. The number of modified nucleobases is not limited, and may be at least one, at least two, at least three, at least four, at least five, or at least six.

In one embodiment, the second nucleic acid strand may comprise a non-complementary base, and/or one or more insertion sequences and/or deletions, relative to the first nucleic acid strand. The number of non-complementary bases in the second nucleic acid strand is not limited, and may be, e.g., 1 to 10, 1 to 9, 1 to 8, 1 to 7, 1 to 6, 1 to 5, 1 to 4, 1 to 3, 1 to 2, or 1 or 2. The base length of the insertion sequence in the second nucleic acid strand is not limited, and may be, e.g., 1 to 10, 1 to 9, 1 to 8, 1 to 7, 1 to 6, 1 to 5, 1 to 4, 1 to 3, 1 to 2, or 1 or 2. The base length of consecutive deletions in the second nucleic acid strand is not limited, and may be, e.g., 1 to 10, 1 to 9, 1 to 8, 1 to 7, 1 to 6, 1 to 5, 1 to 4, 1 to 3, 1 to 2, or 1 or 2. A region composed of a non-complementary base or an insertion sequence may form a bulge.

In one embodiment, the second nucleic acid strand may further comprise at least one overhang region positioned on one or both of the 5' end side and the 3' end side of the complementary region. An example of this embodiment is described in WO 2018/062510. The term "overhang region" refers to a region adjacent to a complementary region, namely a nucleotide region in the second nucleic acid strand in which the 5' end of the second nucleic acid strand extends beyond the 3' end of the first nucleic acid strand and/or the 3' end of the second nucleic acid strand extends beyond the 5' end of the first nucleic acid strand when the first nucleic acid strand and the second nucleic acid strand are annealed to form a double-stranded structure, or protruding from the double-stranded structure. The overhang region in the second nucleic acid strand may be located at the 5' end side of the complementary region or at the 3' end side. The overhang region in the second nucleic acid strand may be located at the 5' end side and 3' end side of the complementary region.

In one embodiment, a functional moiety is bound to the first nucleic acid strand and/or the second nucleic acid strand, e.g., the second nucleic acid strand. The linkage between the first nucleic acid strand and/or second nucleic acid strand and the functional moiety may be a direct linkage or an indirect linkage via another substance, and in some embodiments, it is preferable that the first nucleic acid strand and/or second nucleic acid strand and the functional moiety are directly bound by covalent bonding, ionic bonding, hydrogen bonding, or the like, and from the viewpoint of obtaining a more stable bond, covalent bonding is more preferable.

In one embodiment, the structure of the "functional moiety" is not particularly limited, and provides a desired function to the double-stranded nucleic acid complex to which the functional moiety is bound. The desired function includes a labeling function, a purification function, and a delivery function to a target. Examples of a moiety that provides a labeling function include compounds such as a fluorescent protein and luciferase. Examples of a moiety that provides a purification function include compounds such as biotin, avidin, His-tag peptide, GST-tag peptide, and FLAG-tag peptide. In addition, a molecule having an activity for delivery of a double-stranded nucleic acid complex in an embodiment to a target site is preferably bound as a functional moiety to the first nucleic acid strand and/or the second nucleic acid strand, from the viewpoint that the first nucleic acid strand is efficiently delivered to a target site at a high specificity and expression of a target gene is very effectively suppressed by the nucleic acid. Examples of a moiety for providing a delivery function to a target include a lipid, an antibody, an aptamer, and a ligand to a particular receptor.

In one embodiment, the first nucleic acid strand and/or the second nucleic acid strand, e.g., the second nucleic acid strand, is bound to a lipid. Examples of the lipid include, but are not limited to, tocopherol, cholesterol, fatty acid, phospholipid, and analogs thereof; folic acid, vitamin C, vitamin B1, and vitamin B2; estradiol, androstane, and analogs thereof; steroid and an analog thereof; ligand of LDLR, SRBI or LRP1/2; FK-506, and cyclosporine; and lipids described in WO 2019/182109 and WO 2019/177061. The lipid may be a tocopherol or an analog thereof and/or a cholesterol or an analog thereof, a substituted or unsubstituted C₁₋₃₀ alkyl group, a substituted or unsubstituted C₂₋₃₀ alkenyl group, or a substituted or unsubstituted C₁₋₃₀ alkoxy group. In one embodiment, the second nucleic acid strand may be bound to tocopherol or cholesterol, or an analog thereof.

"Tocopherol" is herein a methylated derivative of tocorol which is a liposoluble vitamin (vitamin E) having a cyclic structure called chroman. Tocorol has a strong antioxidant effect, and therefore functions in vivo as an antioxidant substance to scavenge free radicals produced by metabolism and protect cells from damage.

A plurality of different types of tocopherol consisting of α-tocopherol, β-tocopherol, γ-tocopherol, and δ-tocopherol are known based on the position of the methyl group bound to chroman. A tocopherol herein may be any types of tocopherol. In addition, examples of the analog of tocopherol comprise various unsaturated analogs of tocopherol, such as α-tocotrienol, β-tocotrienol, γ-tocotrienol, and δ-tocotrienol. Preferably, tocopherol is α-tocopherol.

"Cholesterol" is herein a kind of sterol, also called steroid alcohol, which is especially abundant in animals. Cholesterol exerts an important function in the metabolic process in vivo, and in animal cells, it is also a major constituent of the membrane system of a cell, together with phospholipid. In addition, the cholesterol analog refers to various cholesterol metabolism products and their analogs, which are alcohols having a sterol backbone. Examples thereof include, but are not limited to, cholestanol, lanosterol, cerebrosterol, dehydrocholesterol, and coprostanol.

An "analog" herein refers to a compound having a similar structure and property having the same or a similar basic backbone. The analog comprises, e.g., a biosynthetic intermediate, a metabolism product, and a compound having a substituent. Those skilled in the art can determine whether or not a compound is an analog of another compound based on common general technical knowledge.

The functional moiety may be linked to the 5' side, the 3' side, or both ends of the first nucleic acid strand and/or the second nucleic acid strand. Alternatively, the functional moiety may be linked to a nucleotide at an interior position in the first nucleic acid strand and/or the second nucleic acid strand. The first nucleic acid strand and/or the second nucleic acid strand comprise two or more functional moieties, such as lipids, which may be linked to a plurality of positions in the first nucleic acid strand and/or the second nucleic acid strand, and/or may be linked, in the form of one group, to one position in the first nucleic acid strand and/or the second nucleic acid strand. One functional moiety may be lined to each of the 5' side and 3' side of the first nucleic acid strand and/or the second nucleic acid strand.

The linkage between the first nucleic acid strand and/or the second nucleic acid strand and the functional moiety may be a direct linkage or an indirect linkage via another substance. In a specific embodiment, however, it is preferable that the functional moiety is directly bound to the first nucleic acid strand and/or the second nucleic acid strand via a covalent bond, an ionic bond, a hydrogen bond, or the like, and a covalent bond is more preferable considering that a more stable bond can be obtained.

The functional moiety may also be bound to the first nucleic acid strand and/or the second nucleic acid strand via a cleavable or uncleavable linker. In this case, the first nucleic acid strand and the second nucleic acid strand can be linked via a linker to form a single strand. However, even in that case, the functional region has the same configuration as the double-stranded nucleic acid complex, and therefore such a single-stranded nucleic acid is herein also encompassed as an embodiment of the double-stranded nucleic acid complex of the present invention. The linker can be any polymer. Examples thereof include a polynucleotide, polypeptide, alkylene. Specifically, it can be composed of a natural nucleotide such as DNA or RNA, or a non-natural nucleotide such as a peptide nucleic acid or a morpholino nucleic acid. When a linker consists of a nucleic acid, the chain length of a linker may be at least one base, or, e.g., a chain length of from 3 to 10 bases or from 4 to 6 bases. It is preferably 4 bases in length. In this case, the linker can take the form of a hinge (hairpin loop). The position of the linker can be either on the 5' side or the 3' side of the first nucleic acid strand. For example, in the case of a configuration in which the second nucleic acid strand is bound to the 5' side of the first nucleic acid strand, the 5' end of the first nucleic acid strand and the 3' end of the second nucleic acid strand are linked via a linker.

A "cleavable linker" refers to a linking group that is cleaved under physiological conditions, e.g., in a cell or in an animal body (e.g., in a human body). In a specific embodiment, a cleavable linker is selectively cleaved by an endogenous enzyme such as a nuclease. Examples of the cleavable linker include an amide, an ester, one or both esters of a phosphodiester, a phosphoester, a carbamate, and a disulfide bond, as well as a natural DNA linker.

An "uncleavable linker" refers to a linker that is not cleaved under physiological conditions, e.g., in a cell or in an animal body (e.g., in a human body). Examples of an uncleavable linker comprise, but are not limited to, a phosphorothioate linkage, modified or unmodified deoxyribonucleosides linked by a phosphorothioate linkage, and a linker consisting of modified or unmodified ribonucleosides. There is no particular restriction on the chain length, when a linker is a nucleic acid such as DNA, or an oligonucleotide, however, it may be usually from 2 to 20 bases in length, from 3 to 10 bases in length, or from 4 to 6 bases in length.

Specific examples of the linker comprise a linker represented by the following Formula (IV). 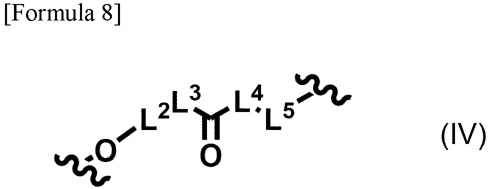 [wherein L² represents a substituted or unsubstituted C₁-C₁₂ alkylene group (e.g., propylene, hexylene, dodecylene), a substituted or unsubstituted C₃-C₈ cycloalkylene group (e.g., cyclohexylene), -(CH₂)₂-O-(CH₂)₂-O-(CH₂)₂-O-(CH₂)₃-, -(CH₂)₂-O-(CH₂)₂-O-(CH₂)₂-O-(CH₂)₂-O-(CH₂)₃-, or CH(CH₂-OH)-CH₂-O-(CH₂)₂-O-(CH₂)₂-O-(CH₂)₂-O-(CH₂)₃-, L³ represents -NH- or a bond, L⁴ represents a substituted or unsubstituted C₁-C₁₂ alkylene group (e.g., ethylene, pentylene, heptylene, or undecylene), a substituted or unsubstituted C₃-C₈ cycloalkylene group (e.g., cyclohexylene), -(CH₂)₂-[O-(CH₂)₂]ₘ-, or a bond, wherein m is an integer of 1 to 25, L⁵ represents -NH-(C=O)-, -(C=O)-, or a bond (here, the substitution is preferably carried out by a halogen atom).]

In one embodiment, regarding a linker represented by Formula (IV), L₂ is unsubstituted C₃-C₆ alkylene group (e.g., propylene, hexylene), -(CH₂)₂-O-(CH₂)₂-O-(CH₂)₂-O-(CH₂)₃-, or - (CH₂)₂-O-(CH₂)₂-O-(CH₂)₂-O-(CH₂)₂-O-(CH₂)₃-, L³ is -NH-, and L₄ and L⁵ are bonds.

In one embodiment, the linker comprises a nucleic acid, polyether group, and/or alkylamino group. The nucleic acid may consist of, e.g., one or 2 to 10 nucleosides and/or non-natural nucleosides linked via internucleoside linkages. Examples of the polyether group include a polyethylene glycol group or triethylene glycol group. Examples of the alkylamino group include a hexylamino group.

The base length of the first nucleic acid strand and the second nucleic acid strand is not particularly limited, and may be at least 8 bases in length, at least 9 bases in length, at least 10 bases in length, at least 11 bases in length, at least 12 bases in length, at least 13 bases in length, at least 14 bases in length, or at least 15 bases in length. Furthermore, the base length of the first nucleic acid strand and the second nucleic acid strand may be 40 bases in length or less, 35 bases in length or less, 30 bases in length or less, 25 bases in length or less, 24 bases in length or less, 23 bases in length or less, 22 bases in length or less, 21 bases in length or less, 20 bases in length or less, 19 bases in length or less, 18 bases in length or less, 17 bases in length or less, or 16 bases in length or less. The first nucleic acid strand and the second nucleic acid strand may have identical lengths or different lengths (for example, one of them is shorter or longer by 1 to 3 bases). The double-stranded structure formed by the first nucleic acid strand and the second nucleic acid strand may comprise a bulge. The length can be determined according to the balance between the strength of the antisense effect and the specificity of the nucleic acid strand to the target, among other factors such as cost, and synthesis yield. When a nucleic acid such as an aptamer is bound to a first nucleic acid strand and/or a second nucleic acid strand, the base length of the first nucleic acid strand and/or the second nucleic acid strand as a whole may be the sum of the above-described base length and the base length of the bound nucleic acid. In this case, the base length of the nucleic acid to be bound is not limited, and may be, e.g., at least 10 bases in length, at least 15 bases in length, or at least 20 bases in length, or may be 100 bases in length or less, 80 bases in length or less, 60 bases in length or less, 40 bases in length or less, or 30 bases in length or less.

### 2-3. Effects

The double-stranded nucleic acid complex of the present invention can simultaneously achieve high efficacy (e.g., steric blocking, exon skipping, exon inclusion, splicing control, decreased expression, increased expression, and/or base editing) and low toxicity or non-toxicity.

### 3. Composition

### 3-1. Overview

A third aspect of the present invention is a composition. The composition of the present invention comprises at least a nucleic acid molecule in the first aspect or a double-stranded nucleic acid complex in the second aspect, and can be used for treatment of a disease. The pharmaceutical composition of the present invention can achieve can simultaneously achieve a high gene regulatory effect and low toxicity or non-toxicity. The composition of the present aspect can exhibit an excellent antisense effect in the central nervous system while reducing neurotoxicity.

### 3-2. Configuration

### 3-2-1. Active ingredient

The composition of the present invention comprises as an active ingredient at least a nucleic acid molecule described in the first aspect or a double-stranded nucleic acid complex described in the second aspect. The composition of the present invention may comprise one or more nucleic acid molecules and/or double-stranded nucleic acid complex.

The amount (content) of the nucleic acid molecule or double-stranded nucleic acid complex in the composition of the present invention varies depending on the kind of the nucleic acid molecule or double-stranded nucleic acid complex, the delivery site, the dosage form of the composition, the dose of the composition, and the kind of a carrier described below. Therefore, it may be determined as appropriate by taking the respective conditions into consideration. Usually, it may be adjusted so that an effective amount of the nucleic acid molecule or the double-stranded nucleic acid complex is comprised in a single dose of the composition. An "effective amount" refers to an amount that is necessary for the nucleic acid molecule or the double-stranded nucleic acid complex to function as an active ingredient, and to an amount that has little or no adverse side effect on a living body to which the amount is applied. This effective amount can vary depending on various conditions such as information on the subject, the administration route, and number of administrations. Ultimately, it may be determined by the judgment of a physician, veterinarian, pharmacist, or the like. "Information on the subject" is various information on an individual of the living body to which the composition is applied. For example, when the subject is a human, it comprises age, body weight, gender, dietary habit, health status, stage of progression or grade of severity of the disease, drug sensitivity, and presence of a combined drug.

### 3-2-2. Carrier

The composition of the present invention may comprise a pharmaceutically acceptable carrier. A "pharmaceutically acceptable carrier" refers to an additive commonly used in the field of pharmaceutical preparation. Examples thereof include a solvent, a vegetable oil, a base, an emulsifier, a suspending agent, a surfactant, a pH adjuster, a stabilizer, an excipient, a vehicle, a preservative, a binder, a diluent, an isotonizing agent, a sedative, a bulking agent, a disintegrating agent, a buffering agent, a coating agent, a lubricant, a thickener, a dissolution aid, and other additives.

The solvent may be any of, e.g., water or other pharmaceutically acceptable aqueous solution, and a pharmaceutically acceptable organic solvent. Examples of an aqueous solution include a physiological saline, an isotonic solution comprising glucose or another additive, a phosphate-buffered saline, and a sodium acetate buffer solution. Examples of the additive include D-sorbitol, D-mannose, D-mannitol, sodium chloride, and further a nonionic surfactant at a low concentration, and polyoxyethylene sorbitan fatty acid ester.

The above carrier is used to avoid or decrease degradation of the double-stranded nucleic acid complex, which is an active ingredient, in vivo by an enzyme and the like, and additionally to facilitate formulation or administration, and to maintain the dosage form and drug efficacy. Therefore, it may be used as appropriate and as needed.

### 3-2-3. Dosage form

There is no particular restriction on the dosage form of the composition of the present invention as long as the nucleic acid molecule or the double-stranded nucleic acid complex, which is an active ingredient, is delivered to a target site without being inactivated by degradation or the like, and the pharmacological effect (an antisense effect on the expression of a target gene) of the active ingredient can be produced in vivo.

When the composition of the present invention is intrathecally administered, the specific dosage form can be a dosage form appropriate for intrathecal administration.

The preferred dosage form is liquid formulation which can be intrathecally administered. Examples of the liquid formulation comprise an injectable. An injectable can be formulated by mixing in an appropriate combination with the aforedescribed excipient, elixir, emulsifier, suspending agent, surfactant, stabilizer, pH adjuster, etc. in the form of a unit dose required according to the generally approved pharmaceutical practices.

There is no particular restriction on the specific shape and size of each of the above-mentioned dosage forms, as long as the respective dosage forms are within the ranges of dosage forms publicly known in the art. As for the manufacturing method of the composition of the present invention, it may be formulated according to the common procedure in the art.

In a specific embodiment, a nucleic acid molecule or double-stranded nucleic acid complex of the present invention has excellent quality as a pharmaceutical product having the following: excellent solubility in water, the second fluid for the dissolution test in accordance with the Japanese Pharmacopoeia, or the second fluid for the disintegration test in accordance with the Japanese Pharmacopoeia; excellent pharmacokinetics (e.g., the drug half-life in the blood, intracerebral transitivity, metabolic stability, and CYP inhibition); low toxicity (e.g., superior as a pharmaceutical product from the viewpoint of acute toxicity, chronic toxicity, genotoxicity, neurotoxicity, reproductive toxicity, cardiotoxicity, drug interaction, carcinogenicity, cell toxicity, and the like); less side effects (e.g., suppression of sedation and avoidance of laminar necrosis); and the like.

### 3-3. Dosing form and dose

There is no particular restriction on dosing form of a composition of the present aspect. The administration may be systemic or local. The administration route may be oral or parenteral administration. Specific examples of the parenteral administration include intrathecal administration (intraventricular administration, suboccipital puncture, or lumbar puncture), transnasal administration, intravenous administration, intraarterial administration, administration by blood transfusion, intraperitoneal administration, intraocular administration, intramuscular administration, subcutaneous administration (comprising implantable continuous subcutaneous administration), intradermal administration, intravesical administration, intravaginal administration, rectal administration, inhalation or intranasal administration, and trachea/bronchial administration. When the application target site of the present invention is the central nervous system, such as the brain, intrathecal administration or transnasal administration, which is advantageous for delivery to the target site, is preferred. The composition can also be delivered to the central nervous system by crossing the blood-brain barrier, e.g., by intravenous administration, subcutaneous administration, intraperitoneal administration, or intramuscular administration. The composition of the present invention can be intrathecally administered. The intrathecal administration is, e.g., intraventricular administration, suboccipital puncture or lumbar puncture. In addition, for intrathecal administration, the composition of the present invention may be administered using a shunt, an indwelling catheter, or a subcutaneous port.

When the composition of the present invention is intrathecally administered, the double-stranded nucleic acid complex may be administered to a monkey or a human in an amount of 0.01 mg or more, 0.1 mg or more, or 1 mg or more, e.g., 2 mg or more, 3 mg or more, 4 mg or more, 5 mg or more, 10 mg or more, 20 mg or more, 30 mg or more, 40 mg or more, 50 mg or more, 75 mg or more, 100 mg or more, 200 mg or more, 300 mg or more, 400 mg or more, or 500 mg or more, or may be administered in an amount of 0.01 mg to 1000 mg, 0.1 mg to 200 mg, or 1 mg to 20 mg. The double-stranded nucleic acid complex may be administered to a mouse in an amount of 1 µg or more.

The dose of the composition may be, e.g., from 0.00001 mg/kg/day to 10000 mg/kg/day, or from 0.001 mg/kg/day to 100 mg/kg/day for the nucleic acid molecule or double-stranded nucleic acid complex comprised in the composition. The administration of the composition may be single-dose administration or multiple dose administration. In the case of multiple dose administration, it may be administered daily or at appropriate time intervals (e.g., at intervals of one day, two days, three days, one week, two weeks, or one month), e.g., for 2 to 20 times. A single dose of the nucleic acid molecule or the double-stranded nucleic acid complex described above may be, e.g., 0.001 mg/kg or more, 0.005 mg/kg or more, 0.01 mg/kg or more, 0.25 mg/kg or more, 0.5 mg/kg or more, 1.0 mg/kg or more, 2.0 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any amount in the range of from 0.001 mg/kg to 500 mg/kg (e.g., 0.001 mg/kg, 0.01 mg/kg, 0.1 mg/kg, 1 mg/kg, 5 mg/kg, 10 mg/kg, 50 mg/kg, 100 mg/kg, or 200 mg/kg) may be selected as appropriate.

The nucleic acid molecule or the double-stranded nucleic acid complex in the composition of the present invention may be administered twice a week for total four times at a dose of from 0.01 to 10 mg/kg (e.g., about 6.25 mg/kg). Alternatively, the nucleic acid molecule or the double-stranded nucleic acid complex may be administered once or twice a week for total two to four times, e.g., at a frequency of twice a week for total two times, at a dose of from 0.05 to 30 mg/kg (e.g., about 25 mg/kg). By adopting such a dosing regimen (divided administration), the toxicity can be lowered (e.g., avoidance of platelet reduction) compared to a single-dose administration at a higher dose, and the stress to the subject can be reduced.

Even when the pharmaceutical composition is repeatedly administered, its inhibitory effect can be produced additively in a cell. In the case of repeated administration, the efficacy can be improved with certain administration intervals (e.g., half a day or longer).

### 3-4. Disease as subject of application

A disease to which the pharmaceutical composition is, e.g., a central nervous system disease. The disease may be a disease which may be related to a gene for which the antisense effect of a nucleic acid molecule or double-stranded nucleic acid complex of the present invention can suppress or increase the expression amount of a transcription product or translation product or can inhibit the function of a transcription product or translation product of the gene, or can induce steric blocking, splicing switching, RNA editing, exon skipping, or exon inclusion.

The compositions can be used in an animal comprising a human as a subject. However, there is no particular restriction on the animal other than a human, a variety of domestic animals, domestic fowls, pets, laboratory animal, and the like can be subjects of some embodiments. The subject may be a subject in need of a decrease in the expression amount of a target transcription product in the central nervous system. The subject may also be a subject in need of treatment of a central nervous system disease.

A disease to be treated can be a central nervous system disease associated with increased or decreased gene expression, particularly a disease (such as a tumor) associated with increased expression of a target transcription product or a target gene. Examples of the central nervous system disease include, but are not particularly limited to, brain tumor, Alzheimer's disease, Parkinson's disease, amyotrophic lateral sclerosis, multiple sclerosis, and Huntington's disease.

The nervous system can be divided into the central nervous system and the peripheral nervous system. The central nervous system consists of the brain and the spinal cord. The brain comprises the cerebrum (cerebral cortex, cerebral white matter, and basal ganglion), the diencephalon (thalamus and subthalamic nucleus), the cerebellum (cerebellar cortex and cerebellar nucleus), and the brainstem (midbrain, substantia nigra, pons, and medulla oblongata). The spinal cord comprises the cervical cord, the thoracic cord, the lumbar cord, the sacral cord, and the coccygeal cord. Although the central nervous system herein may be any of these regions, it may be particularly the cerebral cortex (frontal lobe, temporal lobe, parietal lobe, or occipital lobe), the cerebellum, the striatum, the globus pallidus, the claustrum, the hippocampus, the parahippocampal gyrus, the brainstem, the cervical cord, the thoracic cord, or the lumbar cord. The peripheral nerve consists of the cranial nerve, and the spinal nerve.

For example, in a treatment of Alzheimer's disease, drug delivery to the hippocampus and/or the parietal lobe may be effective. In a treatment of frontotemporal dementia (FTD) (frontotemporal lobar degeneration (FTLD), semantic dementia (SD), or progressive nonfluent aphasia (PNFA)), and Pick disease, drug delivery to the frontal lobe, the temporal lobe, and/or the substantia nigra may be effective. In a treatment of Parkinson's disease dementia, drug delivery to the occipital lobe, the substantia nigra, and/or the striatum may be effective. In a treatment of Parkinson's disease, drug delivery to the substantia nigra and/or the striatum may be effective. In a treatment of corticobasal degeneration (CBD), drug delivery to the frontal lobe, the parietal lobe, the basal ganglion, and/or the substantia nigra may be effective. In a treatment of progressive supranuclear palsy (PSP), drug delivery to the frontal lobe, the basal ganglion, and/or the substantia nigra may be effective. In a treatment of amyotrophic lateral sclerosis, drug delivery to the frontal lobe, the parietal lobe, the basal ganglion, and/or the substantia nigra may be effective. In a treatment of spinocerebellar degeneration (SCD) SCA1 type through SCA34 type, drug delivery to the brainstem and/or the cerebellum may be effective. In a treatment of dentatorubral-pallidoluysian atrophy (DRPLA), drug delivery to the basal ganglion, the brainstem, and/or the cerebellum may be effective. In a treatment of spinal and bulbar muscular atrophy (SBMA), drug delivery to the brainstem and/or the spinal cord may be effective. In a treatment of Friedreich's ataxia (FA), drug delivery to the brainstem and/or the cerebellum may be effective. In a treatment of Huntington's disease, drug delivery to the striatum, the frontal lobe, the parietal lobe, and/or the basal ganglion may be effective. In a treatment of a prion disease (mad cow disease, GSS), drug delivery to the cerebral cortex, the cerebral white matter, the basal ganglion, and/or the substantia nigra may be effective. In the treatment of cerebral white matter encephalopathy, drug delivery to the cerebral white matter may be effective. In a treatment of encephalitis (viral, bacterial, fungal, or tuberculous) or meningitis (viral, bacterial, fungal, or tuberculous), drug delivery to the whole brain may be effective. In a treatment of metabolic encephalopathy, toxic encephalopathy, trophopathic encephalopathy, drug delivery to the whole brain may be effective. In a treatment of cerebral white matter encephalopathy, drug delivery to the cerebral white matter may be effective. In a treatment of cerebral infarction, cerebral hemorrhage, subarachnoid hemorrhage, moyamoya disease, anoxic encephalopathy, drug delivery to the whole brain may be effective. In a treatment of cerebral white matter encephalopathy, drug delivery to the cerebral white matter may be effective. In a treatment of diffuse axonal injury, drug delivery to the cerebral white matter may be effective. In a treatment of head trauma, drug delivery to the whole brain may be effective. In a treatment of multiple sclerosis (MS) or neuromyelitis optica (NMO), drug delivery to the cerebral white matter, the cerebral cortex, the optic nerve, and the spinal cord may be effective. In a treatment of myotonic dystrophy (DM1, DM2), drug delivery to the skeletal muscle, the cardiac muscle, the cerebral cortex, and/or the cerebral white matter may be effective. In a treatment of hereditary spastic paraparesis (HSP), drug delivery to the parietal lobe and/or the spinal cord may be effective. In a treatment of Fukuyama muscular dystrophy, drug delivery to the skeletal muscle, the cerebral cortex, and/or the cerebral white matter may be effective. In a treatment of dementia with Lewy bodies (DLB), drug delivery to the substantia nigra, the striatum, the occipital lobe, the frontal lobe, and/or the parietal lobe may be effective. In a treatment of multiple system atrophy (MSA), drug delivery to the striatum, the basal ganglia, the cerebellum, the substantia nigra, the frontal lobe, and/or the temporal lobe may be effective. In a treatment of Alexander' disease drug delivery to the cerebral white matter may be effective. In a treatment of CADASIL and CARASIL, drug delivery to the cerebral white matter may be effective.

### 3-5. Effects

The composition of the present invention can achieve a preventive effect or a therapeutic effect on central nervous system diseases when intrathecally administered.

In addition, the present invention also provides a method for treating and/or preventing a disease such as a central nervous system disease, comprising administering the above-described composition to a subject.

### Examples

The present invention is described below in more detail by means of Examples. However, the technical scope of the present invention is not limited to these Examples.

<Example 1: Evaluation of toxicity of Mapt targeted ASO with 5'-CP modification introduced into gap region>

### (Purpose)

A 5'- cyclopropylene modification (hereinafter abbreviated as a "5'-CP modification") is introduced into a gap region of a gapmer antisense nucleic acid (hereinafter referred to as "ASO") targeting the Mapt gene. Through an in vitro experiment, the toxicity-reducing effect based on 5'-CP modifications is investigated.

### (Method)

### (1) Preparation of nucleic acids

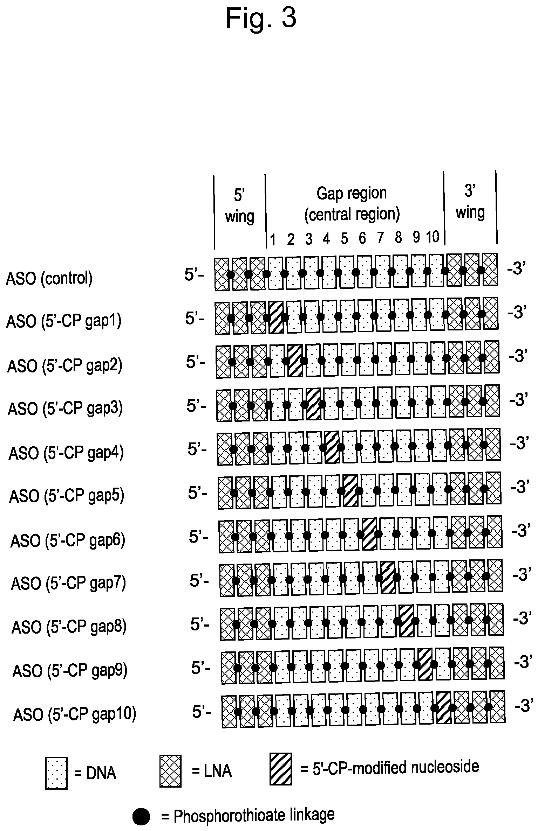

The base sequences and chemical modifications of ASOs used in this Example are shown in Table 1 and Figure 3.

**[Table 1]**

| **Table 1: ASO with 5'-CP modification introduced into the gap region** | | |
|---|---|---|
| | Sequence (5'-3') | SEQ ID NO |
| ASO(control) | a^t^t^t^c^c^a^a^a^t^t^c^a^c(5)^t^t | 1 |
| ASO(5'-CP gap1) | a^t^t^t^{cP}^c^c^a^a^a^t^t^c^a^c(5)^t^t | 2 |
| ASO(5'-CP gap2) | a^t^t^t^c(5)^{CP}^c^a^a^a^t^t^c^a^c(5)^t^t^ | 3 |
| ASO(5'-CP gap3) | a^t^t^t^c^c^(5)^{CP}^c^a^a^a^t^t^c^a^c(5)^t^t^ | 4 |
| ASO(5'-CP gap4) | a^t^t^t^c^a^{CP}^a^a^a^t^t^c^a^c(5)^t^t^ | 5 |
| ASO(5'-CP gap5) | a^t^t^t^c^c^a^a^^{CP}^a^t^t^c^a^c(5)^t^t^ | 6 |
| ASO(5'-CP gap6) | a^t^t^t^c^c^a^a^a^^{CP}^t^t^c^a^c(5)^t^t | 7 |
| ASO(5'-CP gap7) | a^t^t^t^c^c^a^a^a^t^{CP}^t^c^a^c(5)^t^t^ | 8 |
| ASO(5'-CP gap8) | a^t^t^t^c^c^a^a^a^t^t^{CP}^c^a^c(5)^t^t^ | 9 |
| ASO(5'-CP gap9) | a^t^t^t^c^c^a^a^a^t^t^c^(5)^{CP}^a^c(5)^t^t | 10 |
| ASO(5'-CP gap10) | a^t^t^t^c^c^a^a^a^t^t^c^a^{CP}^c(5)^t^t | 11 |

| | | |
|---|---|---|
| Lower case letter: DNA; Underlined lower case letter: LNA; Superscript "CP": 5'-CP modification; ^: PS linkage; c(5) represents that the base moiety is 5-methylcytosine. | | |

The ASO (control) used in this Example is an LNA/DNA gapmer ASO targeting a microtubule-associated protein tau (Mapt) mRNA of a mouse, has a base sequence complementary to a part of the Mapt mRNA, and has a structure wherein three LNA nucleosides at the 5' end, three LNA nucleosides at the 3' end, and ten DNA nucleosides therebetween are linked via a phosphorothioate linkage. The three LNA nucleosides at the 5' end correspond to the 5'-wing region of the gapmer ASO, the three LNA nucleosides at the 3' end correspond to the 3'-wing region, and ten DNA nucleosides therebetween correspond to the gap region (central region).

ASO(5'-CP gap1) to ASO(5'-CP gap10) used in this Example comprise a 5'-cyclopropylene-modified nucleoside (5'-CP-modified nucleoside) of the following formula (I) at the 1st to 10th base position, respectively, from the 5' side of the gap region of ASO (control). 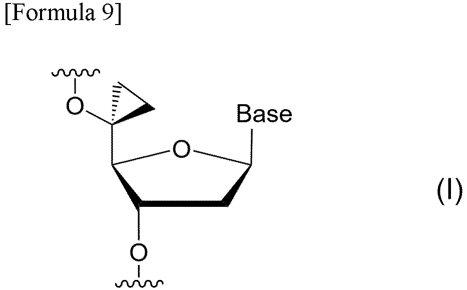 The 5'-cyclopropylene modification refers to a modification in which a cyclopropane group is bound to carbon at the 5' position of ribose. In the following Examples, a base position located on the most 5' side in the gap region is referred to as "gap 1", and a base position located at the Nth position in the 3' direction is referred to as "gap N". Therefore, ASO (5'-CP gap1) to ASO (5'-CP gap10) have nucleosides located at gap 1 to gap 10, respectively, with a 5'-CP modification.

ASOs used in the following Examples were synthesized by GeneDesign Inc. (Osaka, Japan) on consignment.

### (2) Evaluation of cell toxicity

The ASO prepared in (1) was introduced into a mouse neuroblastoma-derived cells (Neuro 2a cell line) at 50 nM using a lipofection method (lipofectamine 2000). To evaluate the neurotoxicity at 72 hours after the introduction, the number of viable cells, the lactate dehydrogenase (LDH) activity in the cell supernatant, and the expression amounts of Cdkn1a and Il-6 mRNAs were measured.

The number of viable cells was measured using Scepter 3.0 (Millipore) after aspiration of the medium to prepare a cell solution.

The LDH activity was measured using Cytotoxicity LDH Assay Kit-WST (Dojindo Laboratories) in accordance with the attached protocol. A value standardized with respect to the LDH activity of a PBS-treated group was determined as a relative LDH release level.

The expression amounts of Cdkn1a mRNA and Il-6 mRNA were measured according to the following procedure. Using an IsogenI kit (GeneDesign Inc.), RNA was extracted from the cell solution after measurement of the number of cells. A cDNA was synthesized using Transcriptor Universal cDNA Master, DNase (Roche Diagnostics) in accordance with the protocol. Next, the resulting cDNA template was used to perform quantitative RT-PCR, whereby the expression amounts of Cdkn1a mRNA, Il-6 mRNA, and Gapdh mRNA (the internal standard gene) were measured. Quantitative RT-PCR was performed with TaqMan (Roche Applied Science). Primers used in quantitative RT-PCR were products designed and manufactured by Thermo Fisher Scientific Inc. (formerly known as Life Technologies Corp.). Amplification was performed by repeating, 40 times, the following cycle: 95°C for 15 seconds, 60°C for 30 seconds, and 72°C for 1 second.

The ratio of the expression amounts of Cdkn1a mRNA and Il-6 mRNA to the expression amount of Gapdh mRNA (internal standard gene) was calculated, and values standardized with respect to the value of the PBS-treated group were determined as expression levels of Cdkn1a mRNA and Il-6 mRNA.

### (Results)

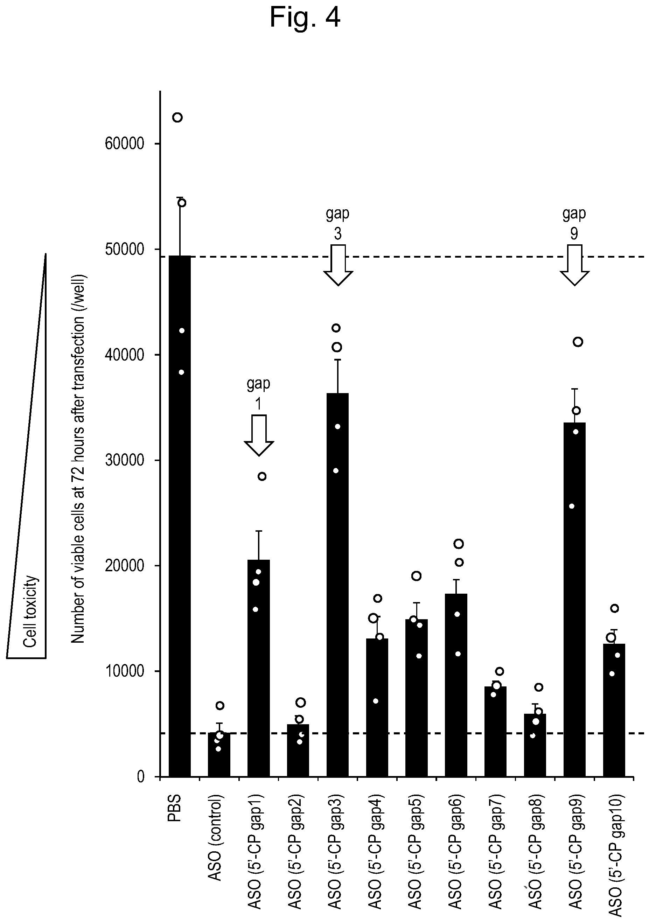

Figure 4 shows the results of measurement of the number of viable cells after transfection with PBS or various nucleic acid agents as the cell toxicity to the Neuro-2a cell line. The number of viable cells decreased in the ASO (control)-treated group, compared with PBS-treated group, indicating cell toxicity. Compared with this, in the administration groups of ASO (5'-CP gap1) to ASO (5'-CP gap10), the administration groups of ASO (5'-CP gap1), ASO (5'-CP gap3), and ASO (5'-CP gap9) exhibited a higher number of viable cells. This result has revealed that introducing a 5'-CP modification at particular base positions (gap1, gap3, and gap9) in the gap region significantly reduces cell toxicity.

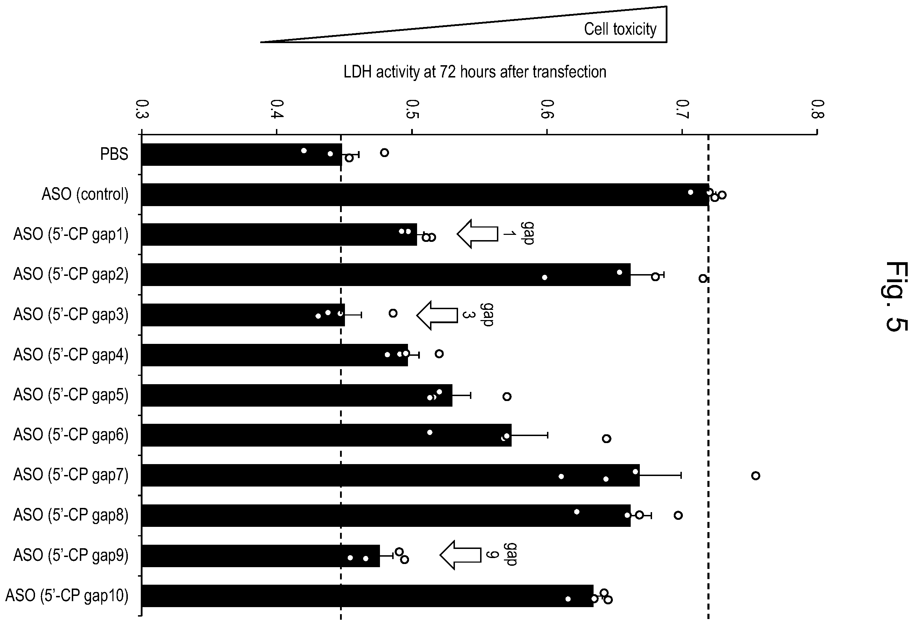

Figure 5 shows the results of measurement of the LDH activity in the supernatant as the cell toxicity when various nucleic acid agents were introduced into the cell. The LDH activity was increased in the ASO (control)-treated group, indicating cell toxicity. Compared with this, in the administration groups of ASO (5'-CP gap1) to ASO (5'-CP gap10), ASO (5'-CP gap1), ASO (5'-CP gap3), and ASO (5'-CP gap9) exhibited lower LDH activity and the effect of reducing cell toxicity.

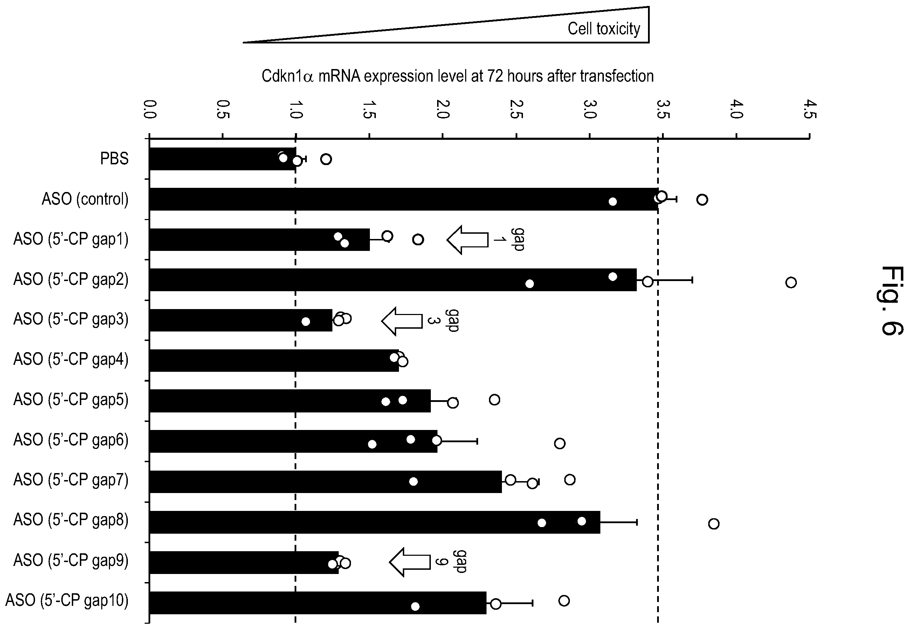

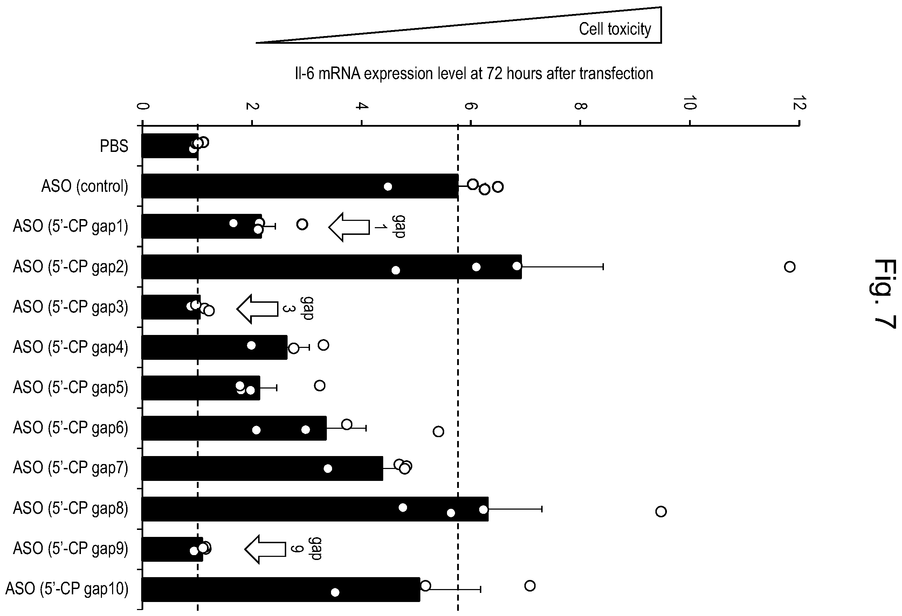

Figures 6 and 7 show the results of evaluation of the expression levels of Cdkn1a mRNA and Il-6 mRNA as the cell toxicity when various nucleic acid agents were introduced into the cell. The expression levels of both were increased in the ASO (control)-treated group, indicating cell toxicity. Compared with this, in the administration groups of ASO (5 '-CP gap1) to ASO (5'-CP gap10), ASO (5'-CP gap1), ASO (5'-CP gap3), and ASO (5'-CP gap9) showed reduced expression of both, exhibiting the effect of reducing cell toxicity.

### <Example 2: Evaluation of toxicity of Mapt targeted ASO with 5'-CP modification and 2'-O-methyl modification introduced into gap region>

### (Purpose)

A 5'-CP modification and a 2'-O-methyl modification (hereinafter referred to as a "2'-OMe modification") are introduced into a gap region of a gapmer ASO targeting the Mapt gene, and the toxicity-reducing effect is investigated through an in vitro experiment.

### (Method)

### (1) Preparation of nucleic acids

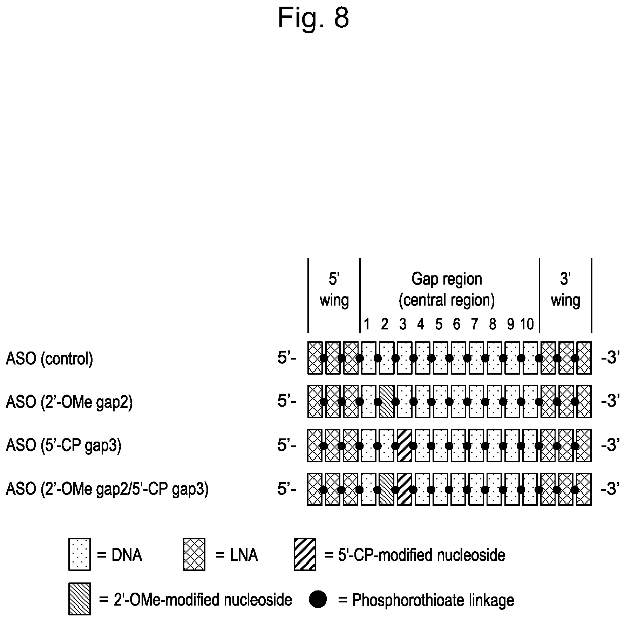

The base sequences and chemical modifications of ASOs used in this Example are shown in Table 2 and Figure 8.

**[Table 2]**

| **Table 2: ASO with 5'-CP modification and 2'-OMe modification introduced into the gap region** | | |
|---|---|---|
| | Sequence (5'-3') | SEQ ID NO |
| ASO(control) | a^t^t^t^c^c^a^a^a^t^t^c^a^c(5)^t^t | 1 |
| ASO(5'-CP gap3) | a^t^t^t^c^c^{CP}^a^a^a^t^t^c^a^c(5)^t^t^ | 4 |
| ASO(2'-OMe gap2) | a^t^t^t^C(M)^c^a^a^a^t^t^c^a^c(5)^t^t^ | 12 |
| ASO(2'-OMe gap2 /5'-CP gap3) | a^t^t^t^C(M)^c^{CP}^a^a^a^t^t^c^a^c(5)^t^t^ | 13 |

| | | |
|---|---|---|
| Lower case letter: DNA; Underlined lower case letter: LNA; Superscript "CP": 5'-CP modification; Upper case letter (M): 2'-O-Me-RNA; ^: PS linkage; c(5) represents that the base moiety is 5-methylcytosine. | | |

ASO (control) and ASO (5'-CP gap3) used in this Example were the same as in Example 1.

ASO (2'-OMe gap2) and ASO (2'-OMe gap2/5'-CP gap3) used in this Example each have a 2'-OMe-modified nucleoside (a ribonucleoside in which the hydroxy group at the 2' position of ribose is substituted with a methoxy group) at the 2nd base position (gap2) from the 5' side of the gap region of ASO (control) and ASO (5'-CP gap3).

### (2) Evaluation of cell toxicity

The ASO prepared in (1) was introduced into a mouse neuroblastoma-derived cells (Neuro 2a cell line) at 50 nM using a lipofection method (lipofectamine 2000). To evaluate the neurotoxicity at 72 hours after the introduction, the number of viable cells and the lactate dehydrogenase (LDH) activity in the cell supernatant were measured by the same methods as in Example 1.

### (Results)

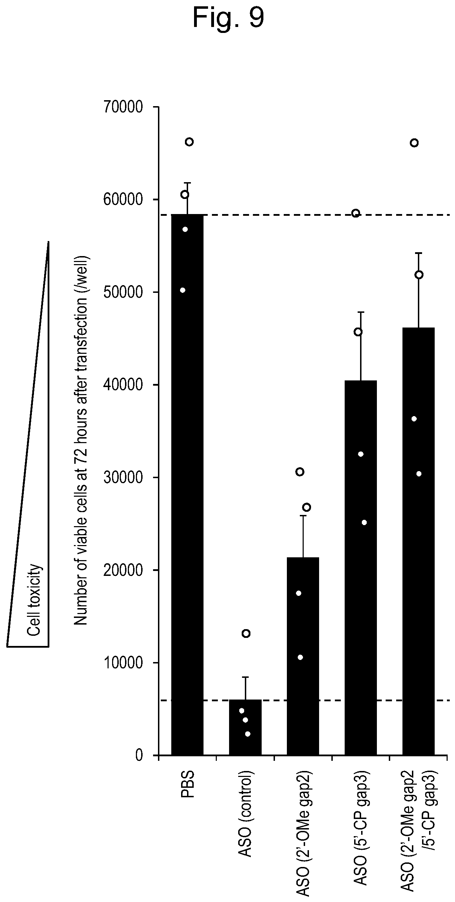

Figure 9 shows the results of measurement of the number of viable cells after transfection with PBS or various nucleic acid agents as the cell toxicity to the Neuro-2a cell line. The number of viable cells decreased in the ASO (control)-treated group, compared with PBS-treated group, exhibiting cell toxicity. In contrast, the number of viable cells increased in the ASO (2'-OMe gap2)-treated group, compared with the ASO (control)-treated group, indicating a reduction in cell toxicity. The number of viable cells was larger in the ASO (5'-CP gap3)-treated group than in the ASO (2'-OMe gap2)-treated group, indicating a further reduction in toxicity. The number of viable cells was larger in the ASO (2'-OMe gap2/5'-CP gap3)-treated group in which two modifications were combined than that in the ASO (5'-CP gap3)-treated group, indicating a remarkable toxicity-reducing effect.

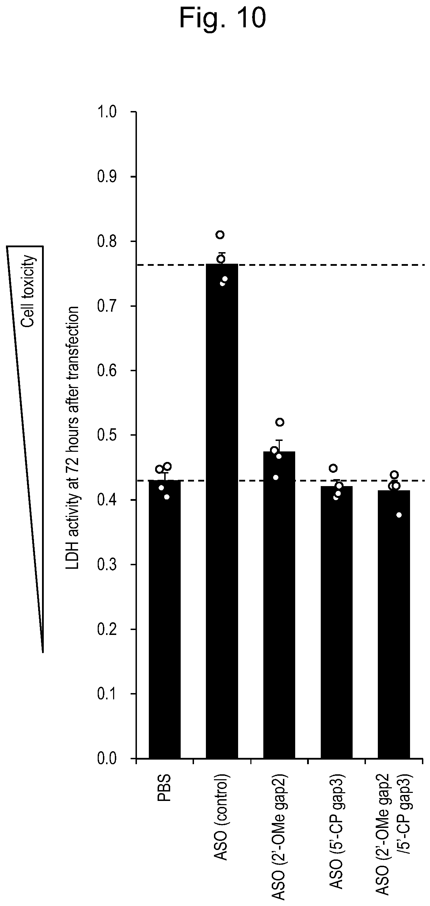

Figure 10 shows the results of measurement of the LDH activity in the supernatant as the cell toxicity when various nucleic acid agents were introduced into the cell. The LDH activity was increased in the ASO (control)-treated group, indicating cell toxicity. Compared with this, the LDH activity in the ASO (2'-OMe gap2)-treated group, ASO (5'-CP gap3)-treated group, and ASO (2'-OMe gap2/5'-CP gap3)-treated group decreased to the same level as that in the PBS-treated group, indicating the effect of significantly reducing cell toxicity.

The above-described results have revealed that a 5 -CP modification at the 3rd base position (gap 3) from the 5' side of the gap region can reduce cell toxicity more than introducing a 2'-OMe modification at the 2nd base position (gap 2) from the 5' side of the gap region, and combining both modifications can further reduce cell toxicity.

### <Example 3: Evaluation of in vivo toxicity of Mapt targeted ASO with 5'-CP modification and 2'-O-methyl modification introduced into gap region>

### (Purpose)

In terms of central nervous system toxicity exhibited when a gapmer ASO targeting the Mapt gene is intraventricularly administered, the toxicity-reducing effect of introducing a 5'-CP modification and a 2'-OMe modification into the gap region is investigated through an in vivo experiment.

### (Method)

### (1) Preparation of nucleic acids

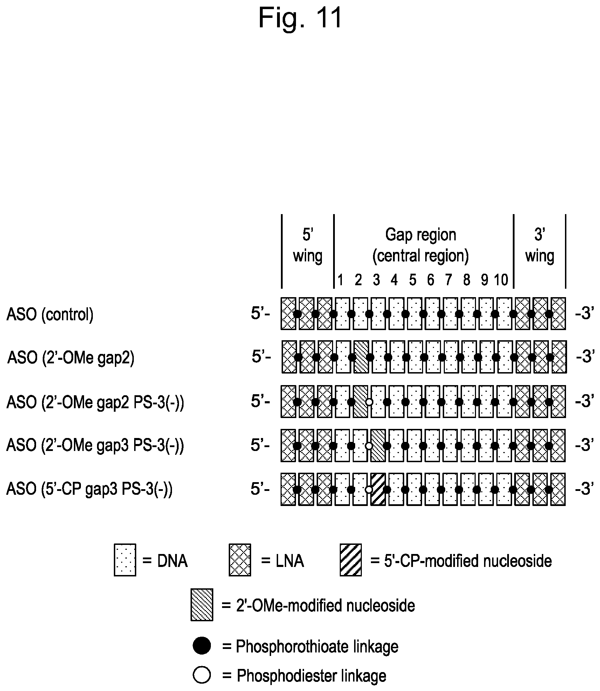

The base sequences and chemical modifications of the ASOs used in this Example are shown in Table 3 and Figure 11.

**[Table 3]**

| **Table 3: ASO with 5'-CP modification and 2'-OMe modification introduced into the gap region** | | |
|---|---|---|
| | Sequence (5'-3') | SEQ ID NO |
| ASO(control) | a^t^t^t^c^c^a^a^a^t^t^c^a^c(5)^t^t^ | 1 |
| ASO(2'-OMe gap2) | a^t^t^t^C(M)^c^a^a^a^t^t^c^a^c(5)^t^t | 12 |
| ASO(2'-OMe gap2 PS-3(-)) | a^t^t^t^C(M)*c^a^a^a^t^t^c^a^c(5)^t^t | 14 |
| ASO(2'-OMe gap3 PS-3(-)) | a^t^t^t^c*C(M)^a^a^a^t^t^c^a^c(5)^t^t | 15 |
| ASO(5'-CP gap3 PS-3(-)) | a^t^t^t^c*c(5)^{CP}a^a^a^t^t^c^a^c(5)^t^t | 16 |

| | | |
|---|---|---|
| Lower case letter: DNA; Underlined lower case letter: LNA; Superscript "CP": 5'-CP modification; Upper case letter (M): 2'-O-Me-RNA; ^: PS linkage; *: PO linkage; c(5) represents that the base moiety is 5-methylcytosine. | | |

ASO (control) and ASO (2'-OMe gap2) used in this Example were the same as in Examples 1 and 2.

ASO (2'-OMe gap PS-3(-)), ASO(2'-OMe gap3 PS-3(-)), and ASO(5'-CP gap3 PS-3(-)) used in this Example have a 2'-OMe modified nucleoside, a 5'-CP modified nucleoside, and a 5'-CP modified nucleoside at the 2nd (gap 2), 3rd (gap 3), 3rd (gap 3) base positions from the 5' side of the gap region of the ASO (control), respectively, and an internucleoside linkage between gap 2 and gap 3 is a phosphodiester linkage (PO linkage) in all cases.

### (2) In vivo experiment

Seven-week-old female ICR mice anesthetized with 2.5 to 4% isoflurane were fixed to a brain stereotaxic apparatus. Then, skin between the ears was cut open 2 to 3 cm anteroposteriorly, and perforated 1 mm leftward and 0.2 mm posteriorly from the bregma using a 1 mm diameter drill. A Hamilton syringe was filled with the nucleic acid agent. Through the perforated site, a needle was inserted 3 mm deep over 10 seconds using MDS-1 One-axis Motorized Stereotaxic Micromanipulator (NARISHIGE). Then, the nucleic acid agent at a dose of 38.4 nmol per mouse was administered into the left lateral ventricle at a rate of 5 µl/min (n=4). The skin was sutured with a nylon thread. In addition, a mouse to which only PBS was administered was provided as a negative control group.

### (3) Evaluation of delayed central nervous system toxicity after administration of nucleic acid agents

Evaluation of delayed central nervous system toxicity and evaluation of motor function were performed through a measurement of body weight/food intake and an open-field test, respectively, on mice to which various nucleic acid agents were administered.

The body weight/food intake was measured at 20 days after the administration of the nucleic acid agents.

Evaluation of motor function was performed at 10, 14, 17, and 20 days after the administration of the nucleic acid agents. Specifically, a mouse was placed in the center of a cage (50 cm in width × 50 cm in diameter × 40 cm in height), and the track of the mouse movement was recorded for 5 minutes. The maximum moving speed (m/s) based on the data recorded were measured using video tracking software (ANY-maze).

### (4) Evaluation of gene suppression effect

A lumbar spinal cord was extirpated from the mice at 21 days after the administration of various nucleic acid agents. Using an IsogenI kit (GeneDesign Inc.), RNA was extracted from the lumbar cord extirpated. A cDNA was synthesized using Transcriptor Universal cDNA Master, DNase (Roche Diagnostics) in accordance with the protocol.

Next, the resulting cDNA template was used to perform quantitative RT-PCR, whereby the expression levels of Mapt mRNA and Actb mRNA (the internal standard gene) were measured. Quantitative RT-PCR was performed with TaqMan (Roche Applied Science). Primers used in quantitative RT-PCR were products designed and manufactured by Thermo Fisher Scientific Inc. (formerly known as Life Technologies Corp.). Amplification conditions (temperature and time) were as follows: the cycle, 95°C for 15 seconds, 60°C for 30 seconds, and 72°C for 1 second, was repeated 40 times.

The ratio of the expression level of Mapt mRNA to the expression level of Actb mRNA (internal standard gene) was calculated, and a value standardized with respect to the value of the PBS-treated group was determined as a relative Mapt mRNA level.

### (Results)

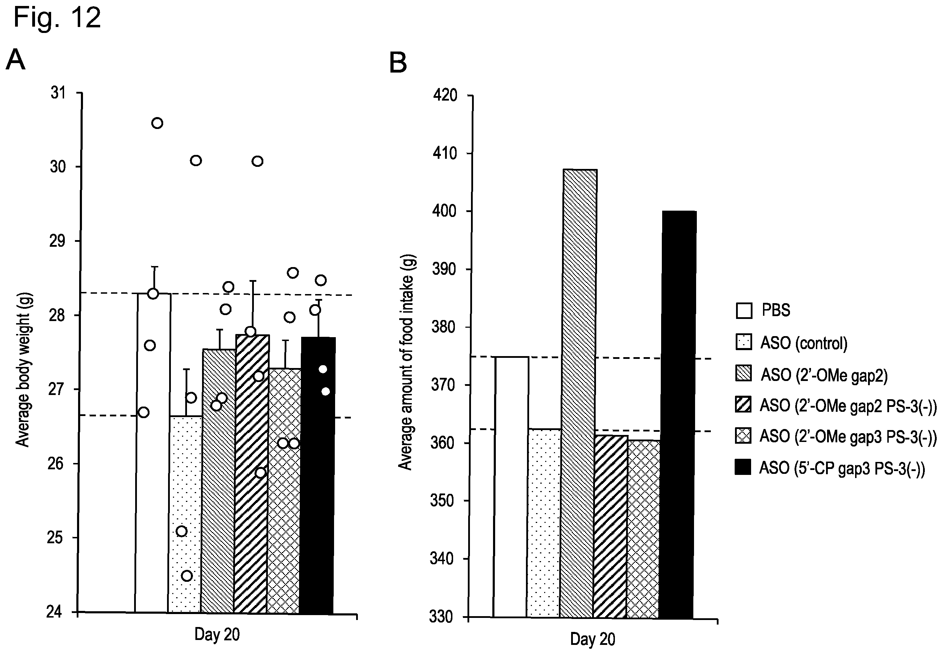

Figure 12 shows the results of measurement of the body weight of mice/their food intake at 20 days after various nucleic acid agents were intraventricularly administered to the mice. The body weight/food intake was lower in the ASO (control)-treated group than in the PBS-treated group, indicating central nervous system toxicity. The body weight was higher in the ASO (2'-OMe gap2 PS-3(-))-treated group and the ASO (2'-OMe gap3 PS-3(-))-treated group than in the ASO (control)-treated group, but there was no difference in food intake. Compared with this, both the body weight and the food intake were higher in the ASO (5'-CP gap3 PS-3(-))-treated group than in the ASO (control)-treated group.

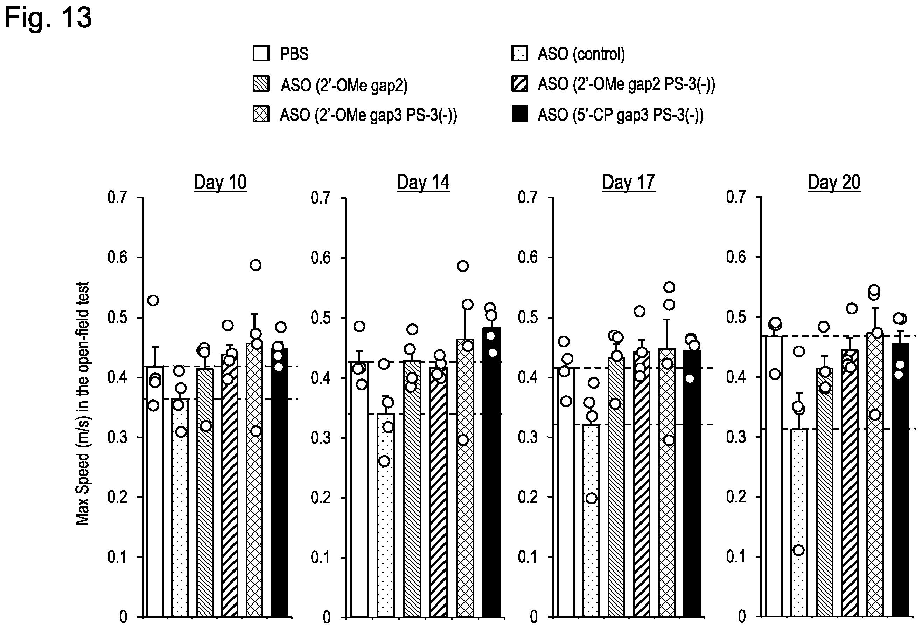

Figure 13 shows the results of evaluation of the motor function of mice at 10, 14, 17, and 20 days after various nucleic acid agents were intraventricularly administered to the mice. The maximum moving speed was higher in the ASO (5'-CP gap3 PS-3(-))-treated group than in the ASO (control)-treated group, resulting in a reduced delayed neurotoxicity.

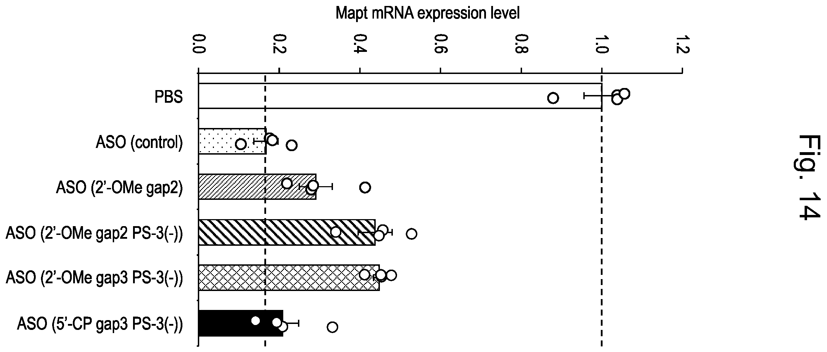

Figure 14 shows the Mapt mRNA expression level in the lumbar spinal cord 21 days after the intraventricular administration of various nucleic acid agents. The gene suppression effect was decreased with the administration groups of ASO (2'-OMegap2), ASO (2'-OMe gap2 PS-3(-)), and ASO (2'-OMegap3 PS-3(-)), compared with the administration group of ASO (control). On the other hand, the ASO (5'-CP gap3 Ps-3(-))-treated group exhibited the same gene suppression effect as the ASO (control)-treated group.

The above-described results have revealed that ASO (5'-CP gap3 PS-3 (-)) has a similar reduction in delayed toxicity as ASO (2'-OMegap2), lower delayed toxicity than ASO (2'-OMe gap2 PS-3 (-)) and ASO (2'-OMegap3 PS-3 (-)), and a higher gene suppression effect than ASO (2'-OMe gap2), ASO (2'-OMegap2 PS-3 (-)), and ASO (2'-OMe gap3 PS-3 (-)).

### <Example 4: Evaluation of toxicity of Snca targeted ASO with 5'-CP modification introduced into gap region>

### (Purpose)

A 5'-CP modification is introduced into a gap region of a gapmer ASO targeting the Snca gene, and the toxicity-reducing effect is investigated through an in vitro experiment (human cells).

### (Method)

### (1) Preparation of nucleic acids

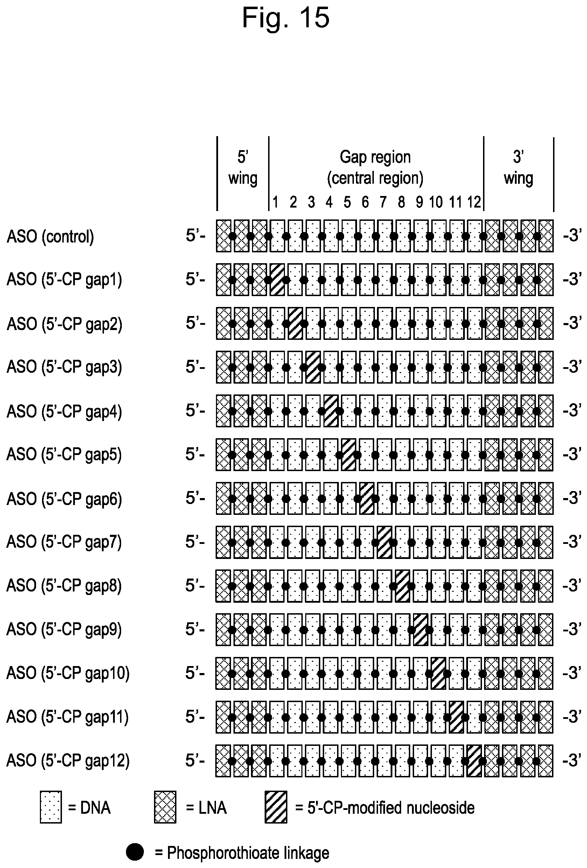

The base sequences and chemical modifications of ASOs used in this Example are shown in Table 4 and Figure 15.

**[Table 4]**

| **Table 4: ASO with 5'-CP modification introduced into the gap region** | | |
|---|---|---|
| | Sequence (5'-3') | SEQ ID NO |
| ASO(control) | c(5)^c(5)^a^t^t^t^a^t^a^t^a^c ^a^a^a^c(5)^a^c(5)^a | 17 |
| ASO(5'-CP gap1) | c(5)^c(5)^a^tCP^t^t^a^t^a^t^a ^c^a^a^a^c(5)^a^c(5)^a | 18 |
| ASO(5'-CP gap2) | c(5)^c(5)^a^t^tCP^t^a^t^a^t^a ^c^a^a^a^c(5)^a^c(5)^a | 19 |
| ASO(5'-CP gap3) | c(5)^c(5)^a^t^t^t^CP^a^t^a^t^a ^c^a^a^a^c(5)^a^c(5)^a | 20 |
| ASO(5'-CP gap4) | c(5)^c(5)^a^t^t^t^aCP^t^a^t^a ^c^a^a^a^c(5)^a^c(5)^a | 21 |
| ASO(5'-CP gap5) | c(5)^c(5)^a^t^t^t^a^tCP^a^t^a ^c^a^a^a^c(5)^a^c(5)^a | 22 |
| ASO(5'-CP gap6) | c(5)^c(5)^a^t^t^t^a^t^aCP^t^a ^c^a^a^a^c(5)^a^c(5)^a | 23 |
| ASO(5'-CP gap7) | c(5)^c(5)^a^t^t^t^a^t^a^t^CP^a ^c^a^a^a^c(5)^a^c(5)^a | 24 |
| ASO(5'-CP gap8) | c(5)^c(5)^a^t^t^t^a^t^a^t^a^CP ^c^a^a^a^c(5)^a^c(5)^a | 25 |
| ASO(5'-CP gap9) | c(5)^c(5)^a^t^t^t^a^t^a^t^a^c (5)CP^a^a^a^c(5)^a^c(5)^a | 26 |
| ASO(5'-CP gap10) | c(5)^c(5)^a^t^t^t^a^t^a^t^a^c ^aCP^a^a^c(5)^a^c(5)^a | 27 |
| ASO(5'-CP gap11) | c(5)^c(5)^a^t^t^t^a^t^a^t^a^c ^a^aCP^a^c(5)^a^c(5)^a | 28 |
| ASO(5'-CP gap12) | c(5)^c(5)^a^t^t^t^a^t^a^t^a^c ^a^a^aCP^c(5)^a^c(5)^a | 29 |

| | | |
|---|---|---|
| Lower case letter: DNA; Underlined lower case letter: LNA; Superscript "CP": 5'-CP modification; ^: PS linkage; c(5) represents that the base moiety is 5-methylcytosine. | | |

The ASO (control) used in this Example is an LNA/DNA gapmer ASO targeting an alpha-synuclein (Snca) mRNA of a mouse, has a base sequence complementary to a part of the Snca mRNA, and has a structure wherein three LNA nucleosides at the 5' end, four LNA nucleosides at the 3' end, and 12 DNA nucleosides therebetween are linked via a phosphorothioate linkage. The three LNA nucleosides at the 5' end correspond to the 5'-wing region of the gapmer ASO, the three LNA nucleosides at the 3' end correspond to the 3'-wing region, and the 12 DNA nucleosides therebetween correspond to the gap region (central region).

ASO (5'-CP gap1) to ASO (5'-CP gap12) used in this Example comprise a 5'-CP-modified nucleoside at the respective 1st to 12th base positions (gap 1 to gap 12) from the 5' side of the gap region of the ASO (control).

### (2) Evaluation of cell toxicity

The ASO prepared in (1) was introduced into a human neuroblastoma-derived cells (BE(2)-M17 cell line) at 50 nM using a lipofection method (lipofectamine 2000). To evaluate the neurotoxicity at 72 hours after the introduction, the number of viable cells, the lactate dehydrogenase (LDH) activity in the cell supernatant, and the expression amount of Cdkn1a mRNA were measured.

The number of viable cells, the LDH activity, and the expression amount of Cdkn1a mRNA were measured by the same methods as in Example 1.

### (Results)

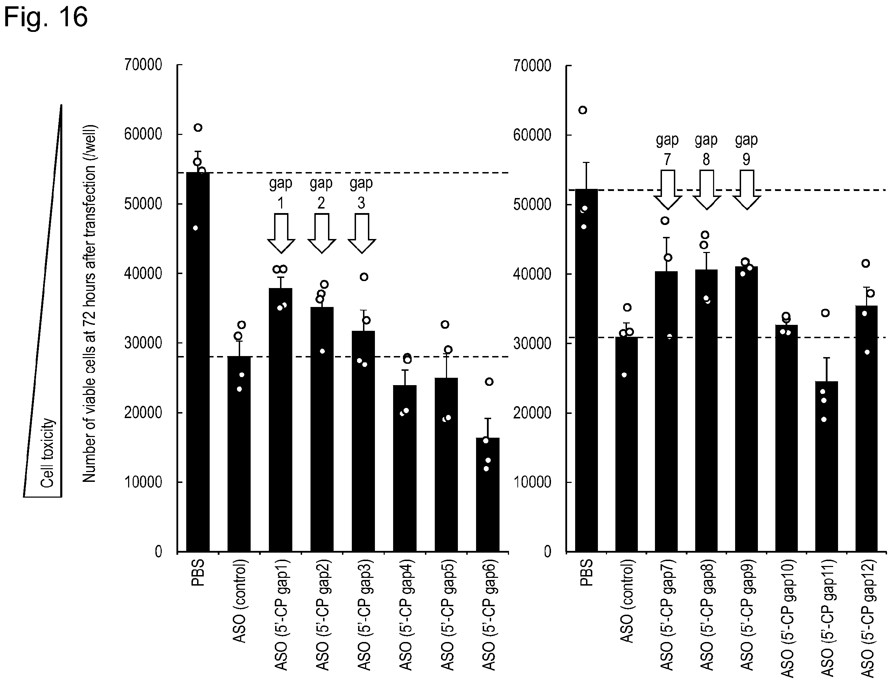

Figure 16 shows the results of evaluation of the number of viable cells as the cell toxicity to the BE(2)-M17 cell line. The number of viable cells decreased in the ASO (control)-treated group, compared with PBS-treated group, indicating cell toxicity. Compared with this, in the administration groups of ASO(5'-CP gap1) to ASO(5'-CP gap12), the number of viable cells was higher in the group treated with ASO in which 5'-CP modifications were introduced into gaps 1 to 3 and gaps 7 to 9, demonstrating the effect of reducing cell toxicity.

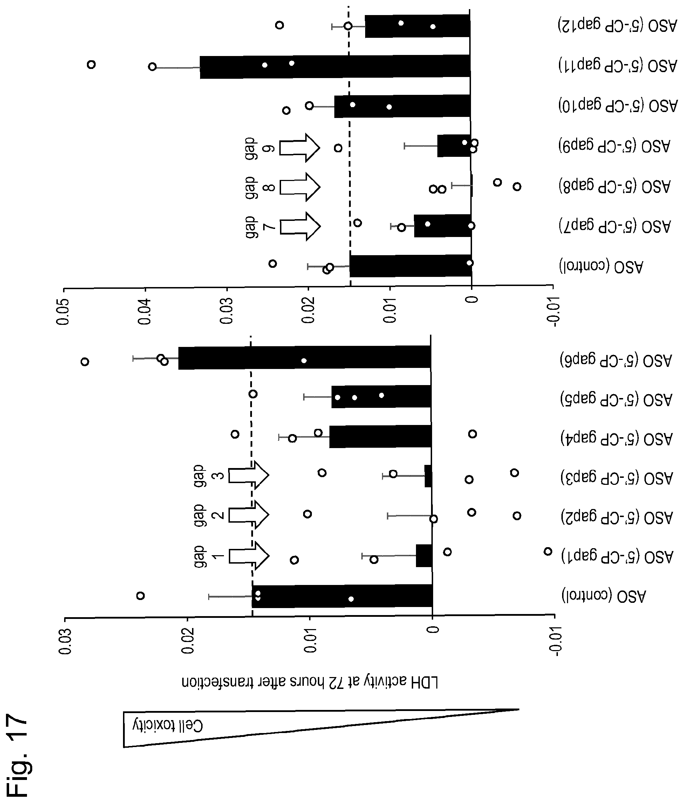

Figure 17 shows the results of evaluation of the LDH activity in the supernatant as the cell toxicity when various nucleic acid agents were introduced into the cell. The LDH activity was increased in the ASO (control)-treated group, indicating cell toxicity. In the administration groups of ASO (5'-CP gap1) to ASO (5'-CP gap12), the LDH activity was low, particularly in the group treated with ASO in which 5'-CP modifications were introduced into gaps 1 to 3 and gaps 7 to 9, indicating the effect of reducing cell toxicity.

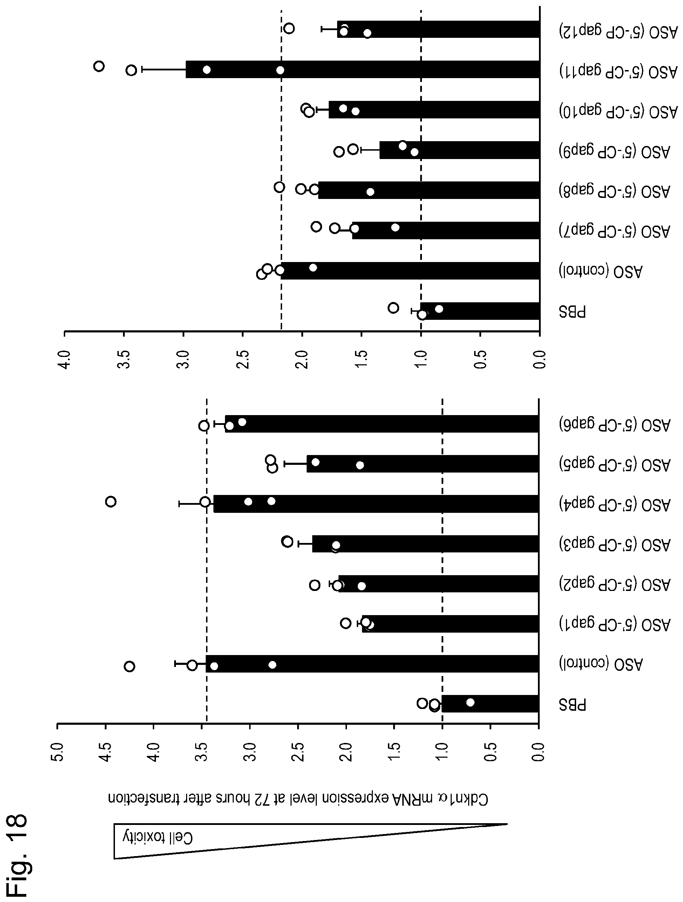

Figure 18 shows the results of evaluation of the Cdkn1a mRNA expression level as the cell toxicity when various nucleic acid agents were introduced into the cell. The expression amount of Cdkn1a mRNA was increased in the ASO (control)-treated group, indicating cell toxicity. On the other hand, in the administration groups of ASO (5'-CP gap1) to ASO(5'-CP gap12, the expression level of Cdkn1a mRNA was decreased in ASO in which 5'-CP modifications were introduced into gaps 1 to 3 and gaps 7 to 9, indicating the effect of reducing cell toxicity.

### <Example 5: Evaluation of toxicity of Snca targeted ASO with 5'-CP modification introduced into gap region>

### (Purpose)

A 5'-CP modification is introduced into a gap region of a gapmer ASO targeting the Snca gene, and the toxicity-reducing effect thereof is investigated through an in vitro experiment (mouse cells).

### (Method)

### (1) Preparation of nucleic acids

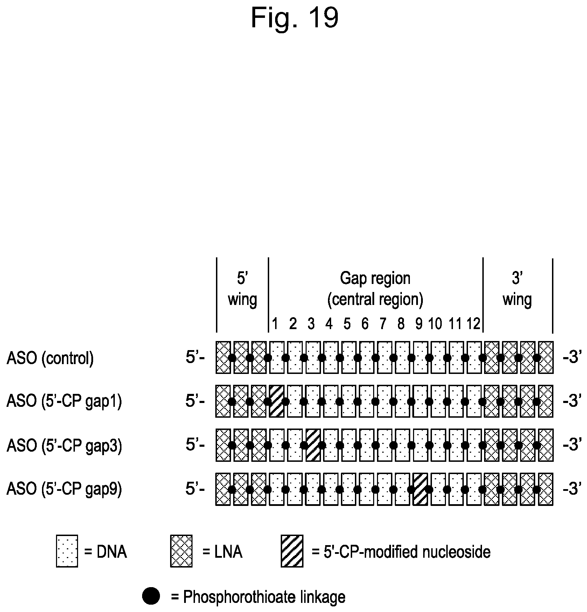

The base sequences and chemical modifications of ASOs used in this Example are shown in Table 5 and Figure 19.

**[Table 5]**

| **Table 5: ASO with 5'-CP modification introduced into the gap region** | | |
|---|---|---|
| | Sequence (5'-3') | SEQ ID NO |
| ASO(control) | c(5)^c(5)^a^t^t^t^a^t^a^t^a^c^a^a^ a^c(5)^a^c(5)^a | 17 |
| ASO(5'-CP gap1) | c(5)^c(5)^a^tCP^t^t^a^t^a^t^a^c^a^a ^a^c(5)^a^c(5)^a | 18 |
| ASO(5'-CP gap3) | c(5) ^c(5) ^a^t^t^tCP^a^t^a^t^a^c^a^a ^a^c(5) ^a^c(5) ^a | 20 |
| ASO(5'-CP gap9) | c(5)^c(5)^a^t^t^t^a^t^a^t^a^c(5)CP^ a^a^a^c(5)^a^c(5)^a | 26 |

| | | |
|---|---|---|
| Lower case letter: DNA; Underlined lower case letter: LNA; Superscript "CP": 5'-CP modification; ^: PS linkage; c(5) represents that the base moiety is 5-methylcytosine. | | |

ASO (control), ASO (5'-CP gap1), ASO(5'-CP gap3), and ASO (5'-CP gap9) used in this Example were the same as the ASOs used in Example 4.

### (2) Evaluation of cell toxicity

The ASO prepared in (1) was introduced into a mouse neuroblastoma-derived cells (Neuro 2a cell line) at 50 nM using a lipofection method (lipofectamine 2000). To evaluate the neurotoxicity at 72 hours after the introduction, the number of viable cells, the lactate dehydrogenase (LDH) activity in the cell supernatant, and the expression amount of Cdkn1a mRNA were measured by the same methods as in Example 1.

### (Results)

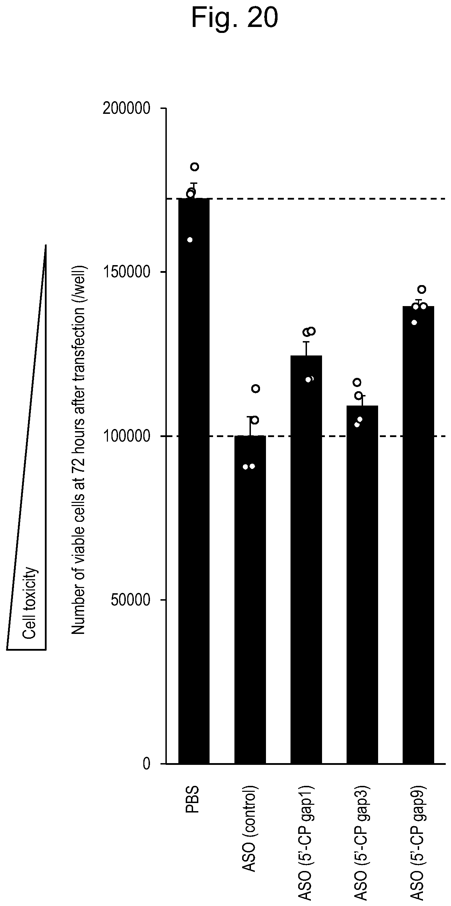

Figure 20 shows the results of measurement of the number of viable cells after transfection with PBS or various nucleic acid agents as the cell toxicity to the Neuro-2a cell line. The number of viable cells decreased in the ASO (control)-treated group, compared with PBS-treated group, indicating cell toxicity. The number of viable cells was high in the administration groups of ASO (5'-CP gap1), ASO (5'-CP gap3), and ASO (5'-CP gap9), indicating a toxicity-reducing effect.

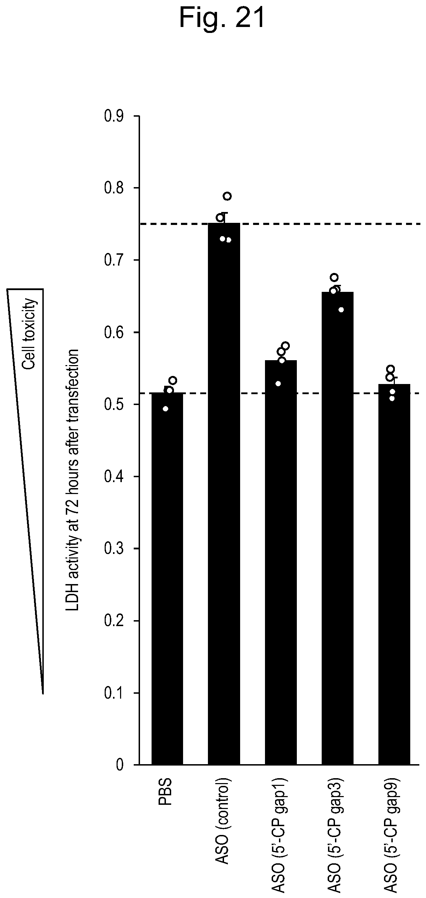

Figure 21 shows the results of measurement of the LDH activity in the supernatant as an indicator of cell toxicity. The LDH activity was increased in the ASO (control)-treated group, indicating cell toxicity. The LDH activity was low in the administration groups of ASO (5'-CP gap1), ASO (5'-CP gap3), and ASO (5'-CP gap9), indicating a toxicity-reducing effect.

The above-described results have revealed that introducing a 5'-CP modification at the 1st, 3rd, 9th base positions (gap 1, gap 3, and gap 9) from the 5' side of the gap region can reduce cell toxicity.

### <Example 6: Evaluation of toxicity of Hdac2 targeted ASO with 5'-CP modification and 2'-O-methyl modification introduced into gap region>

### (Purpose)

A 5'-CP modification and a 2'-O-methyl modification are introduced into a gap region of a gapmer ASO targeting the Hdac2 gene, and the toxicity-reducing effect is investigated through an in vitro experiment.

### (Method)

### (1) Preparation of nucleic acids

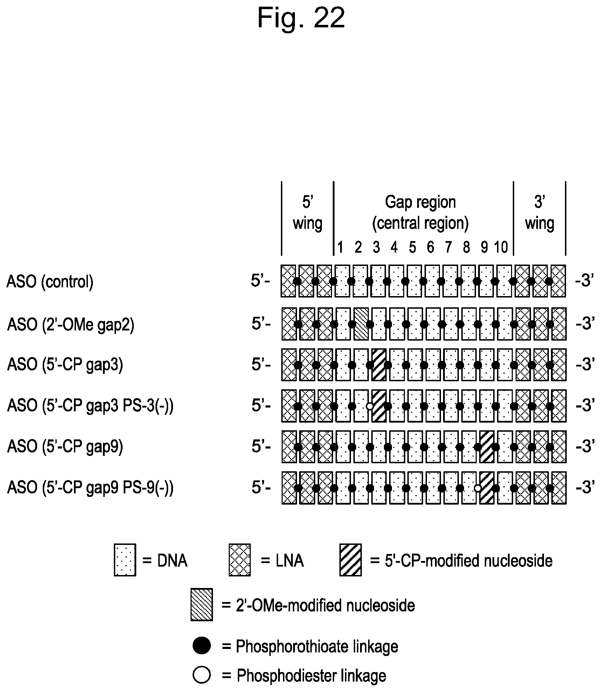

The base sequences and chemical modifications of ASOs used in this Example are shown in Table 6 and Figure 22.

**[Table 6]**

| **Table 6: ASO with 5'-CP modification or 2'-OMe modification introduced into the gap region** | | |
|---|---|---|
| | Sequence (5'-3') | SEQ ID NO |
| ASO(control) | t^a^g^t^c^t^c^t^g^t^c^a^g^t ^t^a | 30 |
| ASO(2'-OMe gap2) | t^a^g^t^C(M)^t^c^t^g^t^c^a^ g^t^t^a | 31 |
| ASO(5'-CP gap3) | t^a^g^t^c^tCP^c^t^g^t^c^a^g ^t^t^a | 32 |
| ASO(5'-CP gap3 PS-3(-)) | t^a^g^t^c*tCP^c^t^g^t^c^a^g ^t^t^a | 33 |
| ASO(5'-CP gap9) | t^a^g^t^c^t^c^t^g^t^c^a^CP^g ^t^t^a | 34 |
| ASO(5'-CP gap9 PS-9(-)) | t^a^g^t^c^t^c^t^g^t^c*a^CP^g ^t^t^a | 35 |

| | | |
|---|---|---|
| Lower case letter: DNA; Underlined lower case letter: LNA; Superscript "CP": 5'-CP modification; Upper case letter (M): 2'-O-Me-RNA; ^: PS linkage; *: PO linkage; c(5) represents that the base moiety is 5-methylcytosine. | | |

The ASO (control) used in this Example is an LNA/DNA gapmer ASO targeting a histone deacetylase 2 (Hdac2) mRNA of a mouse, has a base sequence complementary to a part of the Hdac2 mRNA, and has a structure wherein three LNA nucleosides at the 5' end, three LNA nucleosides at the 3' end, and ten DNA nucleosides therebetween are linked via a phosphorothioate linkage (PS linkage).

ASO(2'-OMe gap2) used in this Example comprises a 2'-OMe-modified nucleoside at the 2nd base position (gap 2) from the 5' side of the gap region of ASO (control). The 2'-OMe modification is a modification wherein the hydroxyl group at the 2' position of ribose is substituted with a methoxy group. ASO (5'-CP gap3) comprises a 5'-CP-modified nucleoside at the 3rd base position (gap 3) from the 5' side of the gap region of ASO (control). ASO (5'-CP gap3 PS-3(-)) comprises a 5'-CP-modified nucleoside at the 3rd base position (gap 3) from the 5' side of the gap region of ASO (control), and an internucleoside linkage between gap 2 and gap 3 is a phosphodiester linkage (PO linkage). ASO (5'-CP gap9) comprises a 5'-CP-modified nucleoside at the 9th base position (gap 9) from the 5' side of the gap region of ASO (control). ASO (5'-CP gap9 PS-9(-)) comprises a 5'-CP-modified nucleoside at the 9th base position (gap 9) from the 5' side of the gap region of ASO (control), and an internucleoside linkage between gap 8 and gap 9 is a phosphodiester linkage (PO linkage).

### (2) Evaluation of cell toxicity

The ASO prepared in (1) was introduced into a human neuroblastoma-derived cells (BE(2)-M17 cell line) at 20 nM using a lipofection method (lipofectamine 2000). To evaluate the neurotoxicity at 72 hours after the introduction, the number of cells and the lactate dehydrogenase (LDH) activity in the cell supernatant were measured in accordance with the method described in Example 1.

### (Results)

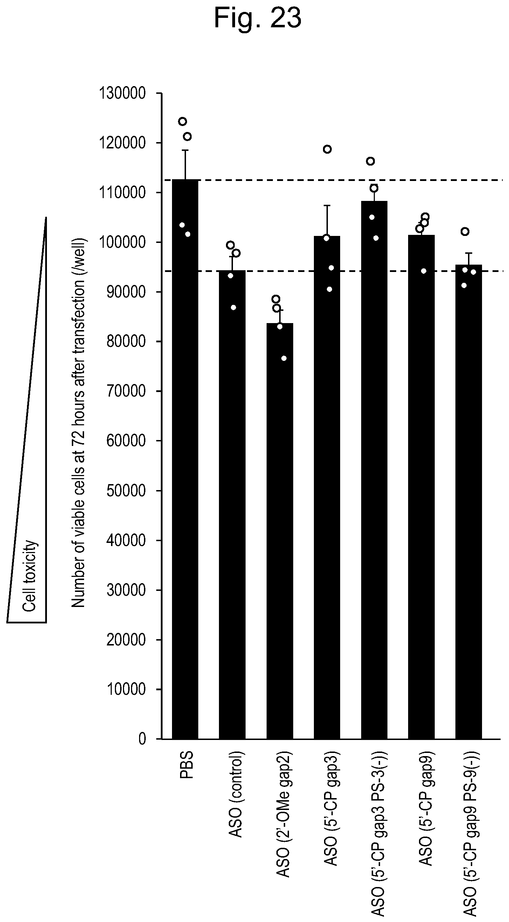

Figure 23 shows the results of measurement of the number of viable cells after transfection with PBS or various nucleic acid agents as the cell toxicity to the BE(2)-M17 cell line. The number of viable cells decreased in the ASO (control)-treated group, compared with PBS-treated group, indicating cell toxicity. The number of viable cells was lower in the ASO (2'-OMe gap2)-treated group with 2'-OMe modification introduced into gap2 than in the ASO (control)-treated group, resulting in enhanced cell toxicity. Compared with this, the number of viable cells was higher in the ASO with a 5'-CP modification introduced into gap 3 or gap 9 than in the ASO (control)-treated group, regardless of whether the internucleoside linkage bound to the 5'-side of the 5'-CP-modified nucleoside is a PS linkage or a PO linkage, indicating an effect of reducing cell toxicity.

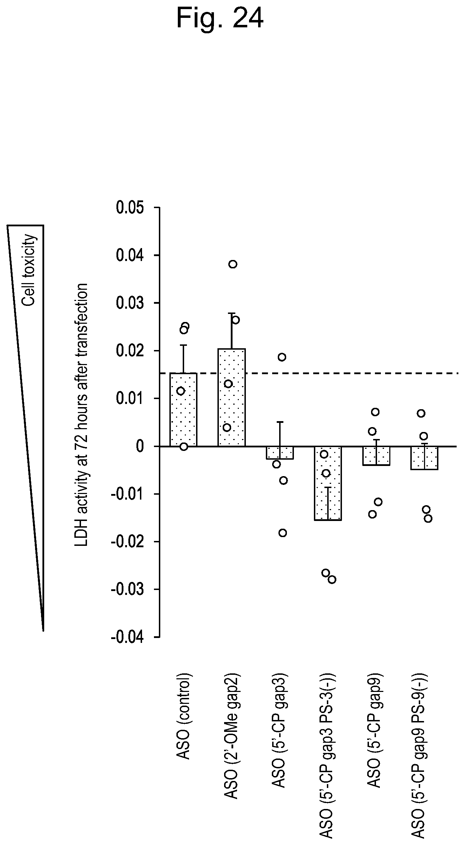

Figure 24 shows the results of measurement of the LDH activity in the supernatant as the cell toxicity when various nucleic acid agents were introduced into the cell. The LDH activity was increased in the ASO (control)-treated group, indicating cell toxicity. Compared with this, the LDH activity was lower in the ASO with a 5'-CP modification introduced into gap 3 or gap 9 than in the ASO (control)-treated group, regardless of whether the internucleoside linkage bound to the 5'-side of the 5'-CP-modified nucleoside is a PS linkage or a PO linkage, indicating an effect of reducing cell toxicity.

The above-described results have revealed that introducing a 5'-CP modification at the 3rd base position (gap 3) and 9th base position (gap 9) from the 5' side of the gap region can reduce cell toxicity.

Although 5'-CP modification is a nucleic acid modification that can strengthen nuclease resistance when a more biocompatible phosphodiester linkage is used for the purpose of avoiding a phosphorothioate linkage, which is concerned about its accumulation in a particular organ or the like, the effect of the 5'-CP modification itself in reducing toxicity was unexpected. In particular, it is surprising the modification produces the toxicity-reducing effect regardless of whether the internucleoside linkage bound to the 5'-side of the 5'-CP-modified nucleoside is a PS linkage or a PO linkage.

### <Example 7: Evaluation of toxicity of Hdac2 targeted ASO with 5'-CP modification introduced into gap region>

### (Purpose)

A 5'-CP modification is introduced into a gap region of a gapmer ASO targeting the Hdac2 gene, and the influence on the gene suppression effect is investigated through an in vitro experiment.

### (Method)

### (1) Preparation of nucleic acids

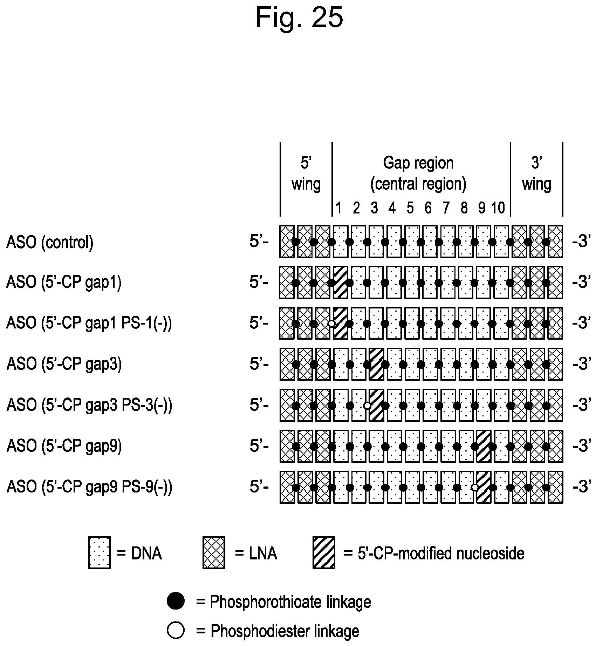

The base sequences and chemical modifications of ASOs used in this Example are shown in Table 7 and Figure 25.

**[Table 7]**

| **Table 7: ASO with 5'-CP modification introduced into the gap region** | | |
|---|---|---|
| | Sequence (5'-3') | SEQ ID NO |
| ASO(control) | t^a^g^t^c^t^c^t^g^t^c^a^g^t^t^a | 30 |
| ASO(5'-CP gap1) | t^a^g^t^{CP}^c^t^c^t^g^t^c^a^g^t^t^a | 36 |
| ASO(5'-CP gap1 PS-1(-)) | t^a^g*t^{CP}^c^t^c^t^g^t^c^a^g^t^t^a | 37 |
| ASO(5'-CP gap3) | t^a^g^t^c^t^{CP}^c^t^g^t^c^a^g^t^t^a | 32 |
| ASO(5'-CP gap3 PS-3(-)) | t^a^g^t^c*t^{CP}^c^t^g^t^c^a^g^t^t^a | 33 |
| ASO(5'-CP gap9) | t^a^g^t^c^t^c^t^g^t^c^a^{CP}^g^t^t^a | 34 |
| ASO(5'-CP gap9 PS-9(-)) | t^a^g^t^c^t^c^t^g^t^c*a^{CP}^g^t^t^a | 35 |

| | | |
|---|---|---|
| Lower case letter: DNA; Underlined lower case letter: LNA; Superscript "CP": 5'-CP modification; Upper case letter (M): 2'-O-Me-RNA; ^: PS linkage; *: PO linkage; c(5) represents that the base moiety is 5-methylcytosine. | | |

The ASO (control) used in this Example is an LNA/DNA gapmer ASO targeting a histone deacetylase 2 (Hdac2) mRNA of a mouse, has a base sequence complementary to a part of the Hdac2 mRNA, and has a structure wherein three LNA nucleosides at the 5' end, three LNA nucleosides at the 3' end, and ten DNA nucleosides therebetween are linked via a phosphorothioate linkage (PS linkage).

ASO (5'-CP gap1) used in this Example comprises a 5'-CP-modified nucleoside at the 1st base position (gap 1) from the 5' side of the gap region of ASO (control). ASO (5'-CP gap 1 PS-3(-)) comprises a 5'-CP-modified nucleoside at the 1st base position (gap 1) from the 5' side of the gap region of ASO (control), and an internucleoside linkage on the 5' side of gap 1 is a phosphodiester linkage (PO linkage). ASO (5'-CP gap3) comprises a 5'-CP-modified nucleoside at the 3rd base position (gap 3) from the 5' side of the gap region of ASO (control). ASO (5'-CP gap3 PS-3(-)) comprises a 5'-CP-modified nucleoside at the 3rd base position (gap 3) from the 5' side of the gap region of ASO (control), and an internucleoside linkage between gap 2 and gap 3 is a phosphodiester linkage (PO linkage). ASO (5'-CP gap9) comprises a 5'-CP-modified nucleoside at the 9th base position (gap 9) from the 5' side of the gap region of ASO (control). ASO (5'-CP gap9 PS-9(-)) comprises a 5'-CP-modified nucleoside at the 9th base position (gap 9) from the 5' side of the gap region of ASO (control), and an internucleoside linkage between gap 8 and gap 9 is a phosphodiester linkage (PO linkage).

### (2) Evaluation of gene suppression effect

The ASO prepared in (1) was introduced into a mouse neuroblastoma-derived cells (Neuro 2a cell line) using a lipofection method (lipofectamine 2000). Using an IsogenI kit (GeneDesign Inc.), RNA was extracted from the cell at 72 hours after the introduction of the ASO, and cDNA synthesis and quantitative RT-PCR were performed according to the method described in Example 1. The ratio of the expression amount of Hdac2 mRNA to the expression amount of Actb mRNA (internal standard gene) was calculated, and a value standardized with respect to the value of the PBS-treated group was determined as a relative Hdac2 mRNA level.

### (Results)

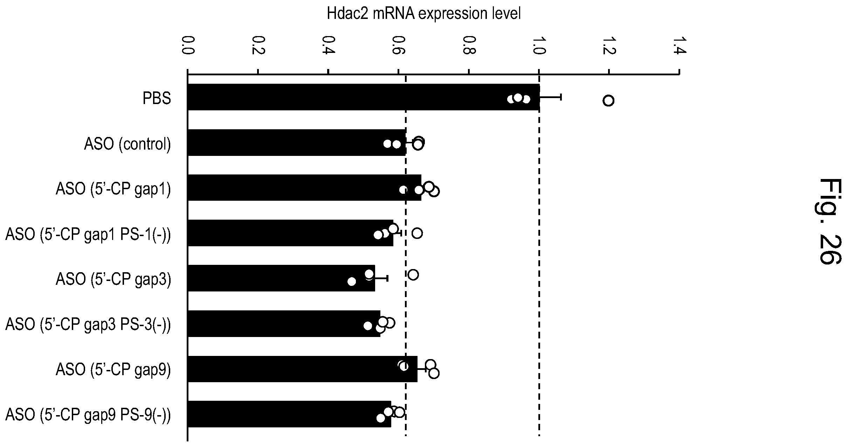

Figure 26 shows the results of measurement of the Hdac2 mRNA expression level in the Neuro 2a cell line into which various nucleic acid agents were introduced. The group treated with ASO in which 5'-CP modifications were introduced into gap 1, gap 3, and gap 9 of the gap region, achieved a gene suppression effect equivalent to that of ASO (control), regardless of whether the internucleoside linkage bound to the 5'-side of the 5'-CP-modified nucleoside is a PS linkage or a PO linkage.

The effect of the 5'-CP modification itself in reducing toxicity is unexpected, and in particular, it is surprising the modification produces the toxicity-reducing effect regardless of the kind of the internucleoside linkage bound to the 5' side of the 5'-CP-modified nucleoside.

### <Example 8: Evaluation of toxicity of Hdac2 targeted ASO with 2'-O-methyl modification introduced into gap region>

### (Purpose)

A 2'-OMe modification is introduced into a gap region of a gapmer ASO targeting the Hdac2 gene and intraventricularly administered. Through an in vivo experiment, the central nervous system toxicity is compared.

### (Method)

### (1) Preparation of nucleic acids

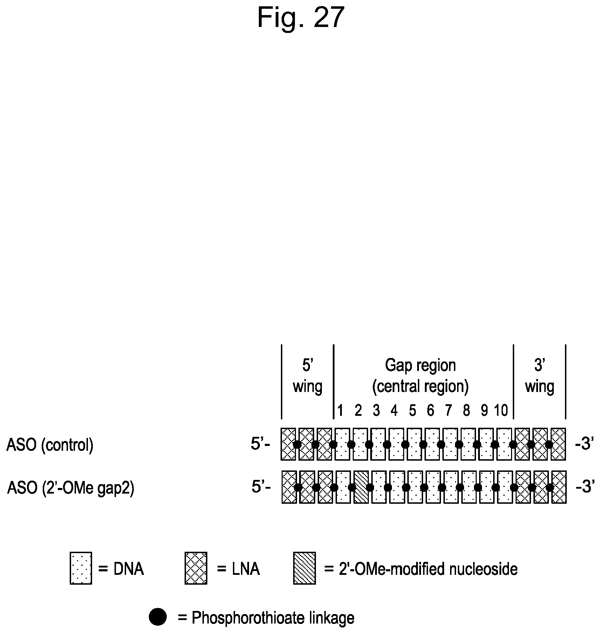

The base sequences and chemical modifications of the ASOs used in this Example are shown in Table 8 and Figure 27.

**[Table 8]**

| **Table 8: ASO with 2'-OMe modification introduced into the gap region** | | |
|---|---|---|
| | Sequence (5'-3') | SEQ ID NO |
| ASO(control) | t^a^g^t^c^t^c^t^g^t^c^a^g^t^t^a | 30 |
| ASO(2'-OMe gap2) | t^a^g^t^C(M)^t^c^t^g^t^c^a^g^t^t^a | 31 |

| | | |
|---|---|---|
| Lower case letter: DNA; Underlined lower case letter: LNA; Upper case letter (M): 2'-O-Me-RNA; ^: PS linkage; c(5) represents that the base moiety is 5-methylcytosine. | | |

ASO (control) and ASO (2'-OMe gap2) described in Example 6 were used as ASO (control) and ASO (2'-OMe gap2) used in this Example.

### (2) In vivo experiment

The nuclear acid agent was intraventricularly administered by the same method as in Example 3. However, the nucleic acid agent at a dose of 18.96 nmol per mouse was administered, and a mouse to which only nuclease free water (NFW) was administered was provided as a negative control group.

### (3) Evaluation of delayed central nervous system toxicity after administration of nucleic acid agents

To evaluate delayed central nervous system toxicity, measurement of the body weight/food intake, evaluation of the motor function through an open-field test, and behavior evaluation on the basis of a scoring system were performed on mice to which various nucleic acid agents were administered.

The body weight and food intake were measured at 3 and 6 days after the administration of the nucleic acid agents.

Evaluation of the motor function was performed through the open-field test by the same methods as in Example 3 at 6 days after the administration of the nucleic acid agent.

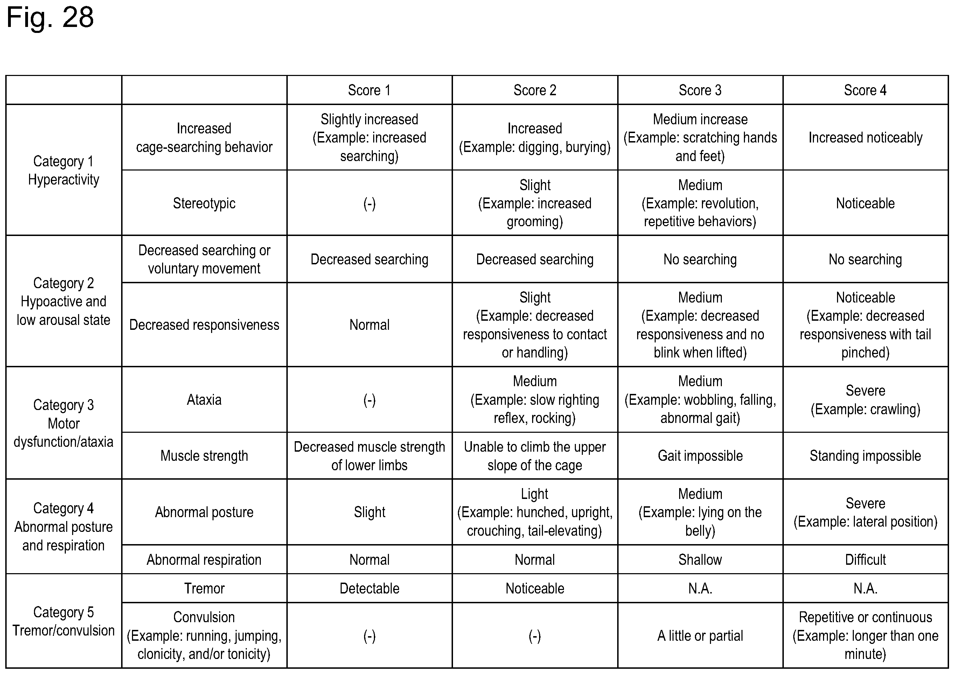

The behavior was evaluated, using the scoring system shown in Figure 28, at 6 days after the administration of the nucleic acid agent. According to the scoring system shown in Figure 28, the behaviors classified into five categories were evaluated (Categories 1 to 5 in Figure 28). Each category comprises two behavior evaluation items. Each behavior evaluation item is rated in five stages from 0 to 4 points (Scores 0 to 4 in Figure 28). A normal behavior is given 0 points, and higher toxicity is given a higher score. In each category, the higher score in the two behavior evaluation items is adopted as the score for the category. The total value of the scores for the five categories is defined as an acute tolerability score (0 to 20 points).

### (Results)

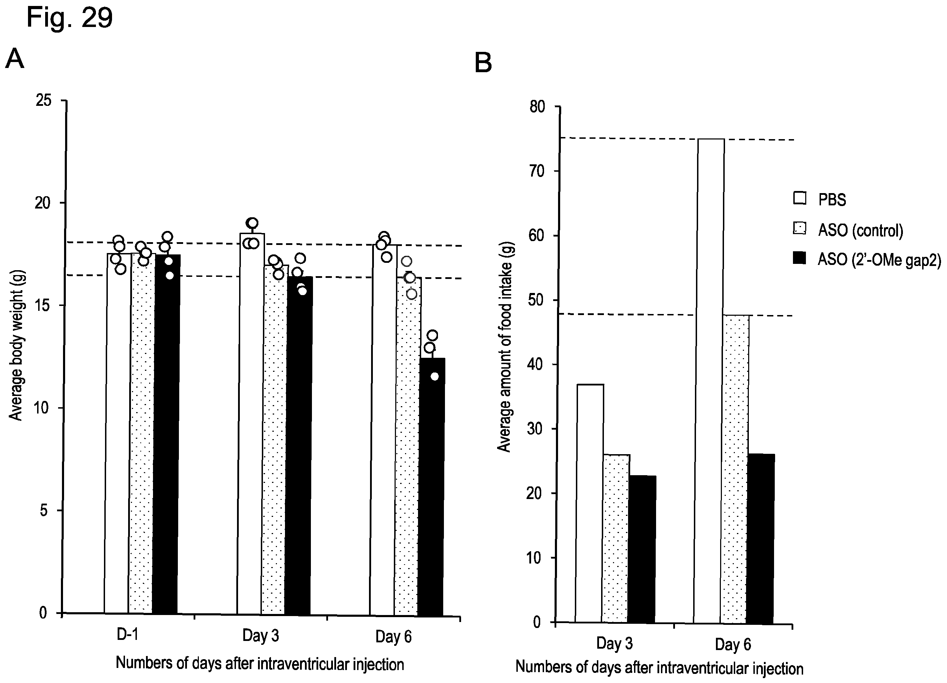

Figure 29 shows the results of measurement of the body weight of mice/their food intake at 3 and 6 days after various nucleic acid agents were intraventricularly administered to the mice. The body weight and food intake were lower in the ASO (control)-treated group than in the PBS-treated group, indicating central nervous system toxicity. The body weight and food intake were even lower in the ASO (2'-OMe gap2)-treated group than in the ASO (control)-treated group, indicating enhanced delayed central nervous system toxicity.

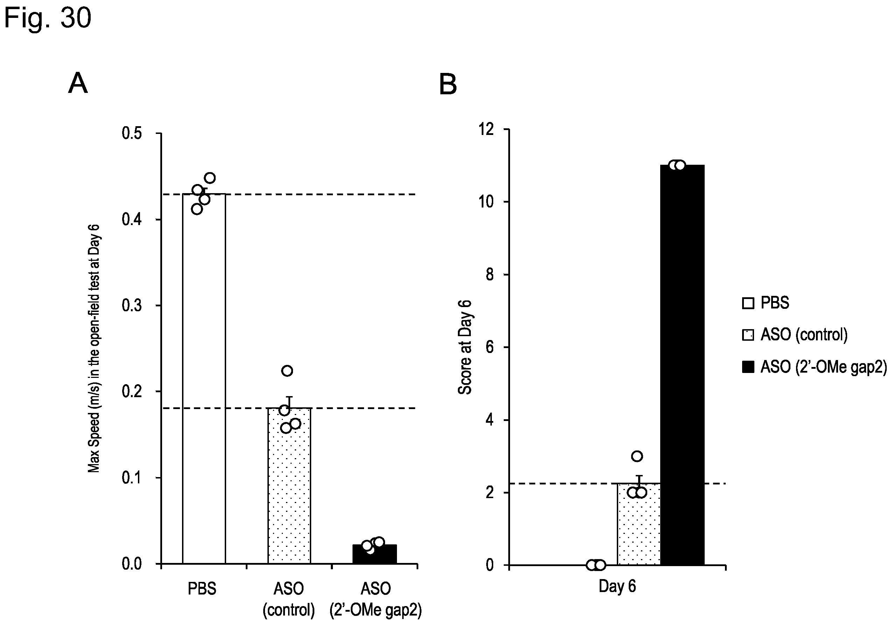

Figure 30A shows the results of evaluation of the motor function of mice at 6 days after various nucleic acid agents were intraventricularly administered to the mice. The maximum moving speed was lower in the ASO (2'-OMe gap2)-treated group than in the ASO (control)-treated group, indicating enhanced delayed central nervous system toxicity.

Figure 30B shows the results of behavior evaluation of mice at 6 days after various nucleic acid agents were intraventricularly administered to the mice. The score was increased in the ASO (2'-OMegap2)-treated group, compared with the ASO (control)-treated group, indicating enhanced delayed central nervous system toxicity.

The above-described results have revealed that introducing a 2'-OMe modification into gap 2 in the gap region enhances delayed neurotoxicity.

### <Example 9: Evaluation of toxicity of Hdac2 targeted ASO with 5'-CP modification introduced into gap region>

### (Purpose)

A 5'-CP modification is introduced into a gap region of a gapmer ASO targeting the Hdac2 gene to evaluate central nervous system toxicity exhibited when intraventricularly administered. Through an in vivo experiment, the toxicity-reducing effect based on 5'-CP modifications is investigated.

### (Method)

### (1) Preparation of nucleic acids

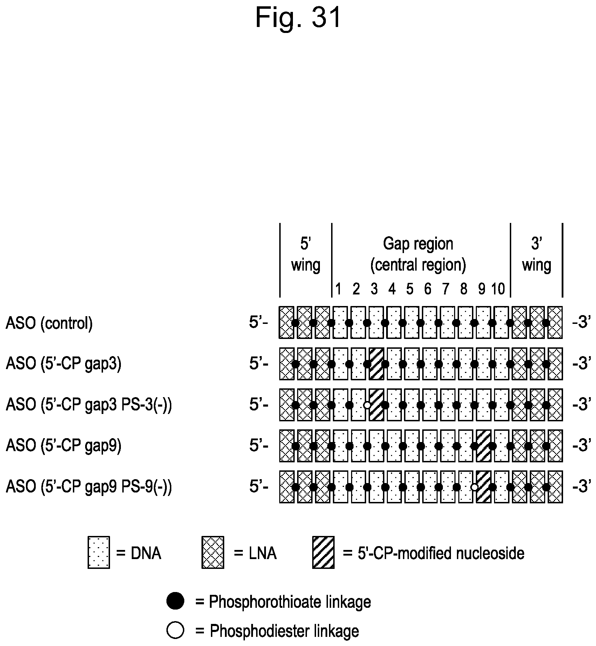

The base sequences and chemical modifications of the ASOs used in this Example are shown in Table 9 and Figure 31.

**[Table 9]**

| **Table 9: ASO with 5'-CP modification introduced into the gap region** | | |
|---|---|---|
| | Sequence (5'-3') | SEQ ID NO |
| ASO(control) | t^a^g^t^c^t^c^t^g^t^c^a^g^t^t^a | 30 |
| ASO(5'-CP gap3) | t^a^g^t^c^t^{CP}c^t^g^t^c^a^g^t^t^a | 32 |
| ASO(5'-CP gap3 PS-3(-)) | t^a^g^t^c*t^{CP}^c^t^g^t^c^a^g^t^t^a | 33 |
| ASO(5'-CP gap9) | t^a^g^t^c^t^c^t^g^t^c^a^{CP}^g^t^t^a | 34 |
| ASO(5'-CP gap9 PS-9(-)) | t^a^g^t^c^t^c^t^g^t^c*a^{CP}^g^t^t^a | 35 |

| | | |
|---|---|---|
| Lower case letter: DNA; Underlined lower case letter: LNA; Superscript "CP": 5'-CP modification; Upper case letter (M): 2'-O-Me-RNA; ^: PS linkage; *: PO linkage; c(5) represents that the base moiety is 5-methylcytosine. | | |

ASOs described in Table 9 above used in this Example are the same as ASO (control), ASO(5'-CP gap3), ASO (5'-CP gap3 PS-3(-)), ASO (5'-CP gap9), and ASO (5'-CP gap9 PS-9(-)) used in Example 7.

### (2) In vivo experiment

Various nucleic acid agents were administered into the left lateral ventricle of seven-week-old female C57BL/6 mice in the same manner as in (2) in vivo experiment of Example 8.

### (3) Evaluation of delayed central nervous system toxicity after administration of nucleic acid agents

To evaluate delayed central nervous system toxicity, measurement of the body weight/food intake, evaluation of the motor function through an open-field test, behavior evaluation on the basis of a scoring system, and measurement of the expression amounts of Tnf-α mRNA and Gfap mRNA were performed on mice to which various nucleic acid agents were administered.

The body weight/food intake was measured at 3, 6, 10, and 13 days after the administration of the nucleic acid agents.

Evaluation of the motor function was performed through the open-field test by the same methods as in Example 8 at 3, 6, 10, and 13 days after the administration of the nucleic acid agents. Behavior evaluation was performed by the same methods as in Example 8 at 6 days after the administration of the nucleic acid agent.

The expression amounts of Tnf-α mRNA and Gfap mRNA were measured according to the following procedure. A left hippocampus was extirpated from the mice at 14 days after the administration of various nucleic acid agents. Using an IsogenI kit (GeneDesign Inc.), RNA was extracted from the left hippocampus extirpated. A cDNA was synthesized using Transcriptor Universal cDNA Master, DNase (Roche Diagnostics) in accordance with the protocol. Next, the expression amounts of Tnf-α mRNA, Gfap mRNA, and Actb mRNA (the internal standard gene) were measured by performing the same quantitative RT-PCR as described above using the resulting cDNA template.

The ratio of the expression amounts of Tnf-α mRNA and Gfap mRNA to the expression amount of Actb mRNA (internal standard gene) was calculated, and values standardized with respect to the value of the NFW-treated group were each determined as a Tnf-α mRNA expression level and a Gfap mRNA expression level.

### (Results)

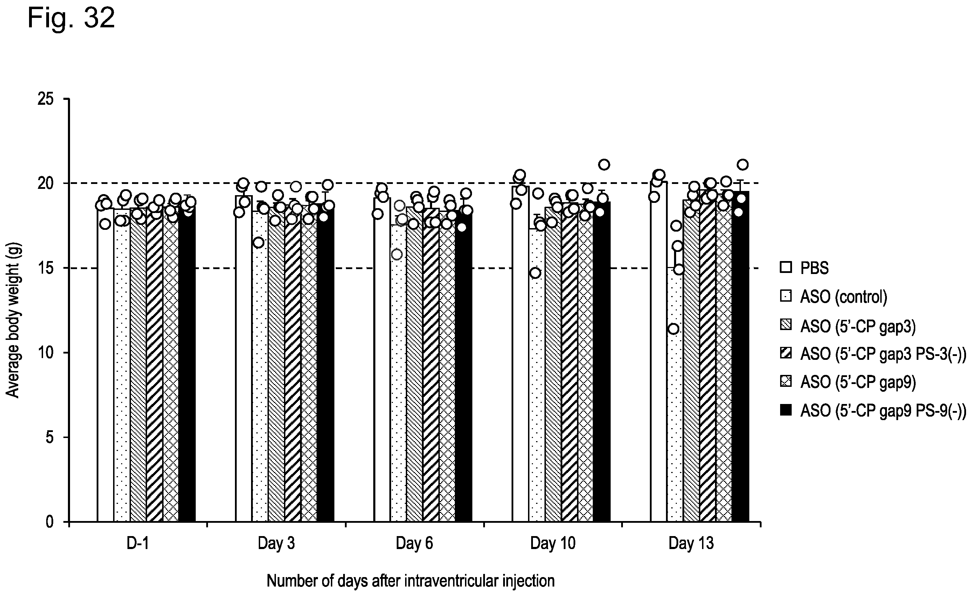

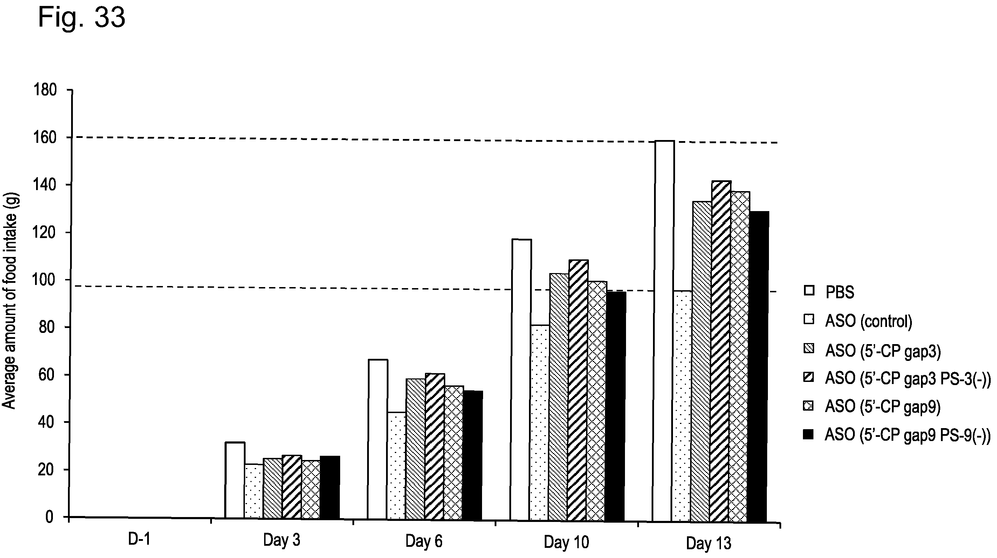

Figures 32 and 33 show the results of measurement of the body weight/food intake. The body weight/food intake was lower in the ASO (control)-treated group than in the PBS-treated group, indicating central nervous system toxicity. Compared with this, the body weight/food intake was improved in the administration groups of ASO (5'-CP gap3), ASO (5'-CP gap3 PS-3(-)), ASO (5'-CP gap9), and ASO (5'-CP gap9 PS-9(-)), compared with the ASO (control)-treated group, and was the same as that in PBS-treated group, indicating an effect of reducing delayed central nervous system toxicity.

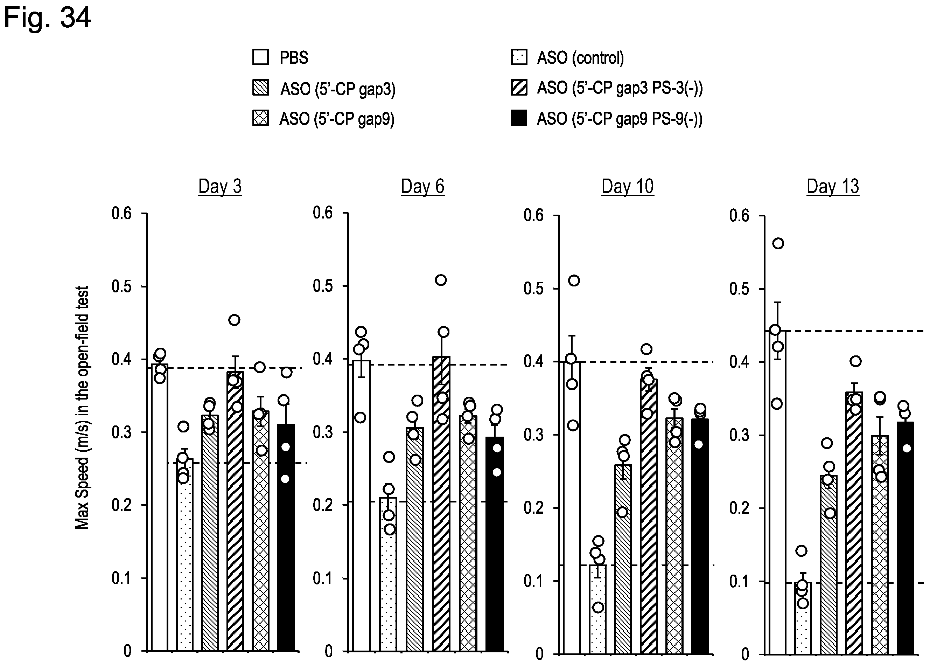

Figure 34 shows the results of evaluation of the motor function through an open-field test. The maximum moving speed was increased in the administration groups of ASO (5'-CP gap3), ASO (5'-CP gap3 PS-3(-)), ASO (5'-CP gap9), ASO (5'-CP gap9 PS-9(-)) compared with the administration group of ASO (control) at any point of time, indicating a reduction in delayed central nervous system toxicity.

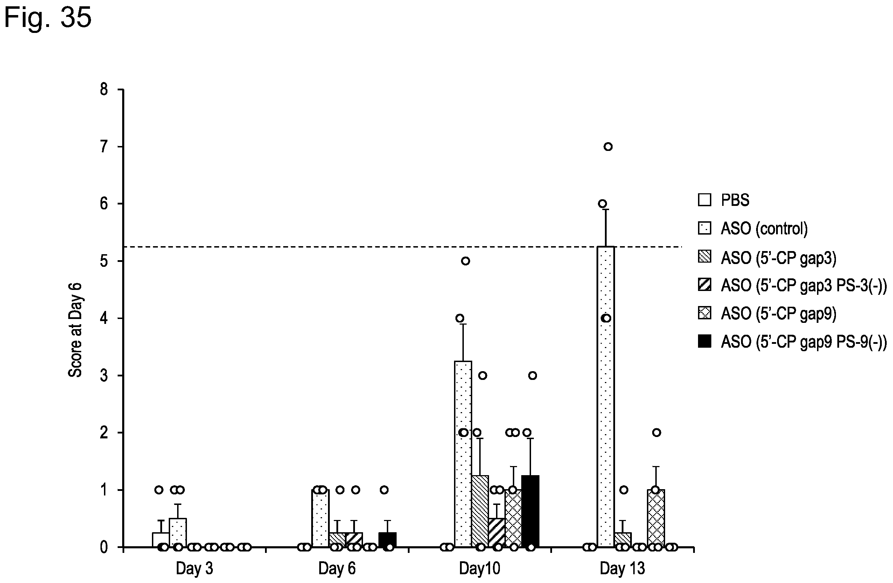

Figure 35 shows results of evaluation of behavior on the basis of a scoring system. The scores were lower in the administration groups of ASO (5'-CP gap3), ASO (5'-CP gap3 PS-3(-)), ASO (5'-CP gap9), and ASO (5'-CP gap9 PS-9(-)) than in the administration group of ASO (control) at any point of time, indicating a reduction in delayed central nervous system toxicity.

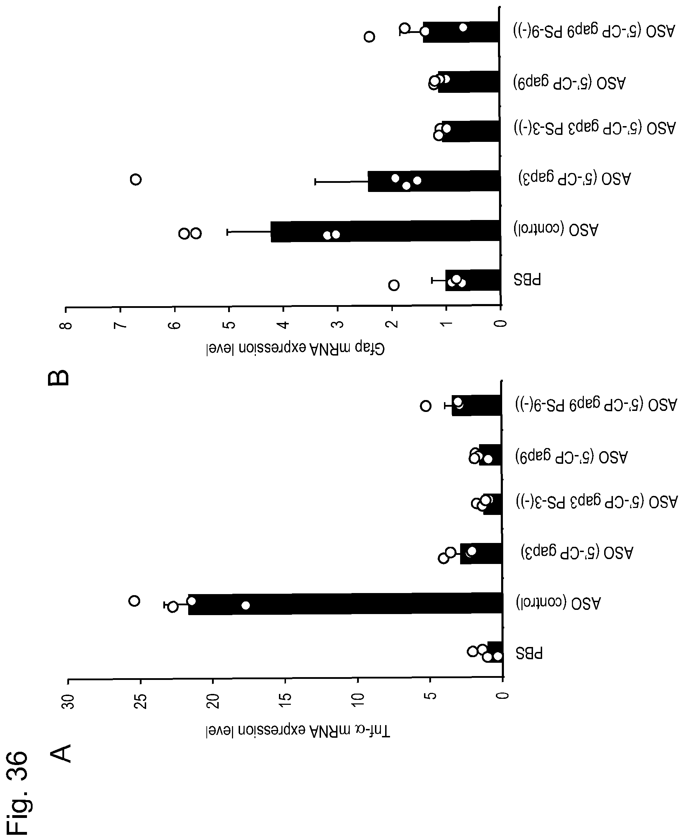

Figure 36 shows the results of measurement of the expression amounts of Tnf-α mRNA and Gfap mRNA in the left hippocampus as an indicator of delayed neurotoxicity. The ASO (control)-treated group showed increased expression of both, exhibiting cell toxicity. Compared with this, the administration groups of ASO (5'-CP gap3), ASO (5'-CP gap3 PS-3(-)), ASO (5'-CP gap9), and ASO (5'-CP gap9 PS-9(-)) showed reduced expression of both, exhibiting an effect of reducing delayed central nervous system toxicity.

The above-described results have demonstrated that the introduction of 5'-CP modifications at 3rd position (gap 3) and 9th position (gap 9) from the 5' side of the gap region can reduce delayed neurotoxicity, regardless of whether the internucleoside linkage bound to the 5'-side of the 5'-CP-modified nucleoside is a PS linkage or a PO linkage.

### <Example 10: Evaluation of in vivo toxicity of Mapt targeted ASO with 5'-CP modification introduced into gap region>

### (Purpose)

In terms of central nervous system toxicity exhibited when a gapmer ASO targeting the Mapt gene is intraventricularly administered, the toxicity-reducing effect of introducing a 5'-CP modification into the gap region is investigated through an in vitro experiment.

### (Method)

### (1) Preparation of nucleic acids

The base sequences and chemical modifications of the ASOs used in this Example are shown in Table 10 below.

**[Table 10]**

| **Table 10: ASO with 5'-CP modification introduced into the gap region** | | |
|---|---|---|
| | Sequence (5'-3') | SEQ ID NO |
| ASO(control) | a^t^t^t^c^c^a^a^a^t^t^c^a^c(5)^t^t | 1 |
| ASO(5'-CP gap1) | a^t^t^t^{CP}^c^c^a^a^a^t^t^c^a^c(5)^t^t | 2 |
| ASO(5'-CP gap3) | a^t^t^t^c^c(5)^{CP}^a^a^a^t^t^c^a^c(5)^t^t | 4 |

| | | |
|---|---|---|
| Lower case letter: DNA; Underlined lower case letter: LNA; Superscript "CP": 5'-CP modification; ^: PS linkage; c(5) represents that the base moiety is 5-methylcytosine. | | |

ASOs described in Table 10 used in this Example have the same structure as ASO (control), ASO (5'-CP gap1), and ASO (5'-CP gap3) used in Example 1.

### (2) In vivo experiment

Seven-week-old female ICR mice anesthetized with 2.5 to 4% isoflurane were fixed to a brain stereotaxic apparatus. Then, skin between the ears was cut open 2 to 3 cm anteroposteriorly, and perforated 1 mm leftward and 0.2 mm posteriorly from the bregma using a 1 mm diameter drill. A Hamilton syringe was filled with the nucleic acid agent. Through the perforated site, a needle was inserted 3 mm deep over 10 seconds using MDS-1 One-axis Motorized Stereotaxic Micromanipulator. Then, the nucleic acid agent at a dose of 38.4 nmol per mouse was administered into the left lateral ventricle at a rate of 5 µl/min (n=4). The skin was sutured with a nylon thread. In addition, a mouse to which only PBS was administered was provided as a negative control group.

### (3) Evaluation of delayed central nervous system toxicity after administration of nucleic acid agents

Evaluation of delayed central nervous system toxicity and evaluation of motor function were performed through a measurement of body weight/food intake and an open-field test, respectively, on mice to which various nucleic acid agents were administered. The body weight/food intake was measured at 21 days after the administration of the nucleic acid agents. Evaluation of motor function was performed at 3, 7, 10, 14, 17, and 21 days after the administration of the nucleic acid agents. Specifically, a mouse was placed in the center of a cage (50 cm in width × 50 cm in diameter × 40 cm in height), and the track of the mouse movement was recorded for 5 minutes. The maximum moving speed (m/s) based on the data recorded were measured using video tracking software (ANY-maze).

### (Results)

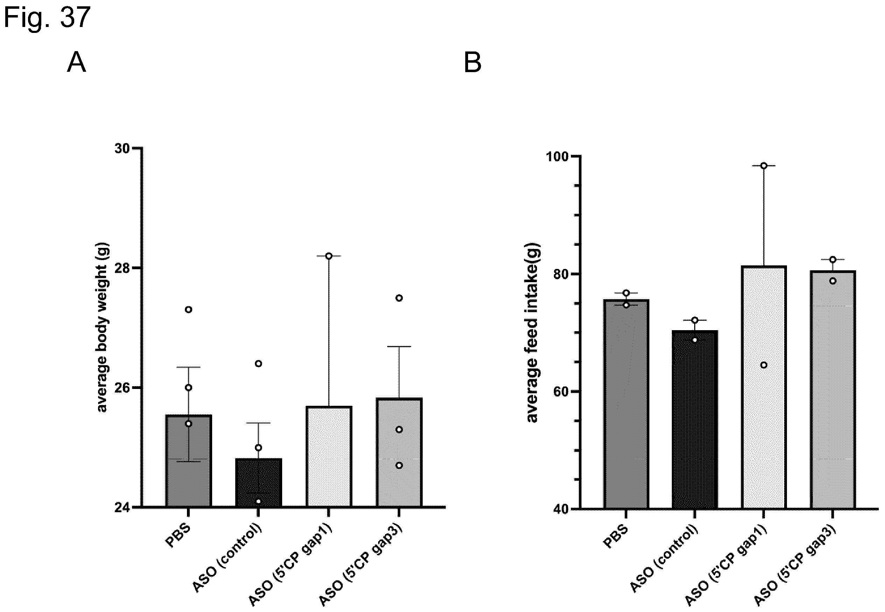

Figure 37 shows the results of measurement of the body weight of mice (Figure 37A) and their food intake (Figure 37B) at 21 days after various nucleic acid agents were intraventricularly administered to the mice. The body weight and food intake were lower in the ASO (control)-treated group than in the PBS-treated group, indicating central nervous system toxicity. On the other hand, both the body weight and the food intake were higher in the ASO (5'-CP gap1)-treated group and the ASO (5'-CP gap3)-treated group than in the ASO (control)-treated group, indicating the toxicity-reducing effect.

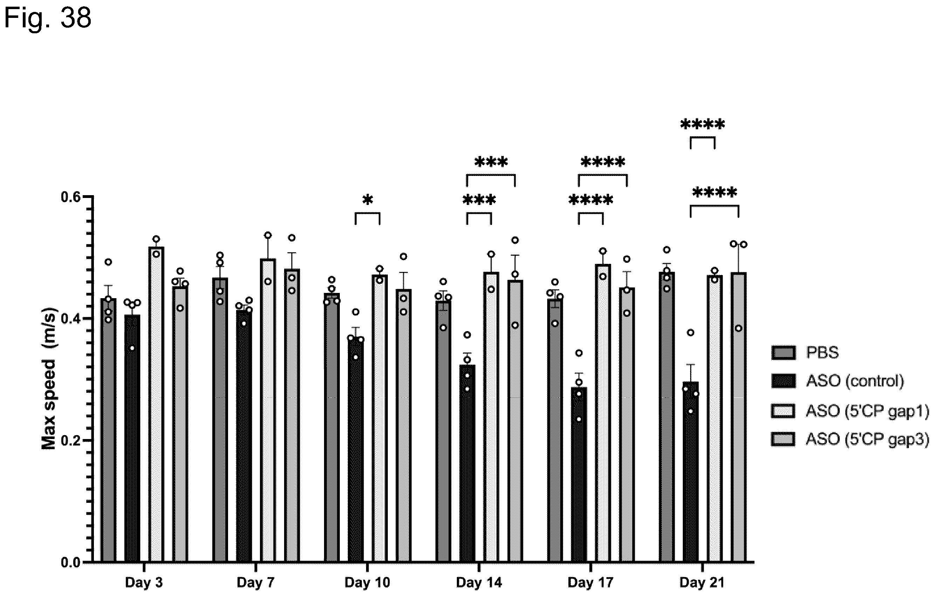

Figure 38 shows the results of evaluation of the motor function of mice at 3, 7, 10, 14, 17, and 21 days after various nucleic acid agents were intraventricularly administered to the mice. The maximum moving speed was higher in the administration groups of ASO (5'-CP gap1) and ASO (5'-CP gap3) than in the administration group of ASO (control).

The above-described results have revealed that the introduction of 5'-CP into ASO can reduce delayed neurotoxicity for all indicators of body weight, food intake, and maximum moving speed.

### <Example 11: Evaluation of toxicity of Hdac2 targeted ASO with one of PS linkages changed to PO linkage in gap region>

### (Purpose)

One of the phosphorothioate linkages (PS linkages) in the gap region of a gapmer ASO targeting the Hdac2 gene is changed to a phosphodiester linkage (PO linkage) to evaluate whether or not the delayed central nervous toxicity in the case of intraventricular administration can be reduced and whether or not the gene suppression effect is retained.

### (Method)

### (1) Preparation of nucleic acids

The base sequences and chemical modifications of the ASOs used in this Example are shown in Table 11 below.

**[Table 11]**

| **Table 11: ASO with one of the PS linkages changed to PO linkage in the gap region** | | |
|---|---|---|
| | Sequence (5'-3') | SEQ ID NO |
| ASO(control) | t^a^g^t^c^t^c^t^g^t^c^a^g^t ^t^a | 30 |
| ASO(PS-1(-)) | t^a^g*t^c^t^c^t^g^t^c^a^g^t ^t^a | 38 |
| ASO(PS-3(-)) | t^a^g^t^c*t^c^t^g^t^c^a^g^t ^t^a | 39 |
| ASO(PS-9(-)) | t^a^g^t^c^t^c^t^g^t^c*a^g^t ^t^a | 40 |

| | | |
|---|---|---|
| Lower case letter: DNA; Underlined lower case letter: LNA; ^: PS linkage; *: PO linkage | | |

ASO (control) used in this Example is an LNA/DNA gapmer ASO targeting mouse Hdac2 mRNA and has the same structure as the ASO (control) used in Example 6. In ASO (control), all internucleoside linkages are PS linkages.

In ASO (PS-1 (-)) used in this Example, the internucleoside linkage between the 5'-wing region and the gap region of ASO (control) (internucleoside linkage adjacent to the 5' side at the most 5'-side base position (gap 1) in the gap region) is changed from a PS linkage to a PO linkage. In ASO (PS-3(-)), and an internucleoside linkage between the 2nd base position (gap 2) from the 5' side of the gap region of ASO (control) and gap 3 is a phosphodiester linkage (PO linkage). In ASO (PS-9(-)), an internucleoside linkage between gap 8 and gap 9 of ASO (control) is a phosphodiester linkage (PO linkage).

### (2) In vivo experiment

Various nucleic acid agents were administered into the left lateral ventricle of seven-week-old female C57BL/6 mice in the same manner as in (2) in vivo experiment of Example 8.

### (3) Evaluation of delayed central nervous system toxicity after administration of nucleic acid agents

To evaluate delayed central nervous system toxicity, evaluation of the motor function was performed on mice to which various nucleic acid agents were administered through an open-field test at 3 days after the administration of the nuclear acid agent by the same method as in Example 8.

### (4) Evaluation of gene suppression effect

A left hippocampus was extirpated from the mice at 7 days after the administration of various nucleic acid agents. Using an QIAGEN miRNeasy Mini kit, RNA was extracted from the left hippocampus extirpated. A cDNA was synthesized using Transcriptor Universal cDNA Master, DNase in accordance with the protocol.

Next, the resulting cDNA template was used to perform quantitative RT-PCR, whereby the expression amounts of Hdac2 mRNA and Actb mRNA (the internal standard gene) were measured. Quantitative RT-PCR was performed with TaqMan. Primers used in quantitative RT-PCR were products designed and manufactured by Thermo Fisher Scientific Inc. Amplification conditions (temperature and time) were as follows: the cycle, 95°C for 15 seconds, 60°C for 30 seconds, and 72°C for 1 second, was repeated 40 times.

The ratio of the expression amount of Hdac2 mRNA to the expression amount of Actb mRNA (internal standard gene) was calculated, and a value standardized with respect to the value of the PBS-treated group was determined as a relative Hdac2 mRNA level.

### (Results)

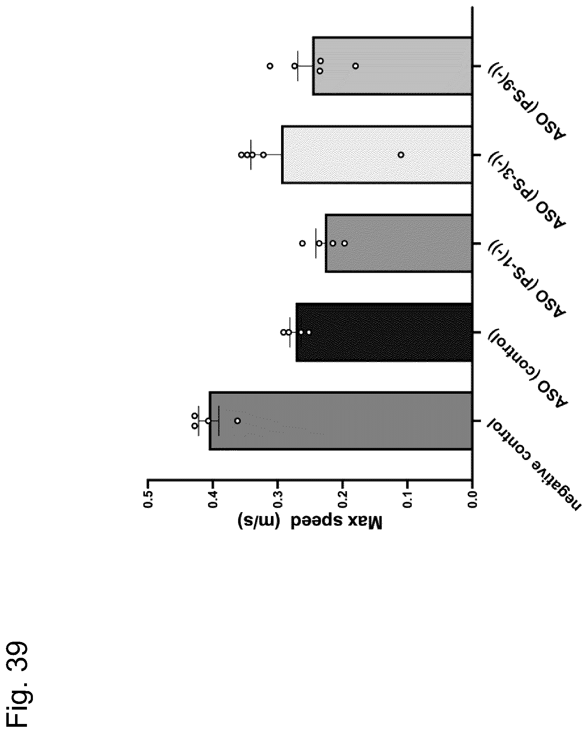

Figure 39 shows the results of evaluation of the motor function through an open-field test. The administration groups of ASO (PS-1(-)), ASO (PS-3(-)), and ASO (PS-9(-)) had the same maximum moving speed as the administration group of ASO (control), indicating no reduction in delayed central nervous system toxicity.

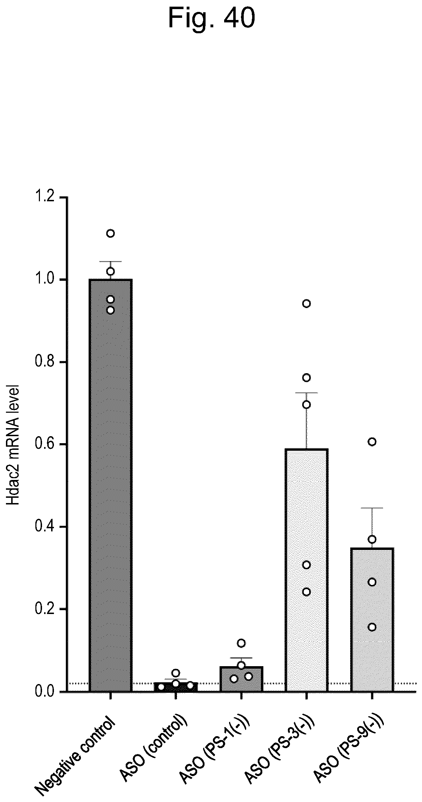

Figure 40 shows the Hdac2 mRNA expression level in the left hippocampus at 7 days after the intraventricular administration of various nucleic acid agents. The gene suppression effect was attenuated with the administration groups of ASO (PS-1(-)), ASO (PS-3(-)), and ASO (PS-9(-))), compared with the administration group of ASO (control).

The above-described results have revealed that changing only one PS linkage to a PO linkage did not reduce delayed toxicity compared with the ASO (control)-treated group, while attenuating the gene suppression effect.

All publications, patents, and patent applications cited herein are incorporated herein by reference in their entirety.

## Claims

1. A nucleic acid molecule having an antisense effect on a target gene or a transcriptional product thereof, comprising a base sequence complementary to at least part of the target gene or the transcriptional product thereof,
wherein the nucleic acid molecule comprises:
(1) a central region which comprises at least three consecutive deoxyribonucleosides,
(2) a 5'-wing region which comprises a non-natural nucleoside, positioned on the 5' end side of said central region, and
(3) a 3'-wing region which comprises a non-natural nucleoside, positioned on the 3' end side of said central region, and
wherein the nucleic acid molecule comprises the 5'-modified nucleoside of the following formula (I) at the 1st, 3rd, and/or 9th base position from the 5' side of said central region.

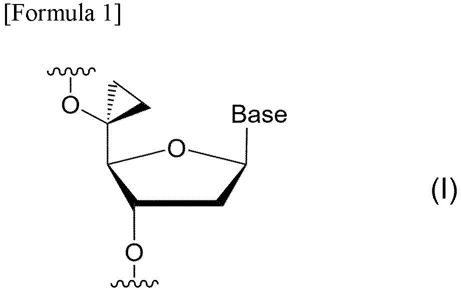

2. The nucleic acid molecule of claim 1, wherein the internucleoside linkage at 5' side of said 5'-modified nucleoside is a modified internucleoside linkage or phosphodiester linkage.

3. The nucleic acid molecule of claim 2, wherein said modified internucleoside linkage is phosphorothioate linkage.

4. The nucleic acid molecule of claim 1, comprising a 2'-modified nucleoside at:
the terminal base position adjacent to said central region in said 5'-wing region,
the 2nd base position from 5' side of said central region, and/or
the 8th base position from 5' side of said central region.

5. The nucleic acid molecule of claim 1, comprising a 2'-modified nucleoside at the base position adjacent to 5' side of said 5'-modified nucleoside.

6. The nucleic acid molecule of claim 4, wherein said 2'-modified nucleoside is 2'-O-methyl-modified nucleoside, 2'-O-methoxyethyl-modified nucleoside, 2'-O-[2-(*N-*methylcarbamoyl)ethyl]-modified nucleoside, or 2'-fluoro-modified nucleoside.

7. The nucleic acid molecule of claim 1, which is 12 to 30 bases in length.

8. The nucleic acid molecule of claim 1, wherein said 5'-wing region and said 3'-wing region comprise a bridged nucleoside and/or a 2'-modified nucleoside.

9. The nucleic acid molecule of claim 8, wherein said bridged nucleoside is selected from the group consisting of LNA nucleoside, 2', 4'-BNA^{NC} nucleoside, cET BNA nucleoside, ENA nucleoside, AmNA nucleoside, GuNA nucleoside, scpBNA nucleoside, scpBNA2 nucleoside, and BANA3 nucleoside.

10. The nucleic acid molecule of claim 8, wherein said 2'-modified nucleoside is 2'-O-methyl-modified nucleoside or 2'-O-methoxyethyl-modified nucleoside.

11. The nucleic acid molecule of claim 1, wherein all or a part of the internucleoside linkages in said nucleic acid molecule are modified internucleoside linkages.

12. The nucleic acid molecule of claim 11, wherein said modified internucleoside linkages are phosphorothioate linkages.

13. The nucleic acid molecule of claim 1, comprising a modified nucleobase.

14. The nucleic acid molecule of claim 1, which is capable of inducing steric blocking, exon skipping, and/or exon inclusion for said target gene or a transcriptional product thereof.

15. A double-stranded nucleic acid complex comprising a first nucleic acid strand consisting of the nucleic acid molecule of any one of claims 1 to 14 and a second nucleic acid strand comprising a base sequence complementary to said first nucleic acid strand.

16. The double-stranded nucleic acid complex of claim 15, wherein said second nucleic acid strand is at least 8 bases in length.

17. The double-stranded nucleic acid complex of claim 15, wherein said second nucleic acid strand comprises any one or more selected from the group consisting of a deoxyribonucleoside, 2'-modified nucleoside, 5'-modified nucleoside, and bridged nucleoside.

18. The double-stranded nucleic acid complex of claim 15, wherein said second nucleic acid strand comprises a noncomplementary base, and/or an insertion sequence and/or deletion of one or more bases, relative to said first nucleic acid strand.

19. The double-stranded nucleic acid complex of claim 18, wherein said second nucleic acid strand comprises one to three of said noncomplementary base.

20. The double-stranded nucleic acid complex of claim 18, wherein said insertion sequence consists of 1 to 8 bases.

21. The double-stranded nucleic acid complex of claim 18, wherein said deletion consists of 1 to 4 consecutive bases.

22. The double-stranded nucleic acid complex of claim 15, wherein said second nucleic acid strand comprises at least one overhang region positioned on the 5' end side and/or 3' end side of a region consisting of the base sequence complementary to said first nucleic acid strand.

23. The double-stranded nucleic acid complex of claim 22, wherein said overhang region is 1 to 20 bases in length.

24. The double-stranded nucleic acid complex of claim 15, wherein said first nucleic acid strand and said second nucleic acid strand are bound via a linker.

25. The double-stranded nucleic acid complex of claim 24, wherein said linker is a cleavable or uncleavable linker.

26. The double-stranded nucleic acid complex of claim 24, wherein said linker comprises the group represented by the following formula (IV). 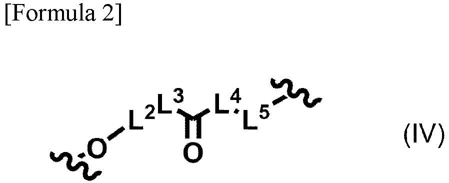 [wherein L² represents a substituted or unsubstituted C₁-C₁₂ alkylene group, a substituted or unsubstituted C₃-C₈ cycloalkylene group, -(CH₂)₂-O-(CH₂)₂-O-(CH₂)₂-O-(CH₂)₃-, or CH(CH₂-OH)-CH₂-O-(CH₂)₂-O-(CH₂)₂-O-(CH₂)₂-O-(CH₂)₃-, L³ represents -NH- or a bond, L⁴ represents a substituted or unsubstituted C₁-C₁₂ alkylene group, a substituted or unsubstituted C₃₋₈ cycloalkylene group, -(CH₂)₂-[O-(CH₂)₂]ₘ-, or a bond, wherein m is an integer of 1 to 25, L⁵ represents -NH-(C=O)-, -(C=O)-, or a bond.]

27. The double-stranded nucleic acid complex of claim 24, wherein said linker comprises a nucleic acid, polyether group, and/or alkylamino group.

28. The double-stranded nucleic acid complex of claim 27, wherein said nucleic acid consists of one or 2 to 10 nucleosides and/or non-natural nucleosides linked via internucleoside linkages.

29. The double-stranded nucleic acid complex of claim 27, wherein said polyether group is a polyethylene glycol group or triethylene glycol group.

30. The double-stranded nucleic acid complex of claim 27, wherein said alkylamino group is a hexylamino group.

31. The double-stranded nucleic acid complex of claim 15, wherein all or a part of the internucleoside linkages in said second nucleic acid strand are modified internucleoside linkages.

32. The double-stranded nucleic acid complex of claim 31, wherein said modified internucleoside linkages are phosphorothioate linkages.

33. A composition comprising the nucleic acid molecule of claim 1 or the double-stranded nucleic acid complex of claim 15.

34. The composition of claim 33 for treating a central nervous system disease of a subject.

35. The composition of claim 34, wherein said central nervous system is selected from the group consisting of cerebral cortex, basal ganglia, cerebral white matter, diencephalon, brainstem, cerebellum, and spinal cord.

36. The composition of claim 34, wherein said central nervous system is selected from the group consisting of frontal lobe, temporal lobe, hippocampus, parahippocampal gyrus, parietal lobe, occipital lobe, striatum, globus pallidus, claustrum, thalamus, subthalamic nucleus, midbrain, substantia nigra, pons, medulla oblongata, cerebellar cortex, cerebellar nuclei, cervical cord, thoracic cord, and lumbar cord.

37. The composition of claim 33, which is for intrathecal administration, intranasal administration, intravenous administration, subcutaneous administration, intraperitoneal administration, or intramuscular administration.

38. The composition of claim 37, wherein said intrathecal administration is intraventricular administration, suboccipital puncture or lumbar puncture.

39. The composition of claim 37, which is administered with a shunt, indwelling catheter, or subcutaneous port.

40. The composition of claim 33, wherein 0.1 mg to 200 mg of said double-stranded nucleic acid complex is administered.
